# EUROPÄISCHE PATENTSCHRIFT

(11) **EP 1 421 634 B1**
(45) Veröffentlichungstag und Bekanntmachung des Hinweises auf die Patenterteilung: **16.11.2005**
(21) Anmeldenummer: 02760128.5
(22) Anmeldetag: 21.08.2002
(51) Int. Cl.: H01L 51/30, C09K 11/06, C07C 229/62, C07C 255/58, C07D 209/08, C07D 213/74, C07D 239/42, C07D 295/12, C07D 295/18, C07D 317/66, C07D 455/04, C07D 498/04

(54) **ORGANISCHE ELEKTROLUMINESZIERENDE VORRICHTUNG AUF BASIS VON 2,5-DIAMINOTEREPHTHALSÄUREDERIVATEN**
ORGANIC ELECTROLUMINESCENT DEVICE BASED ON 2,5-DIAMINOTEREPHTHALIC ACID DERIVATIVES
DISPOSITIF ORGANIQUE ELECTROLUMINESCENT SE BASANT SUR DES DERIVES D'ACIDE 2,5-DIAMINOTEREPHTHALIQUE

(30) Priorität: 21.08.2001 DE 10141266
(43) Veröffentlichungstag der Anmeldung: 26.05.2004
(73) Patentinhaber: Sensient Imaging Technologies GmbH, 06766 Wolfen (DE)
(72) Erfinder: RICHTER, Andreas, M., 06188 Plössnitz (DE); SCHÖNEWERK, Jens, 04668 Grimma (DE); DIENER, Gerhard, 06366 Köthen (DE)
(74) Vertreter: Walter, Wolf-Jürgen
(86) Internationale Anmeldenummer: PCT/DE2002/003110
(87) Internationale Veröffentlichungsnummer: WO 2003/019697

(56) Entgegenhaltungen:
- EP-A- 0 468 439
- PATENT ABSTRACTS OF JAPAN vol. 1999, no. 02, 26. Februar 1999 (1999-02-26) -& JP 10 294178 A (MITSUI CHEM INC), 4. November 1998 (1998-11-04)
- PATENT ABSTRACTS OF JAPAN vol. 1999, no. 01, 29. Januar 1999 (1999-01-29) -& JP 10 284250 A (MITSUI CHEM INC), 23. Oktober 1998 (1998-10-23)

## Beschreibung

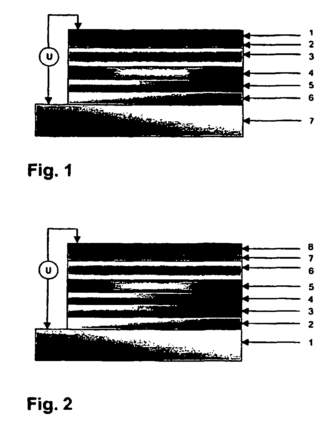

Die Erfindung betrifft eine neue organische elektrolumineszierende Vorrichtung auf der Basis von 2,5-Diaminoterephthalsäurederivaten. Diese Derivate sind Emittersubstanzen für organische Leuchtdioden (OLED).
In organischen Leuchtdioden, die seit langem bekannt sind, wird die Elektrolumineszenz von bestimmten organischen Verbindungen ausgenutzt. Der Aufbau und die Aufgaben der einzelnen Schichten in einer OLED ist beispielhaft in Fig. 1 skizziert:
Zwischen zwei Elektroden, von denen mindestens eine lichtdurchlässig sein muß, befindet sich eine Schichtfolge von organischen Substanzen, die im Device jeweils eine spezielle Funktion haben.
1. die Kathode besteht aus einem unedlen Metall oder einer Legierung (z. B. Aluminium oder Calcium); verantwortlich für die Elektroneninjektion
2. die Pufferschicht aus bestimmten Metallsalzen oder deren Oxiden, wie z. B. LiF; verantwortlich für Verbesserung der Elektroneninjektion in die Schicht 3
3. der Elektronenleiter kann z. B. aus Alq3 (Tris-(8-hydroxychinolinato)-aluminium) bestehen; leitet die Elektronen von der Kathode in das Innere des Devices zur Emissionsschicht bzw. dem Lochleiter
4. der Lochleiter besteht vorrangig aus Derivaten des Triphenylamins; es können mehrere Lochleiterschichten vorhanden sein, deren Eigenschaften auf das Device abgestimmt sind; verantwortlich für den Transport der Löcher zur Emissionsschicht
5. die Anode besteht aus ITO, das die Löcher in die Lochtransportschicht injiziert
6. der Träger besteht aus einem transparenten Material, z. B. Glas

Eine solche Anordnung emittiert grünes Licht, das durch die Anregung des Alq3 durch die aus den Löchern und Elektronen gebildeten Excitonen entsteht.

Eine solche einfache Anordnung besitzt jedoch Nachteile:
1. Alq3 emittiert nur im grünen Spektralbereich
2. die Emission von Alq3 ist zu breitbandig

Diese Nachteile lassen sich durch sog. Dotieren z. T. beseitigen. Dabei wird im Herstellungsprozeß der Diode eine oder mehrere Substanzen co-verdampft. Der Gehalt an diesen Substanzen in der Alq3-Schicht beträgt in der Regel nur wenige Prozent. Dieser Co-Verdampfungsprozeß ist schwierig zu steuern.

Die Erfindung bezieht sich auf neue Emittersubstanzen, welche die bekannten Nachteile von Alq3 als Emitter- und Hostmaterial für Dopanden beseitigt. Alq3 wird dadurch generell nur noch als Elektronenleiter benötigt. Die neuen Emittersubstanzen zeichnen sich aus durch
1. schmalere Emissionsbanden;
2. Abdeckung eines breiten Spektralbereiches in den Devices durch Einsatz unterschiedlicher Vertreter, entweder in voneinander getrennten Schichten oder in Mischschichten;
3. niedrige Treiberspannungen;
4. hohe photometrische Effizienz (niedrige Leistungsaufnahme);
5. hohe Leuchtdichten;
6. hohe thermische Stabilität.

Unter "Device" im Sinne der Erfindung wird die Anordnung verstanden, mit der Träger und Schichten gemäß Fig. 1 oder 2 aufgetragen sind, ohne daß eine Konfektionierung zu einer Leuchtdiode erfolgt ist. Der Aufbau einer solchen erfindungsgemäßen Vorrichtung kann prinzipiell wie in Fig. 1 oder 2 gezeigt erfolgen. Die erfindungsgemäßen 2,5-Diaminoterephthalsäurederivate können in solchen Vorrichtungen allein oder zusammen mit anderen Verbindungen, gegebenenfalls auch bekannten Verbindungen, als Emitter co-verdampft werden. Sie werden zusammen mit bekannten Lochleitern verwendet.

Der Erfindung liegt die Aufgabe zugrunde, neue organische elektrolumineszierende Vorrichtungen mit verbesserten Emittersubstanzen bereitzustellen. Erfindungsgemäß enthält die organische elektrolumineszierende Vorrichtung 2,5-Diaminoterephthalsäurederivate der nachfolgenden Formel **1a** in einer oder mehreren Emitterschichten rein oder dotiert in einem Device 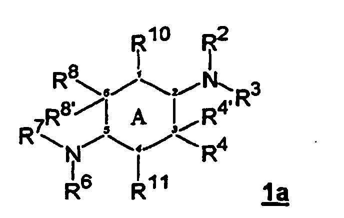 worin der Ring A ein dreifach ungesättigter Benzolring ist, in dem R^{4'} und R^{8'} entfallen, oder der Ring A ist ein zweifach ungesättigter Ring mit je einer Doppelbindung in 1,2-Position und 4,5-Position, und
worin R¹⁰ einen Nitrilrest -CN oder einen Rest -C(=X¹)-X²R¹ bedeutet,
R¹¹ ist ein Nitrilrest -CN oder ein Rest -C(=X³)-X⁴R⁵,
wobei
X¹ und X³ gleiche oder ungleiche Atome oder Gruppen, wie Sauerstoff, Schwefel, Imino, vorzugsweise Sauerstoff, sein können;
X² und X⁴ gleiche oder ungleiche Atome oder Gruppen sein können, wie Sauerstoff, Schwefel, Amino, wobei der Aminostickstoff substituiert sein kann durch Alkyl mit 1 bis 20 C-Atomen, vorzugsweise C1 bis C8 oder durch Aryl, wie z. B. Phenyl, Naphthyl, oder durch Heteroaryl, wie z. B. Cumaryl, Pyridyl, Chinolyl, Indolyl, Carbazolyl, Imidazolyl, Thienyl, Thiazolyl, Furyl, Oxazolyl;
R¹ bis R⁸, R^{4'} und R^{8'} gleiche oder ungleiche Substituenten sein können, wie Wasserstoff, Alkyl mit 1 bis 20 Atomen, vorzugsweise C1 bis C8; Aryl, wie z. B. Phenyl, Naphthyl, sowie Heteroaryl, wie z. B. Cumaryl, Pyridyl, Chinolyl, Indolyl, Carbazolyl, Imidazolyl, Thienyl, Thiazolyl, Furyl, Oxazolyl und diese Reste durch Atome oder Gruppen wie z. B. Di-C1-C3-amino oder Alkoxy mit Alkylresten C1 bis C10, vorzugsweise C1-C4; C1-C4-Alkyl, Cyano, Fluor, Chlor, Brom oder lod sowie Phenyl ein- oder zweifach substituiert sein können;
R⁴ und R⁸ können auch gleiche oder ungleiche Substituenten, wie Halogen, Nitro, Cyano oder Amino sein;
R² bis R⁴ , R⁶ bis R⁸ , R^{4'} und R^{8'} können auch Trifluormethyl oder Pentafluorphenyl sein,
und worin die folgenden Reste einen gesättigten oder ungesättigten Ring bilden können
X¹ und X², R¹ und R², R² und X², R² und R³, R³ und R⁴, R⁴ und X³, X³ und X⁴, R⁵ und X⁴, R⁶ und X⁴, R⁶ und R⁷, R⁷ und R⁸, R⁸ und X¹, R³ und R^{4'}, R⁷und R^{8'}, R⁴ und R^{4'} und R⁸ und R^{8'}, an die weitere Ringe ankondensiert sein können.

Bevorzugt sind R², R³, R⁶ und R⁷ Trifluormethyl oder Pentafluorphenyl , R⁴ und R⁸ sind Halogen, Nitro, Cyan oder Amino, und die anderen Substituenten haben die oben genannte Bedeutung.
Besonders bevorzugt sind R⁴ und R⁸ Trifluormethyl oder Pentafluorphenyl , und die anderen Substituenten haben die oben genannte Bedeutung.

Bezüglich der Schreibweise im folgenden Text bedeutet R¹⁻⁸ R¹ bis R⁸; X^{2,4} ist X² und X⁴; R^{4',8'} ist R^{4'} und R^{8'}.

Die Erfindung betrifft auch neue 2,5-Diaminoterephthatsäurederivate der Formel 19

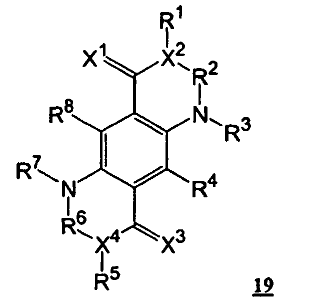

X¹ gleich O und X² gleich O oder N ist; R² und R⁶ sind Methylen (-CH₂-), worin das durch Trifluormethyl substituiert sein kann, R³ und R⁷ sind gleich oder verschieden H, C₁-C₈-Alkyl, Aryl oder Heteroaryl und R⁴ und R⁸ sind gleich oder verschieden H, Alkyl, Aryl oder Trifluormethyl.

Besonders bevorzugt ist Alkyl C1-C4-Alkyl, Aryl ist Phenyl oder Naphthyl, und Heteroaryl ist Pyridyl, Thienyl oder Furyl.

Generell bevorzugt bei allen Strukturen der Erfindung ist, wenn in der Struktur gegenüberliegende Substituenten wie X¹ und X³, X² und X⁴, R¹ und R⁵, R² und R⁶, R³ und R⁷, R⁴ und R⁸, R^{4'} und R^{8'}, R¹⁰ und R¹¹ gleich sind, d.h. nicht unterschiedlich sind.

Erfindungsgemäße elekrolumineszierende Vorrichtungen enthalten vorzugsweise 2 bis 3 unterschiedliche Substanzen im Gemisch miteinander in einem Device.

Nachfolgend werden bevorzugte Strukturen aufgeführt, wobei bei den Strukturen 1

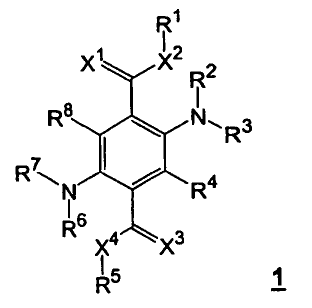

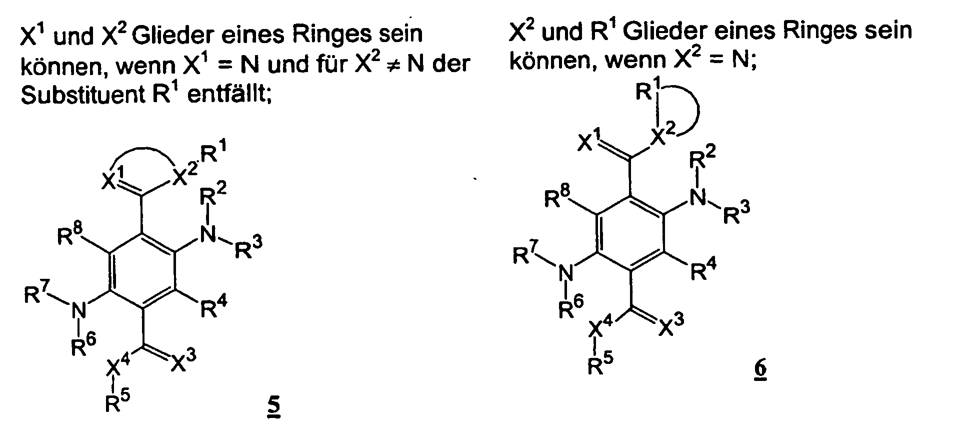

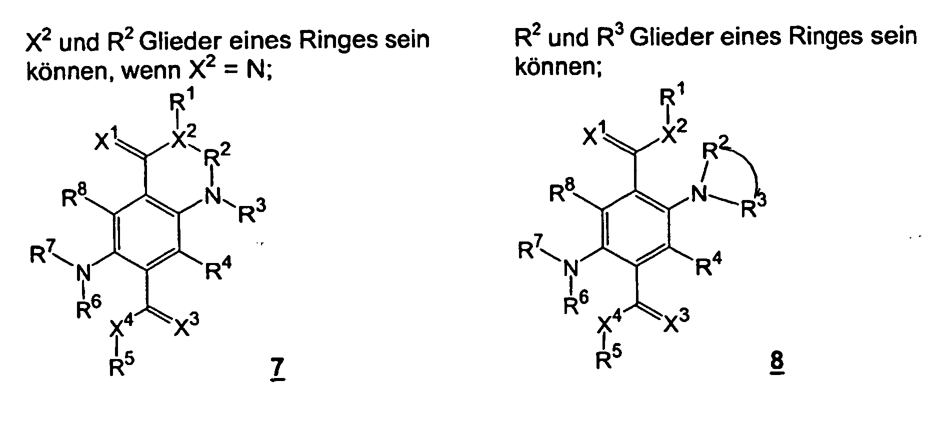

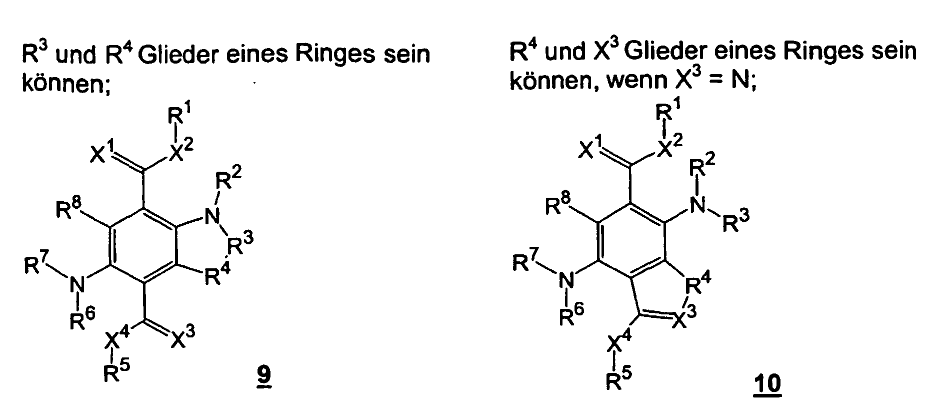

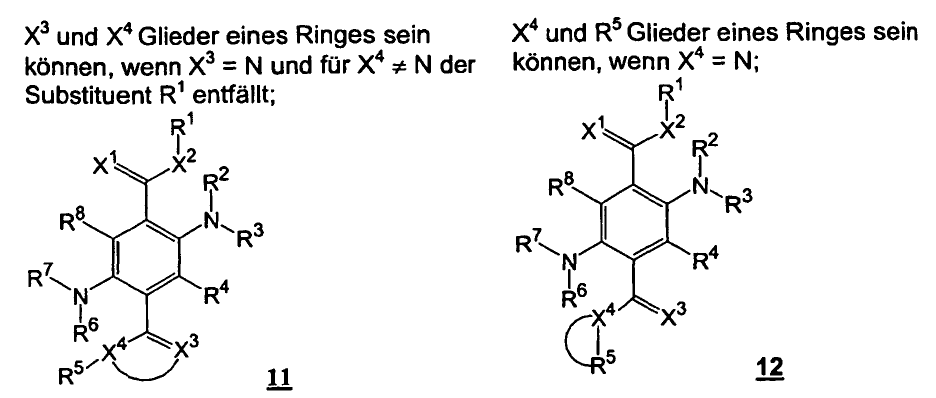

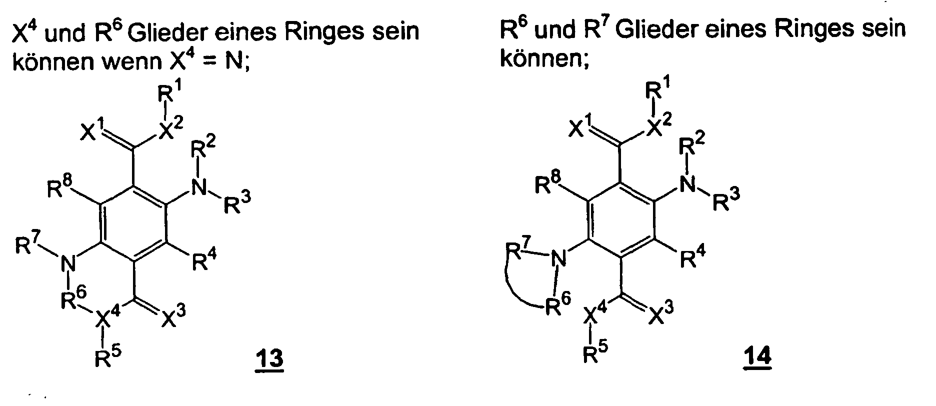

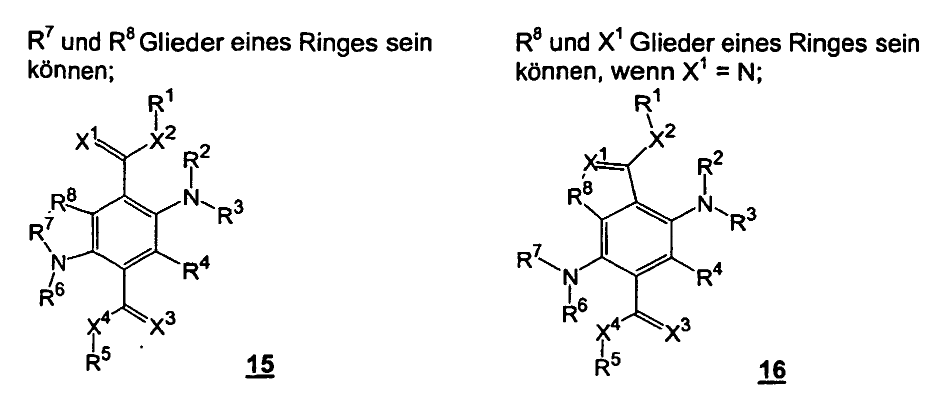

Wobei es sich bevorzugt um die symmetrischen Kombinationen dieser Strukturtypen handelt 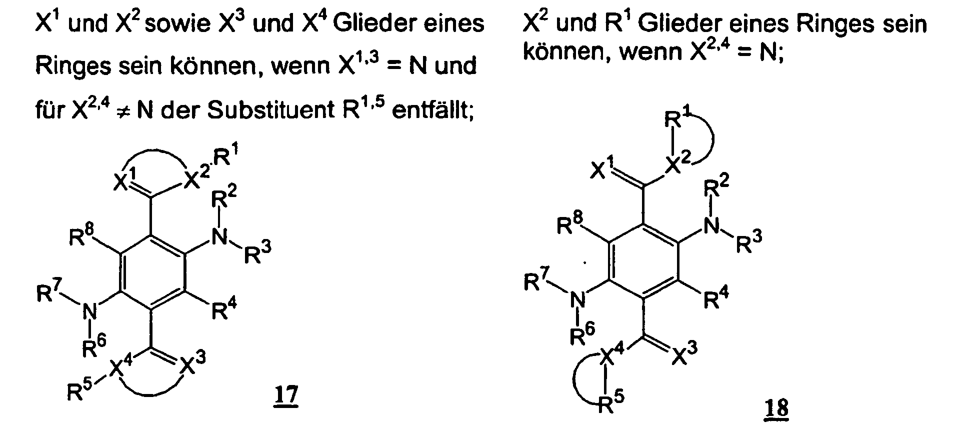 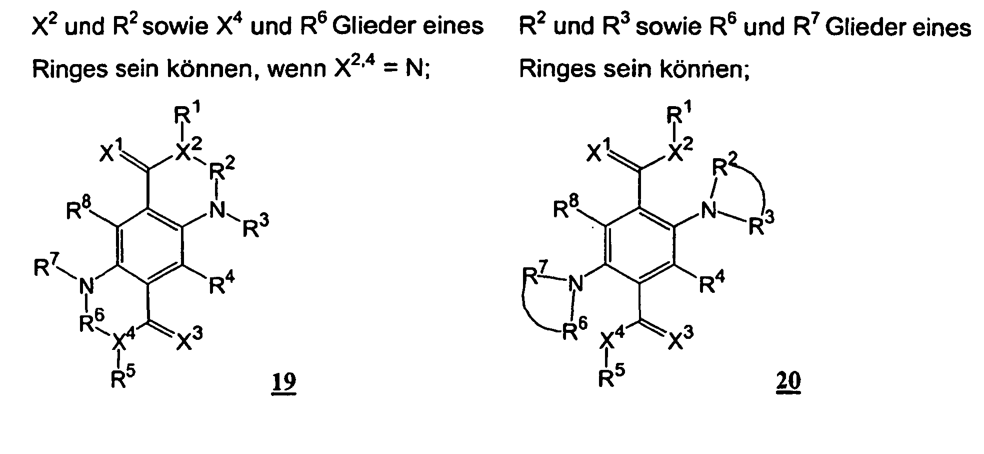 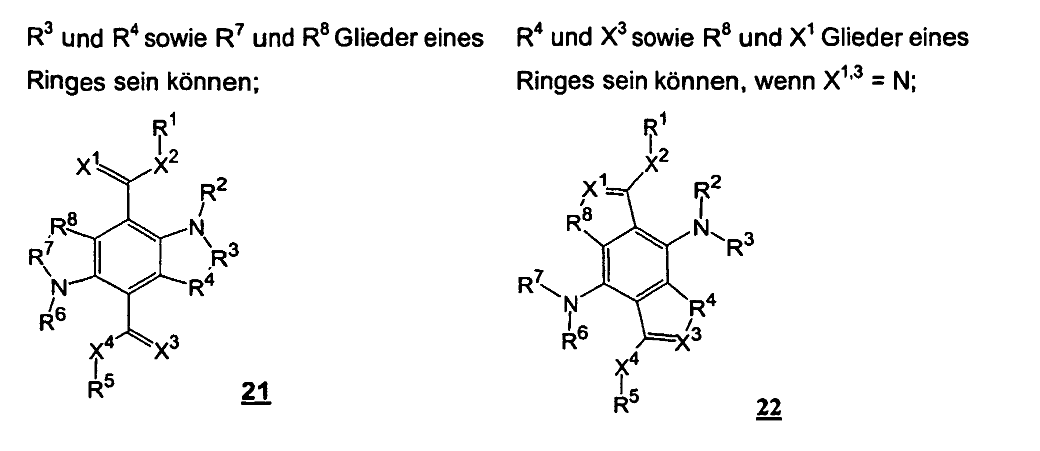 und bei den den Strukturen **2**

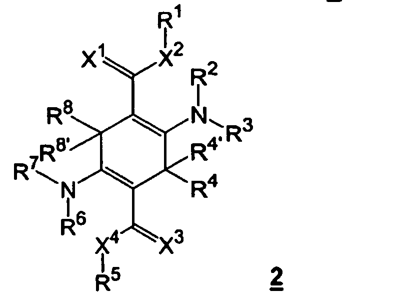

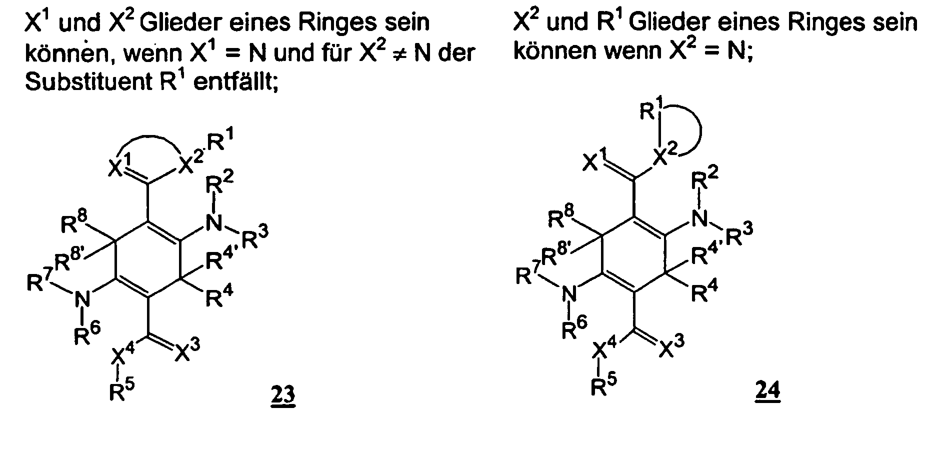

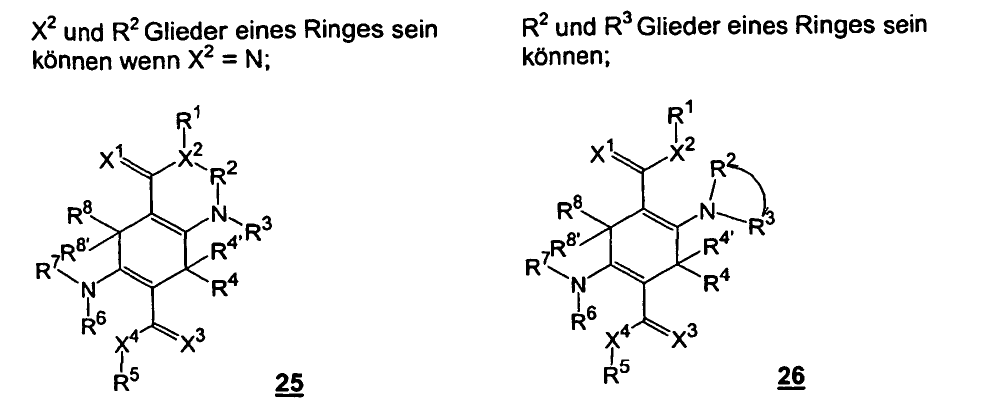

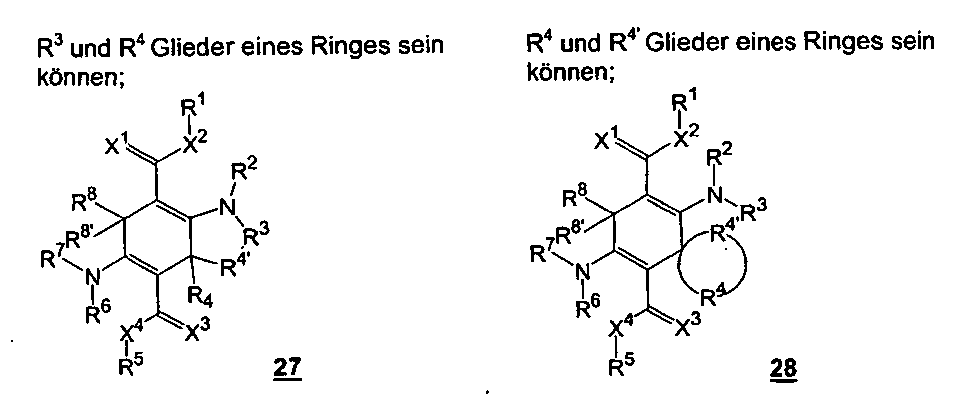

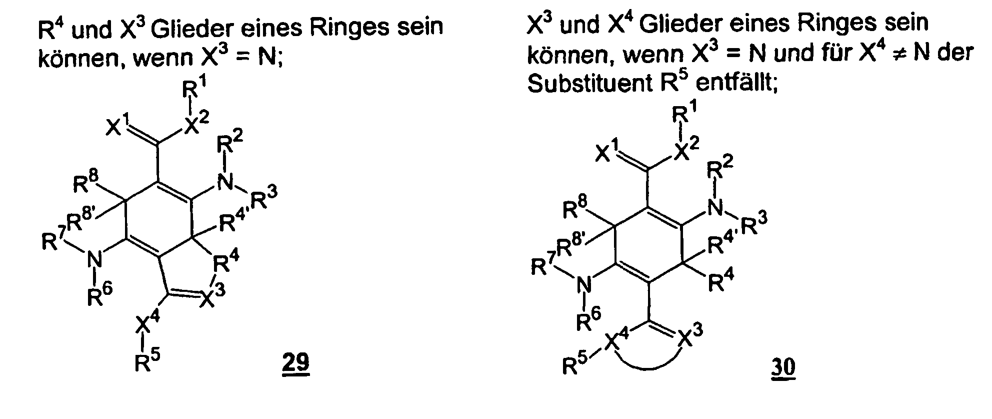

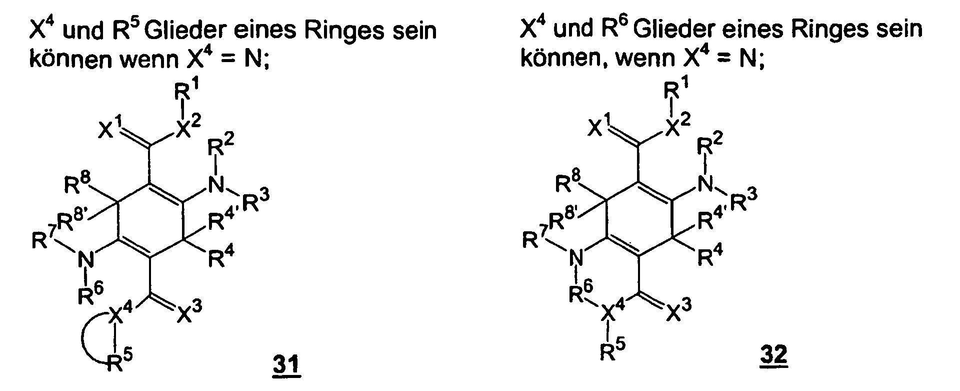

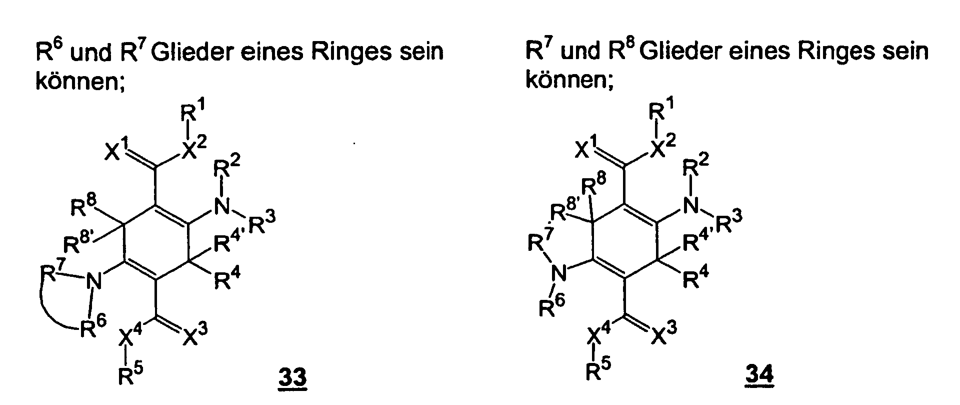

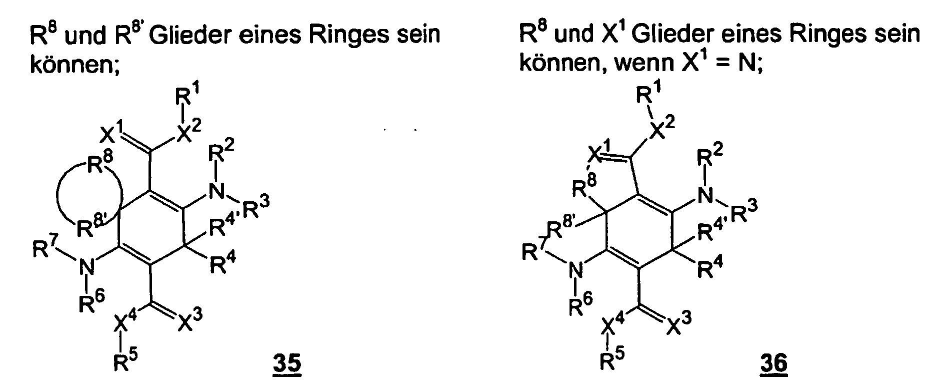

Wobei es sich bevorzugt um die symmetrischen Kombinationen dieser Strukturtypen handelt 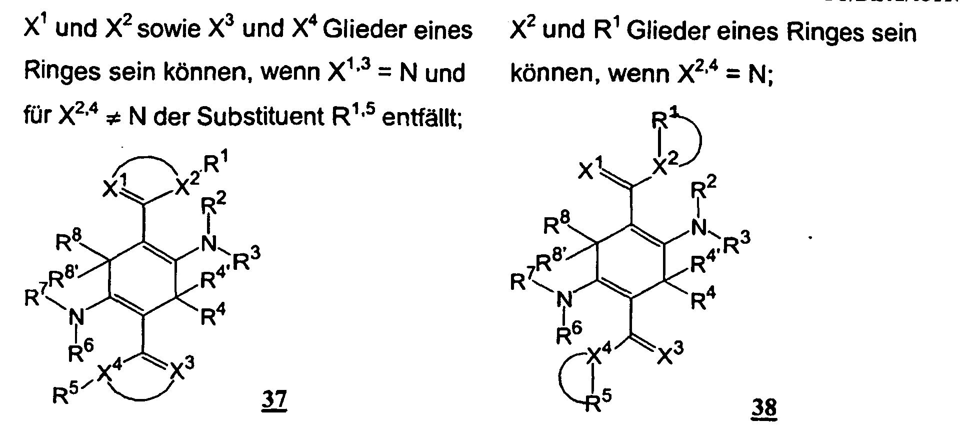 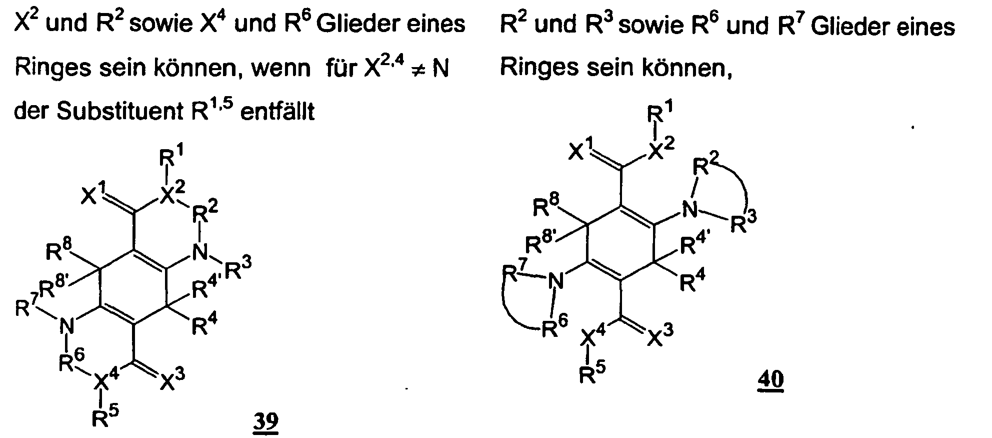 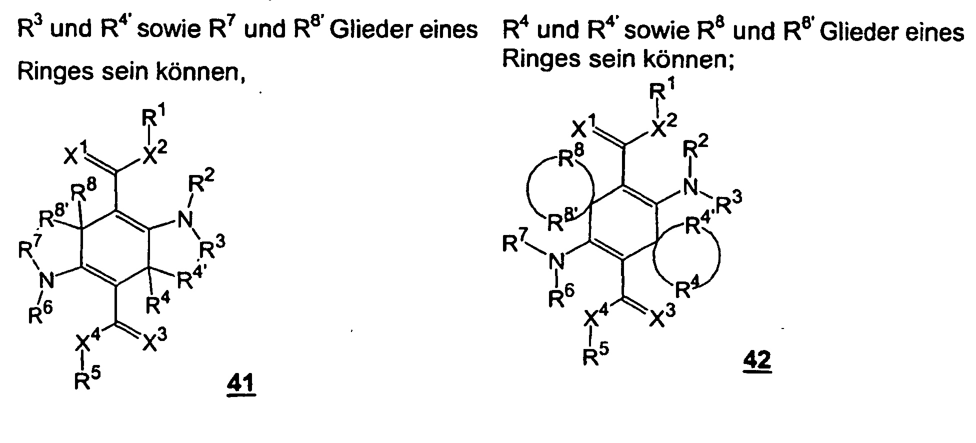 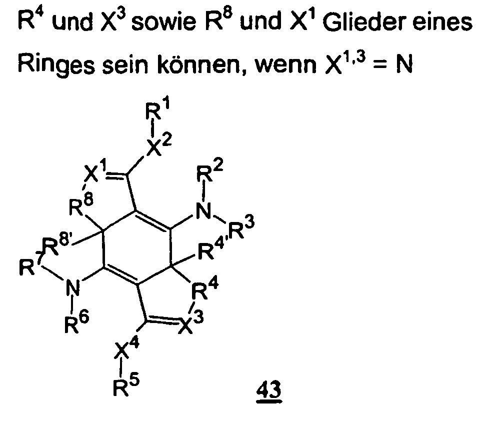 Und bei den Strukturen **3** 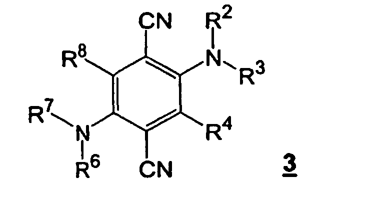 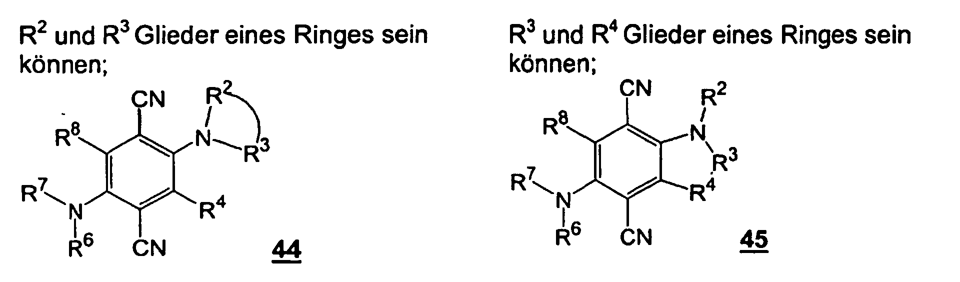 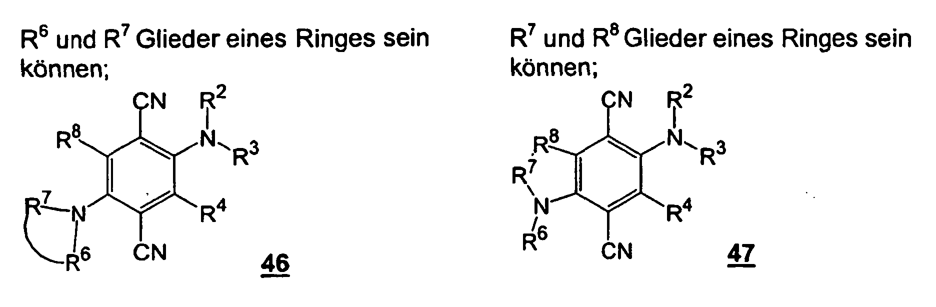 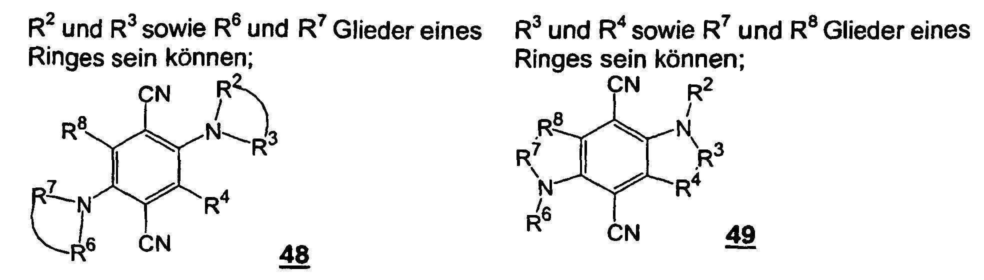 Und bei den Strukturen **4**

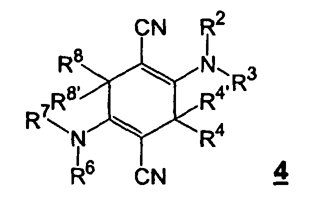

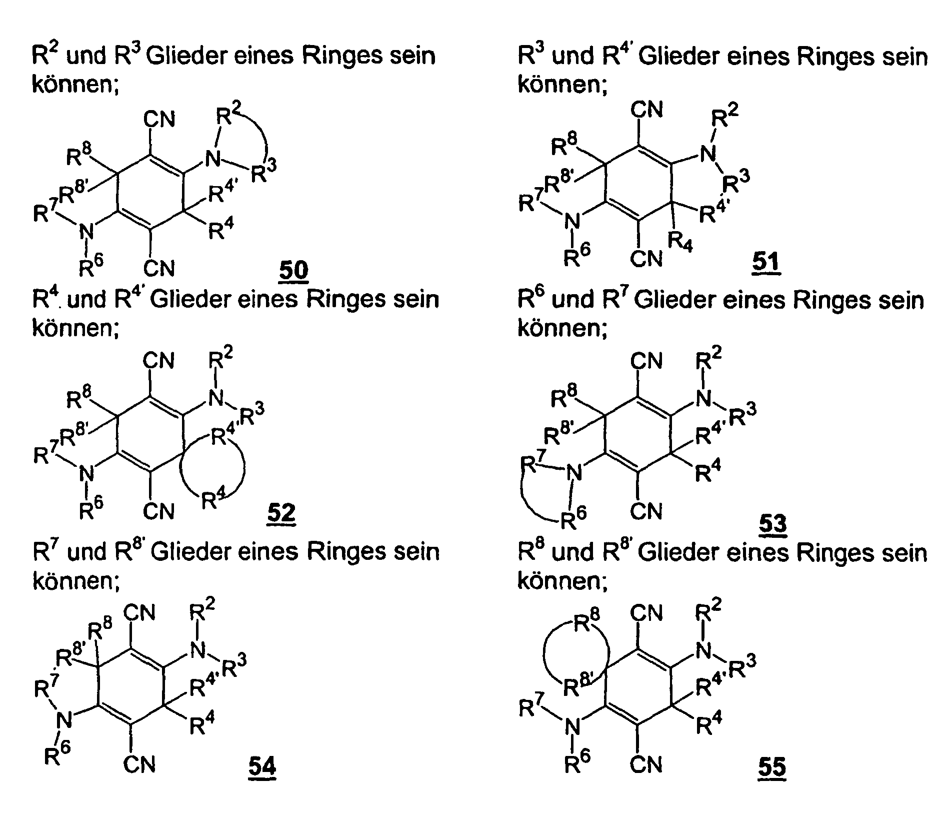

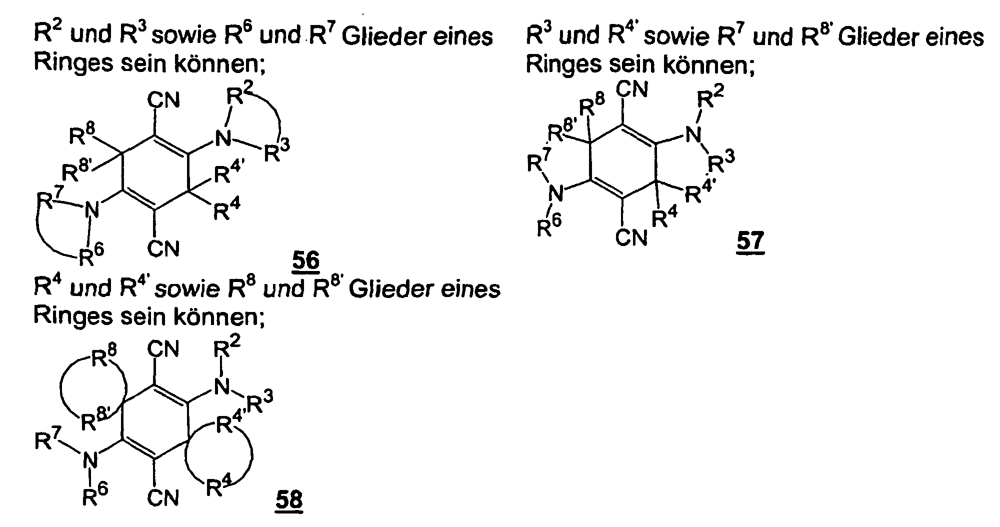

Die Emittersubstanzen der Formel **1** können ausgehend von Cyclohexan-2,5-dion-1,4-dicarbonsäureestern durch Umsetzung mit primären Anilinen bzw. Aminen, anschließender Oxidation und gegebenenfalls weiterer Abwandlung als Derivate der 2,5-Diamino-terephthalsäure erhalten werden. Cyclisierte Derivate können daraus in an sich bekannter Weise hergestellt werden, wie z. B. in Formelschema I und II gezeigt.

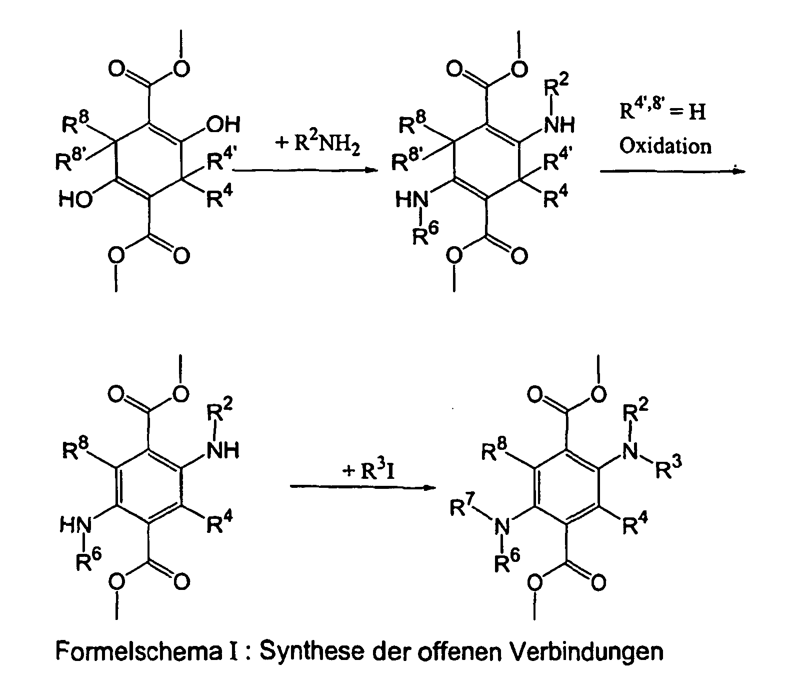

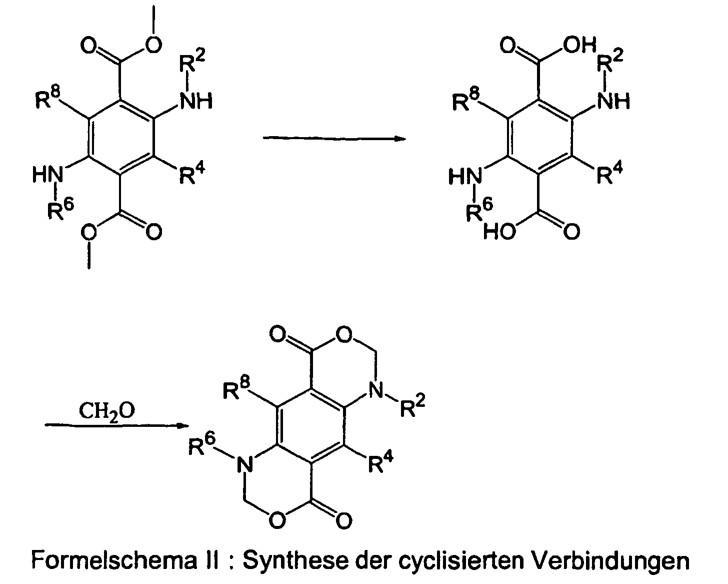

Verbindungen der Formel 3 können über die entsprechenden 2,5-Diaminoterephtalsäureamide durch Umsetzung mit wasserentziehenden Mittel hergestellt werden.

Zu Herstellung von Verbindungen der Formel 4 mit R⁴ und R⁸ sowie R^{4'} und R^{8'} ungleich H werden die 2,5-Diaminocyclohexan-1,4-dicarbonsäureester in die Hydrazide überführt und mit Kaliumhexacyanoferrat(III) zum Aldehyd umgesetzt. Die 2,5-Diaminocyclohexan-1,4-dicarbaldehyde können in die Oxime überführt werden, und durch Reaktion mit Ameisensäure erhält man die Verbindungen nach Formel 4.

Beispiele für die neuen Emitter entsprechend Formel **1** sind in Tabelle 1 zusammengestellt:

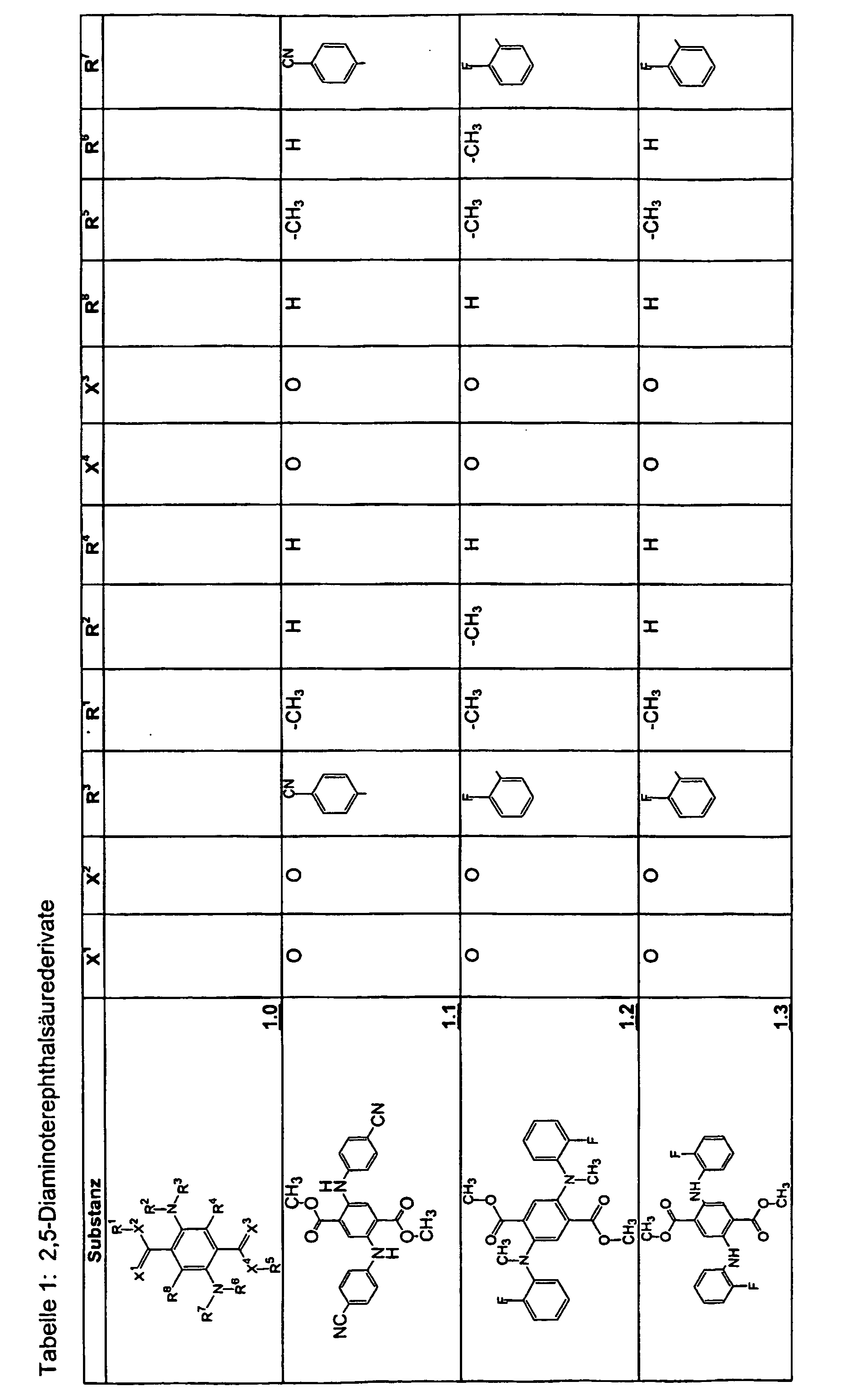

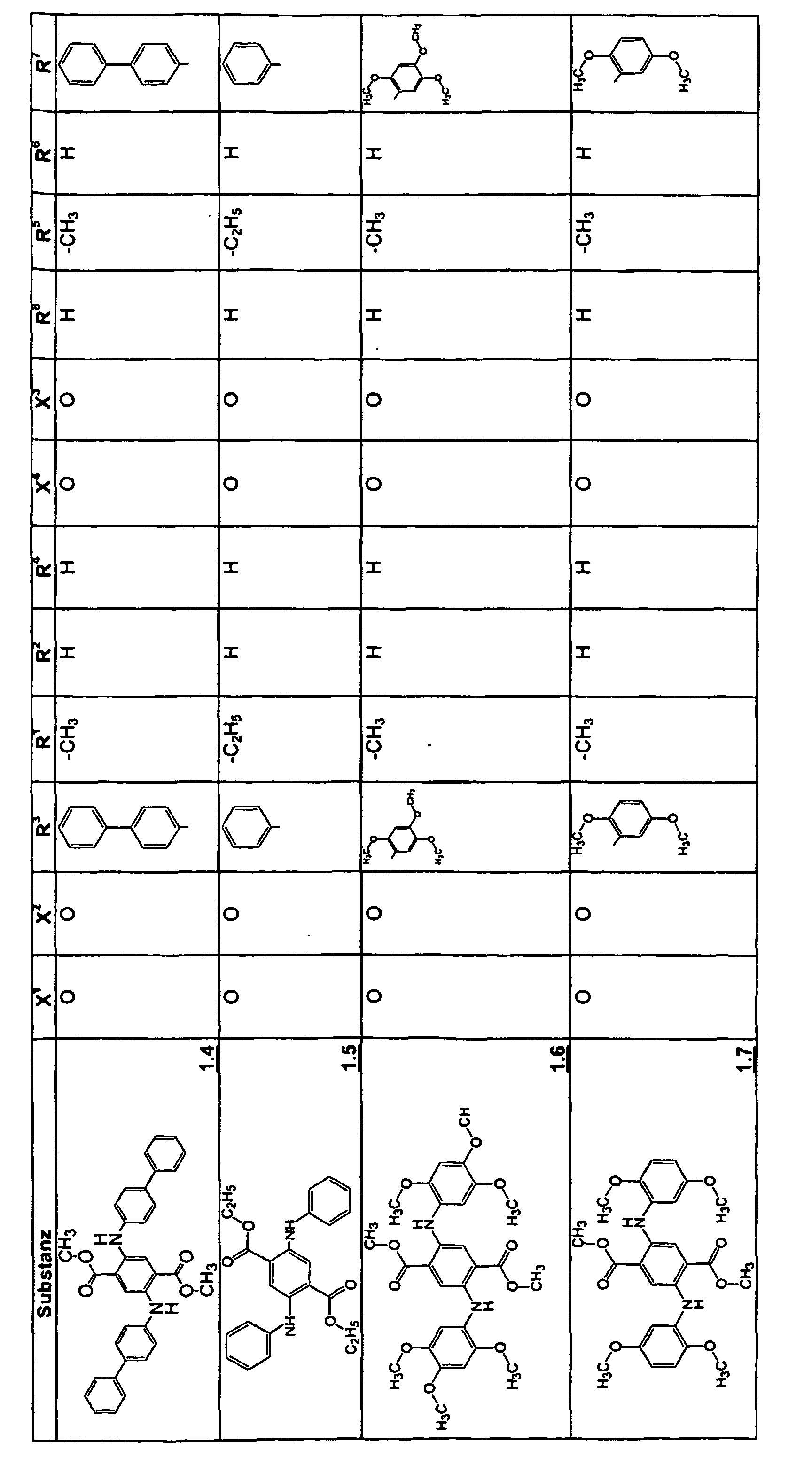

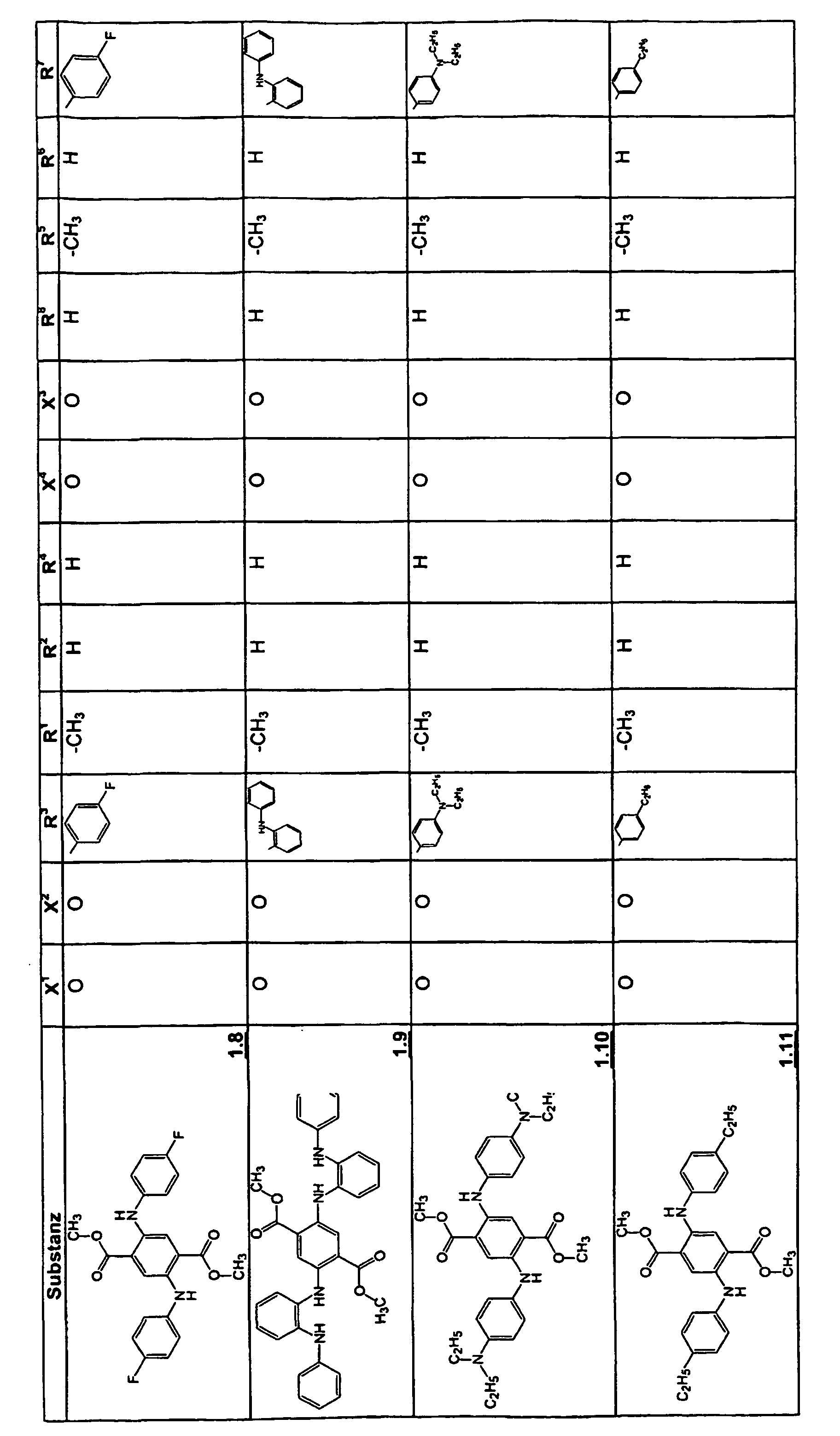

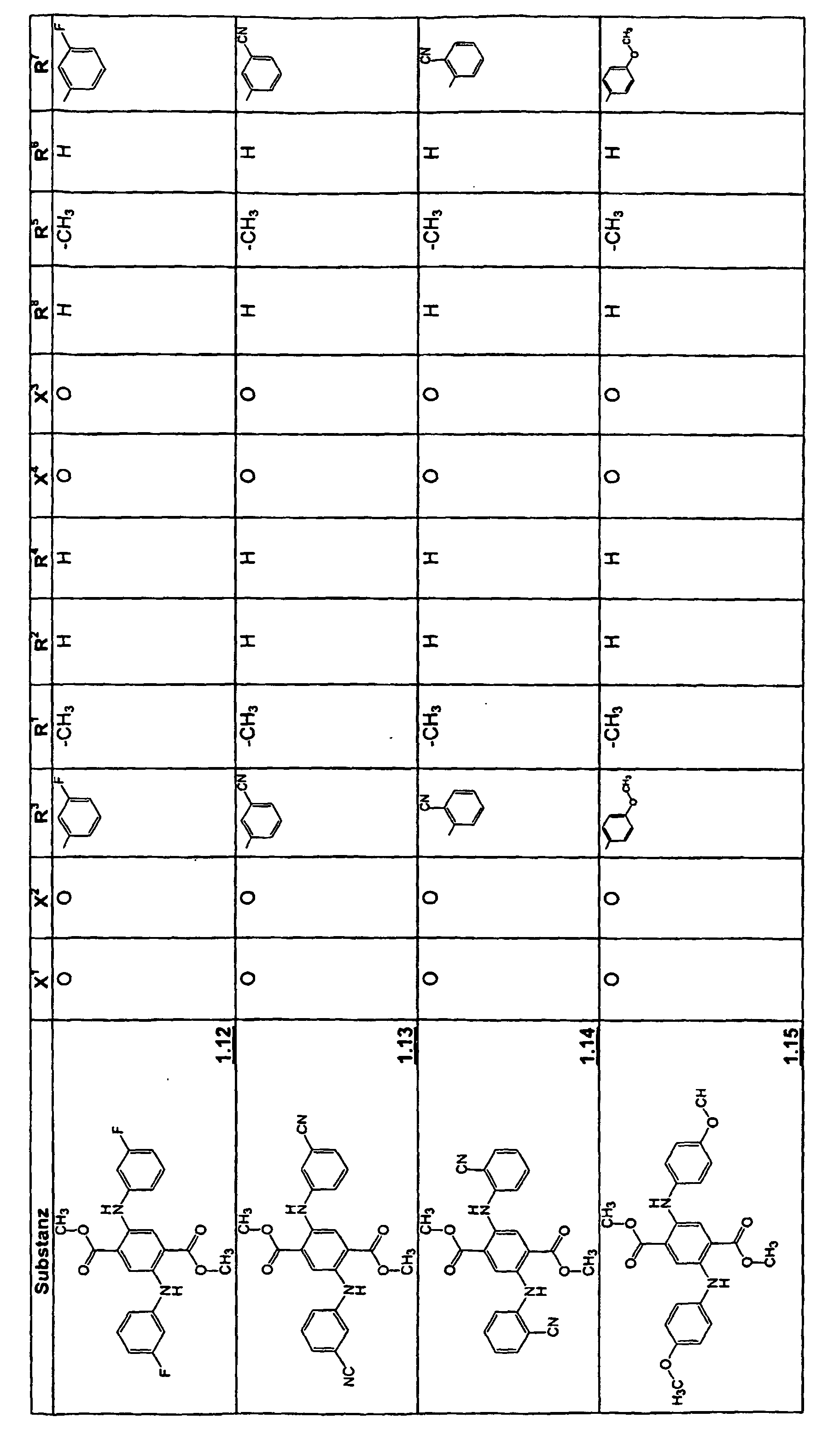

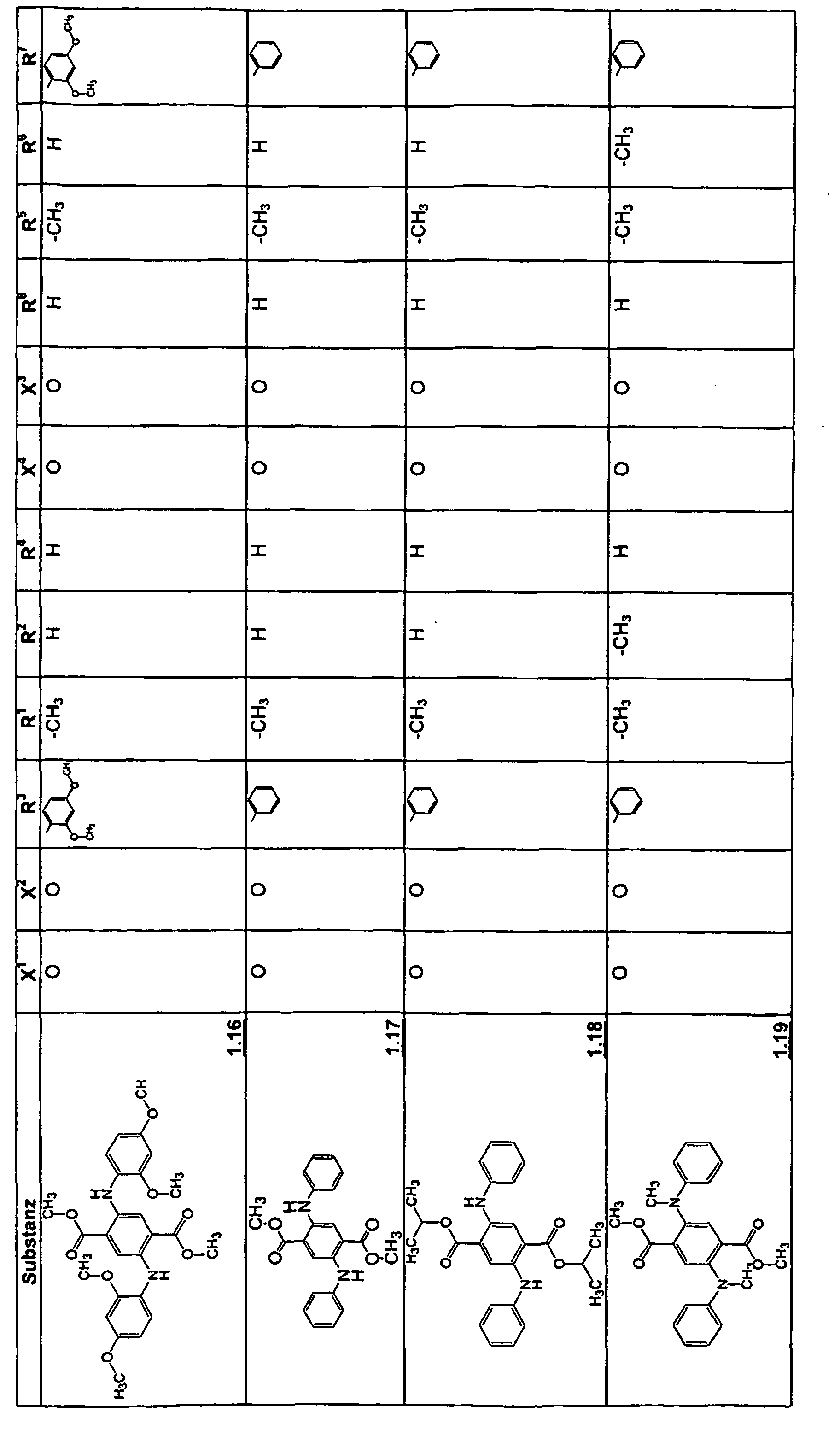

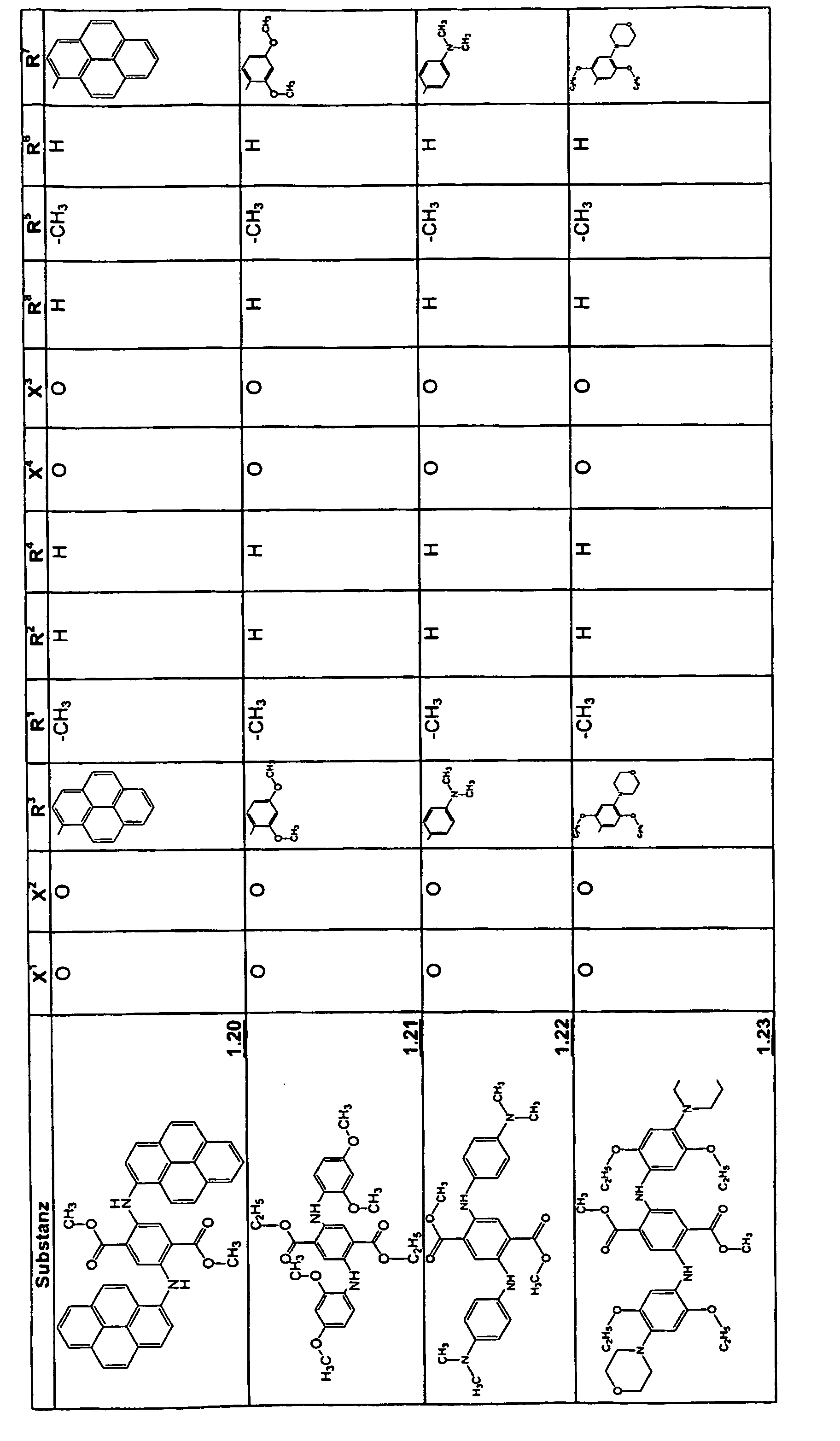

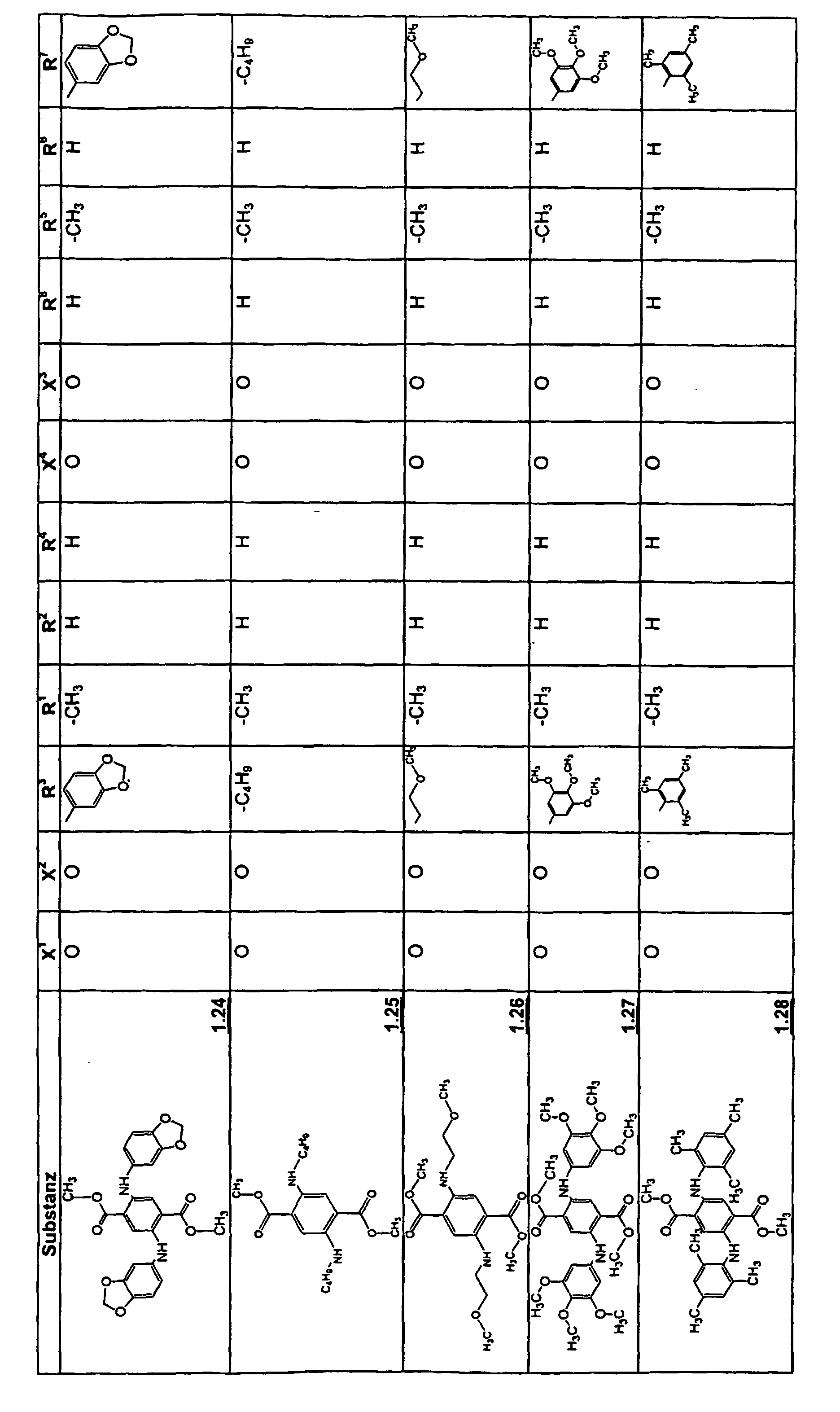

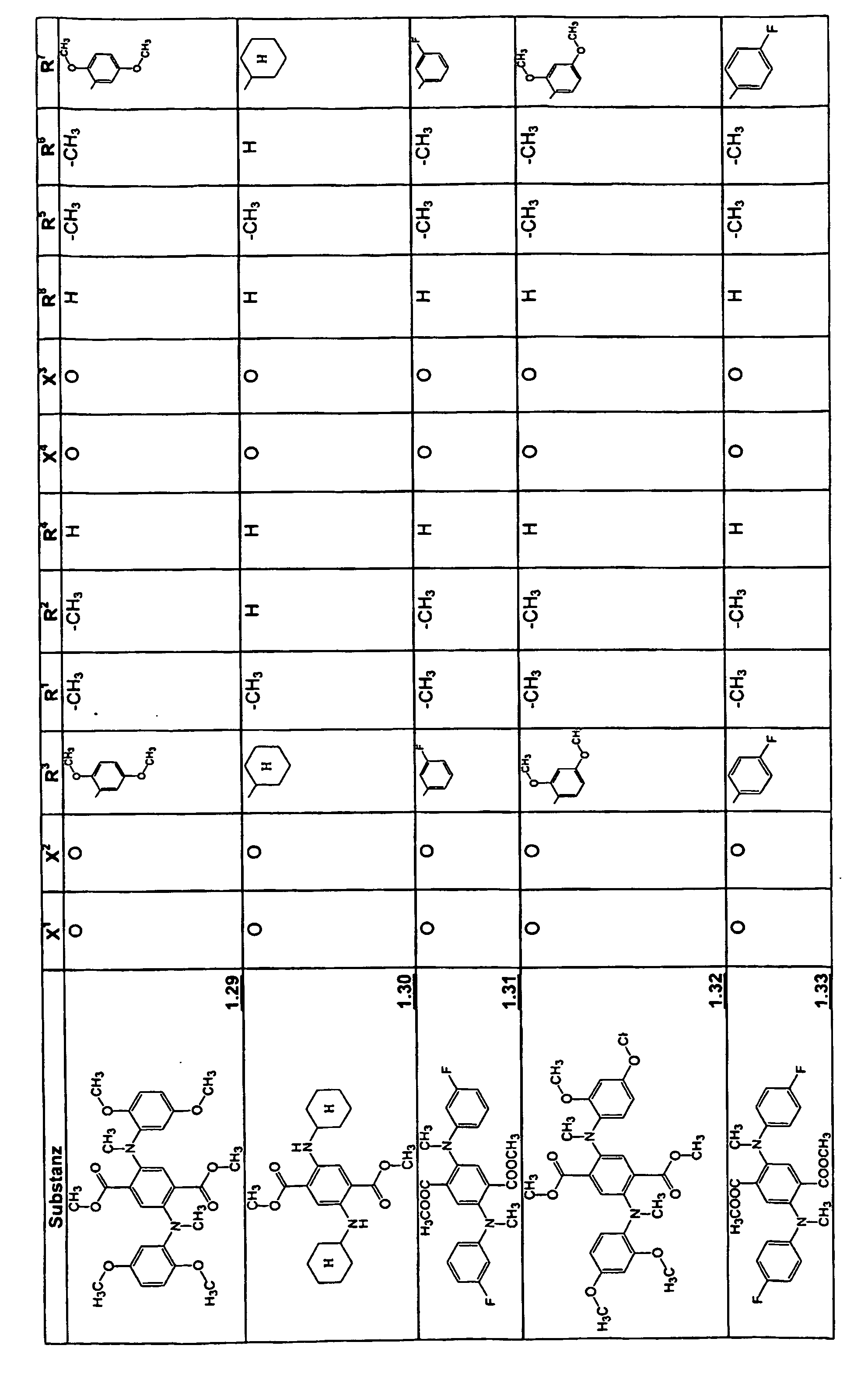

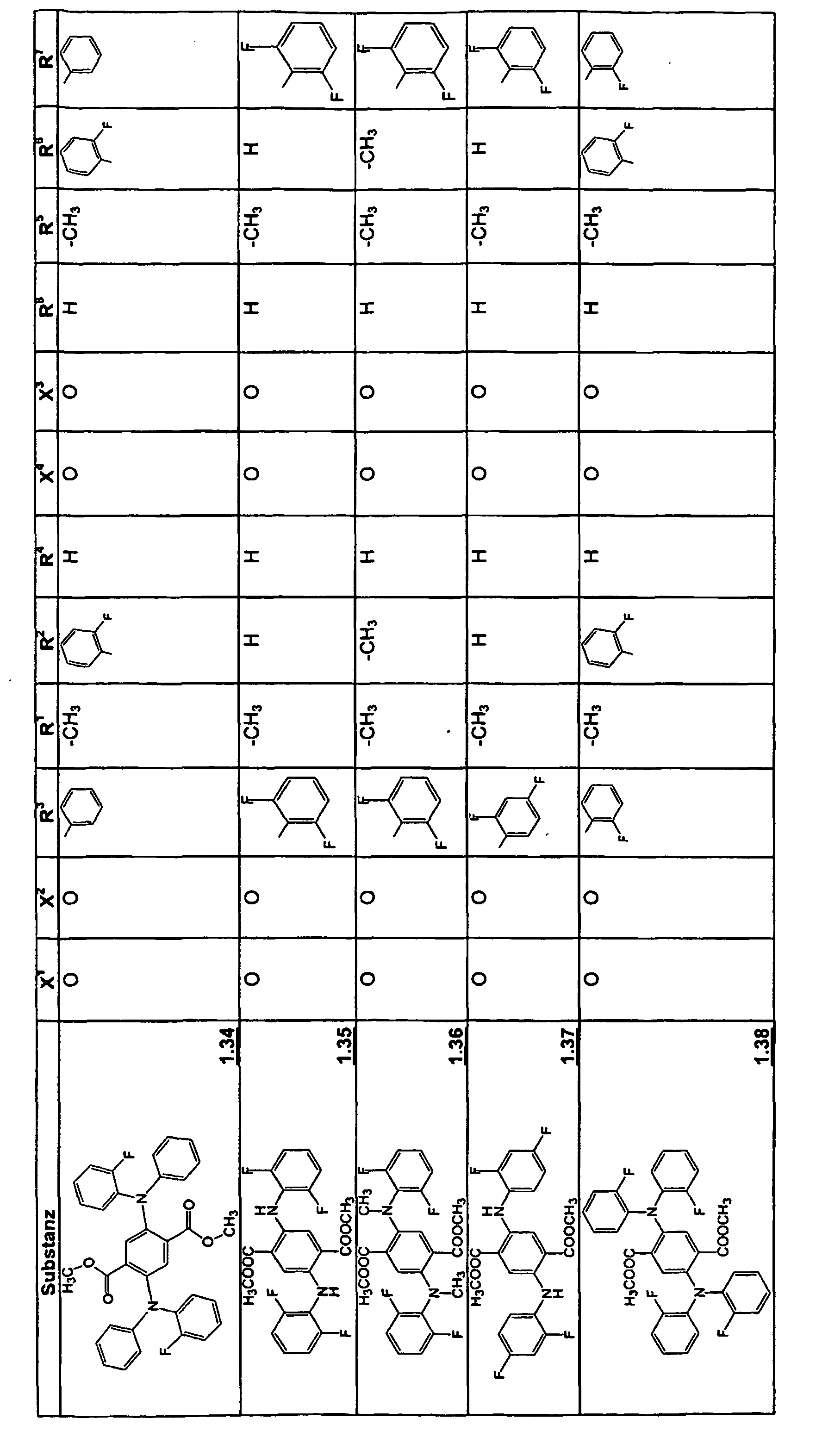

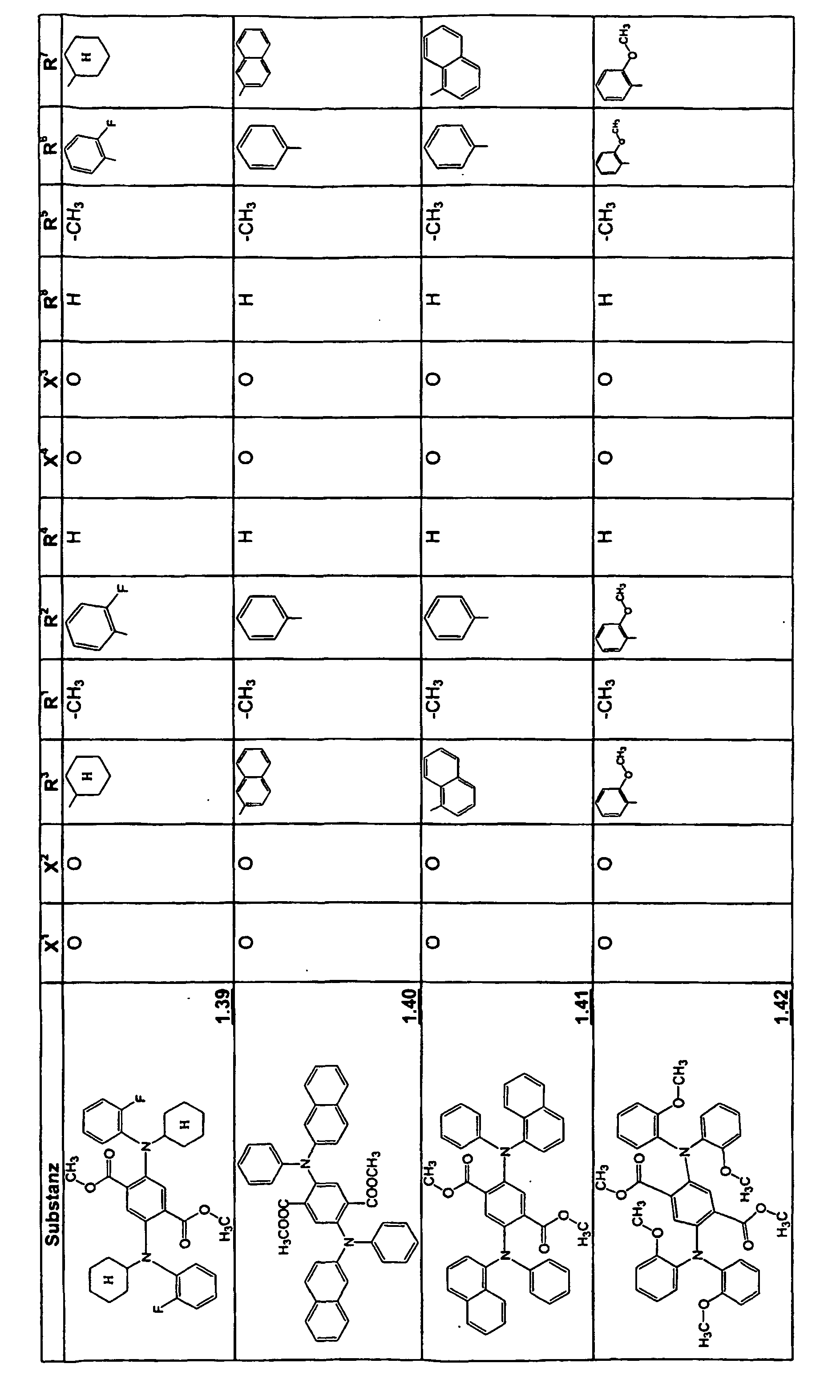

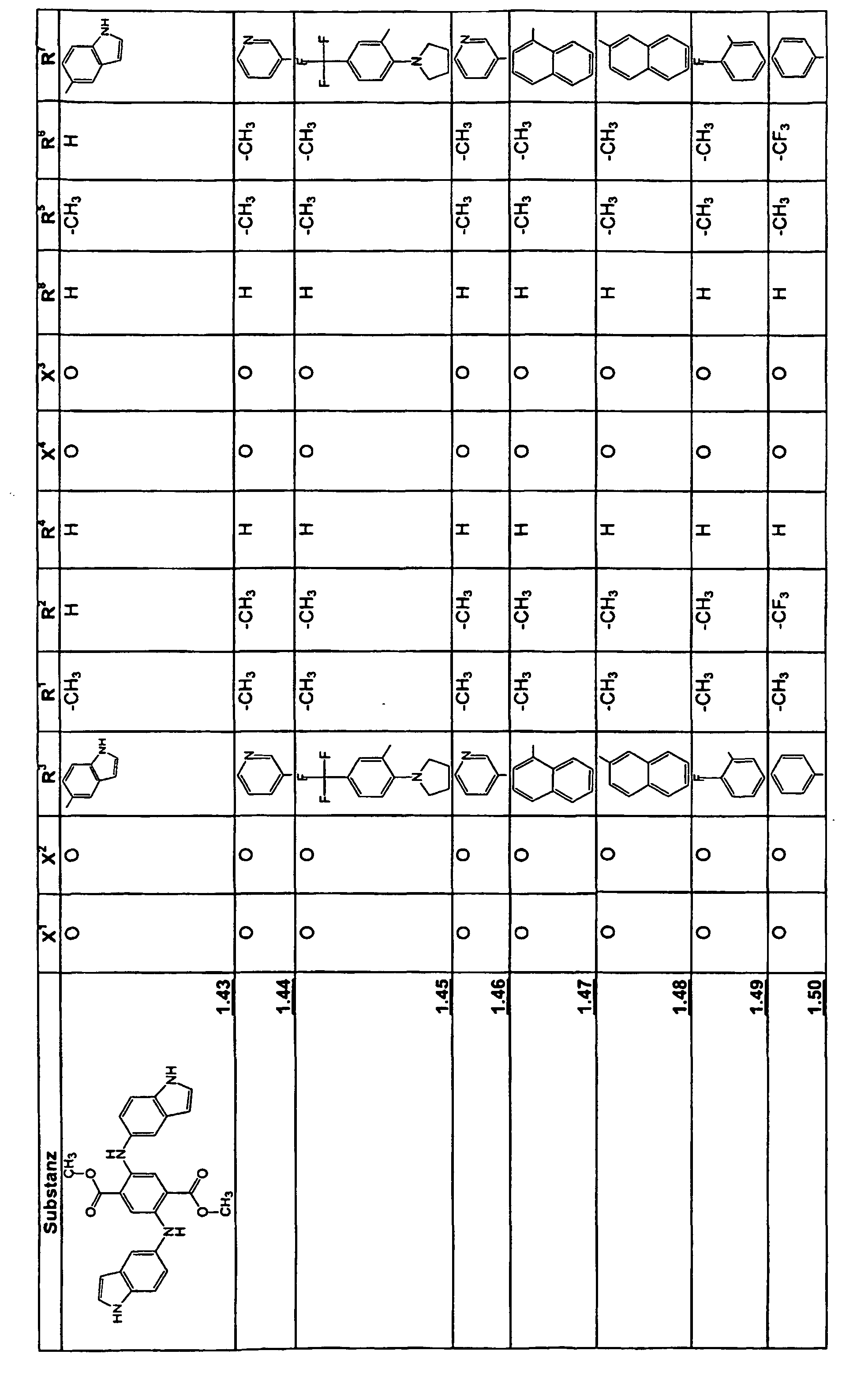

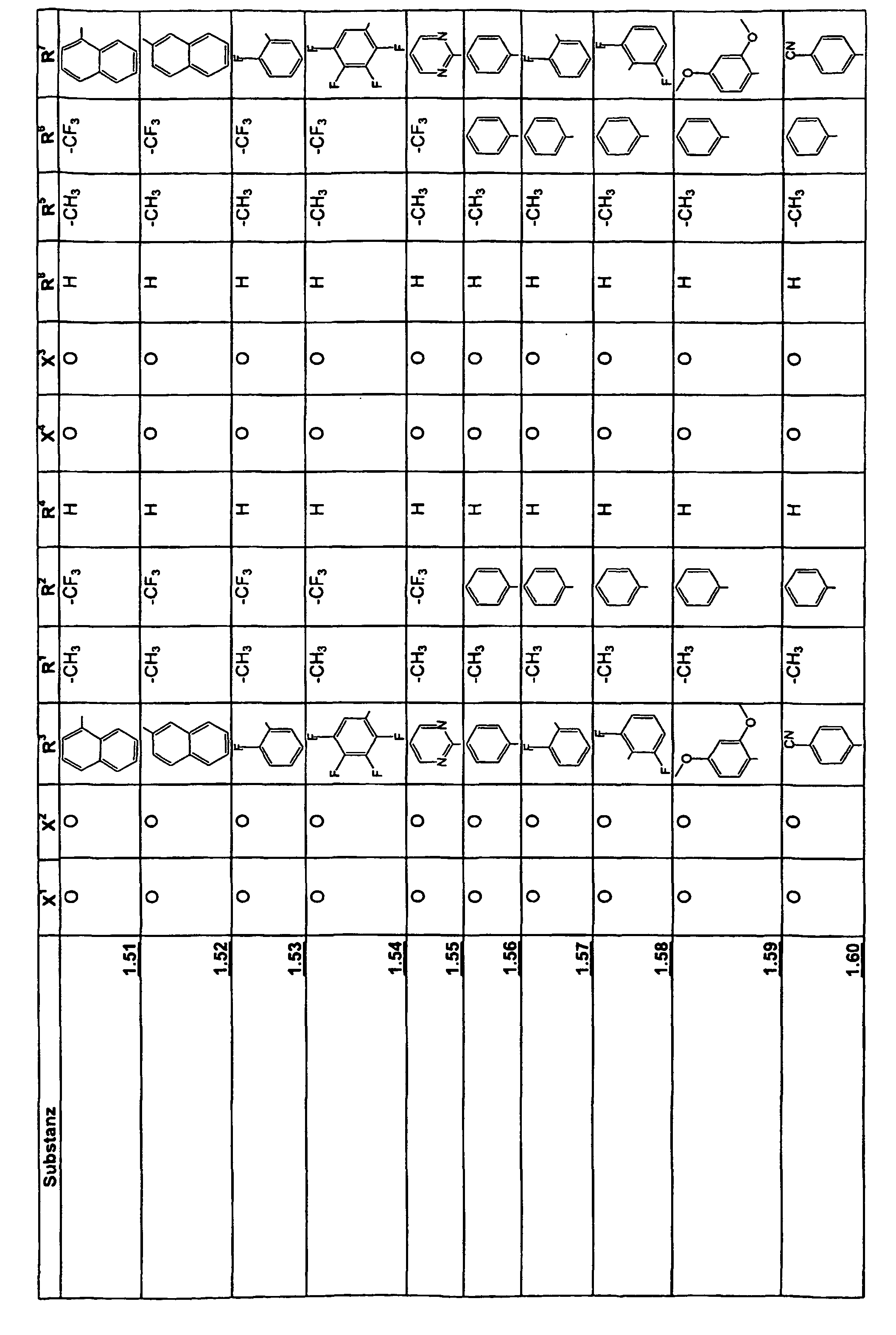

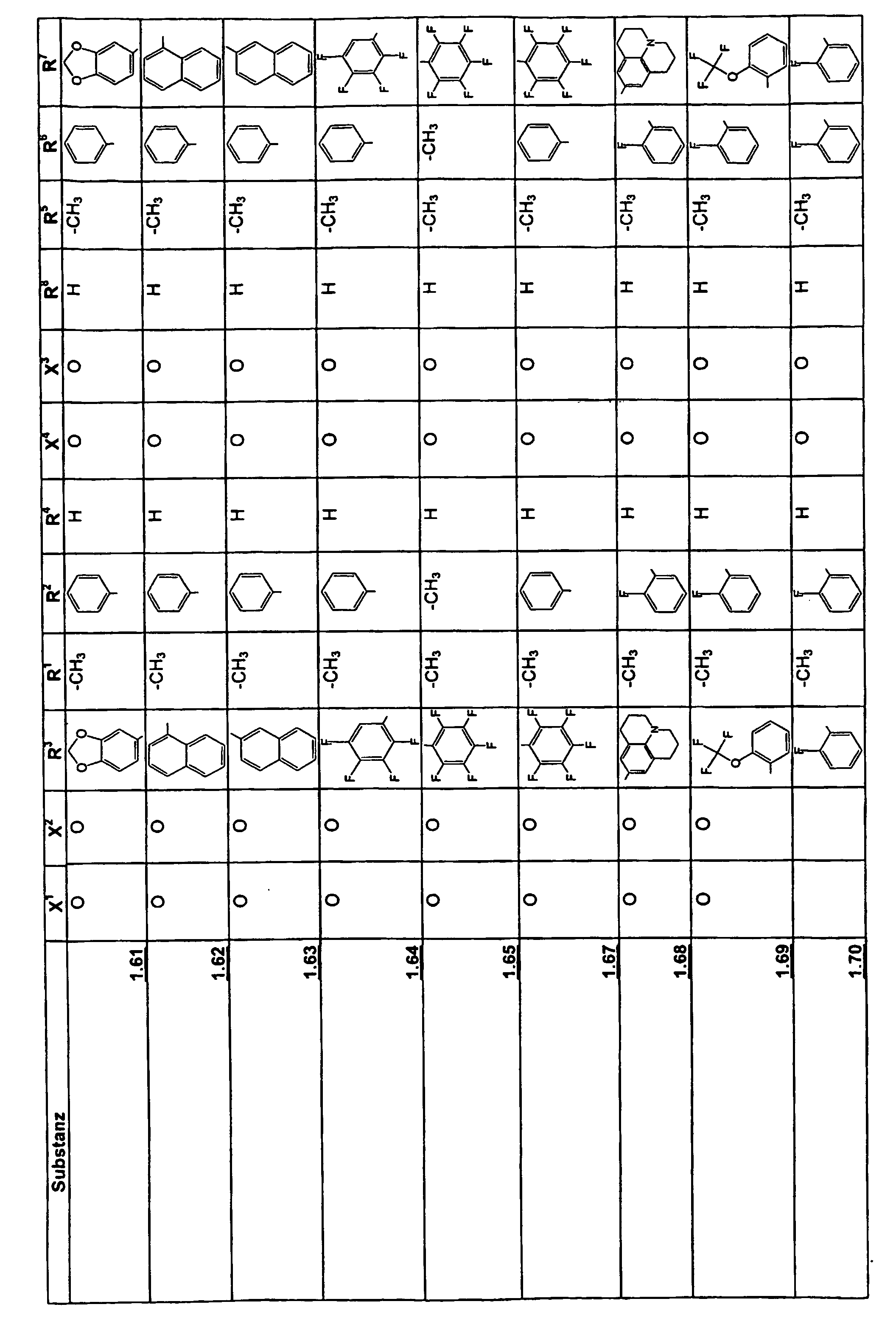

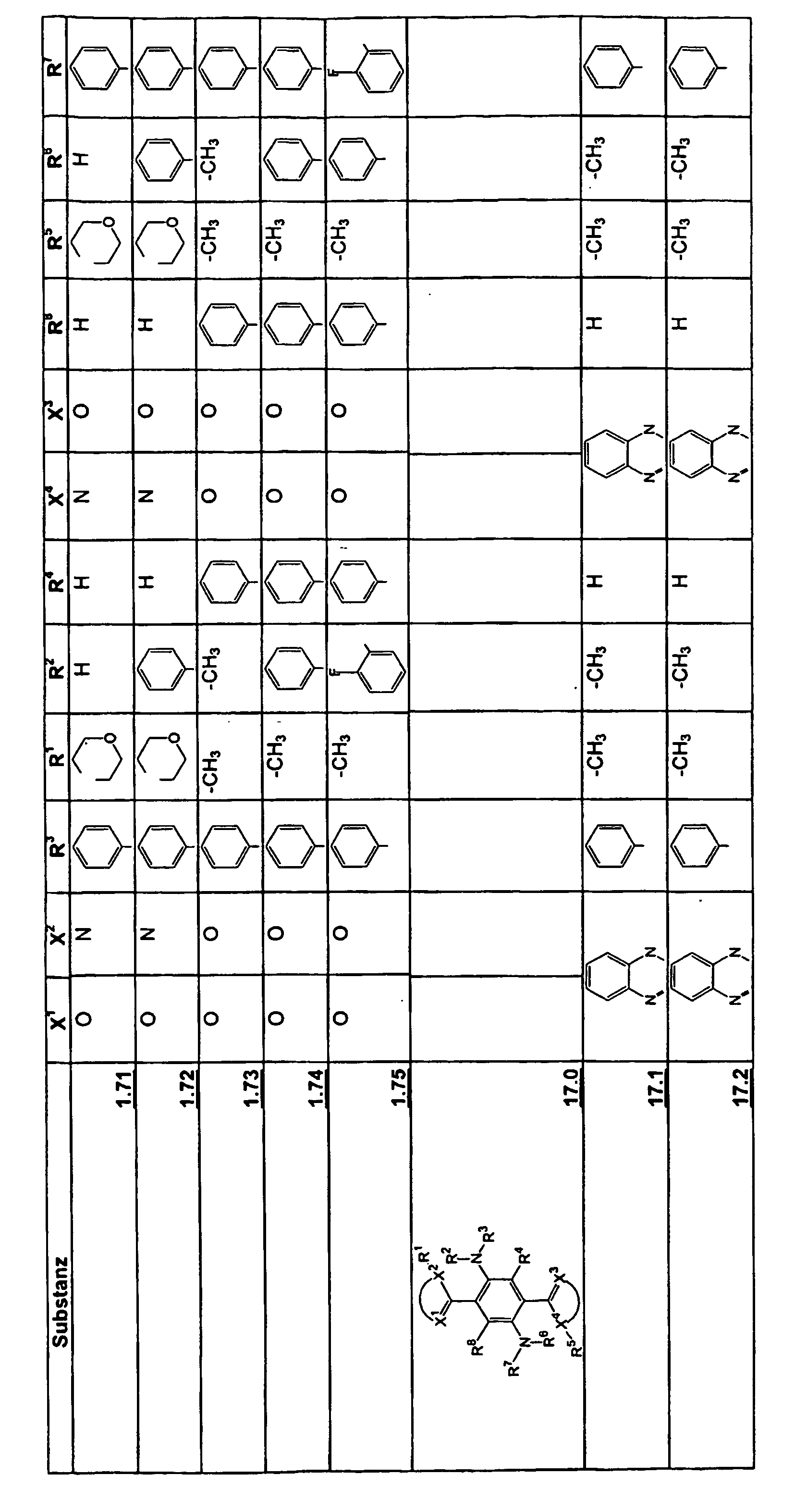

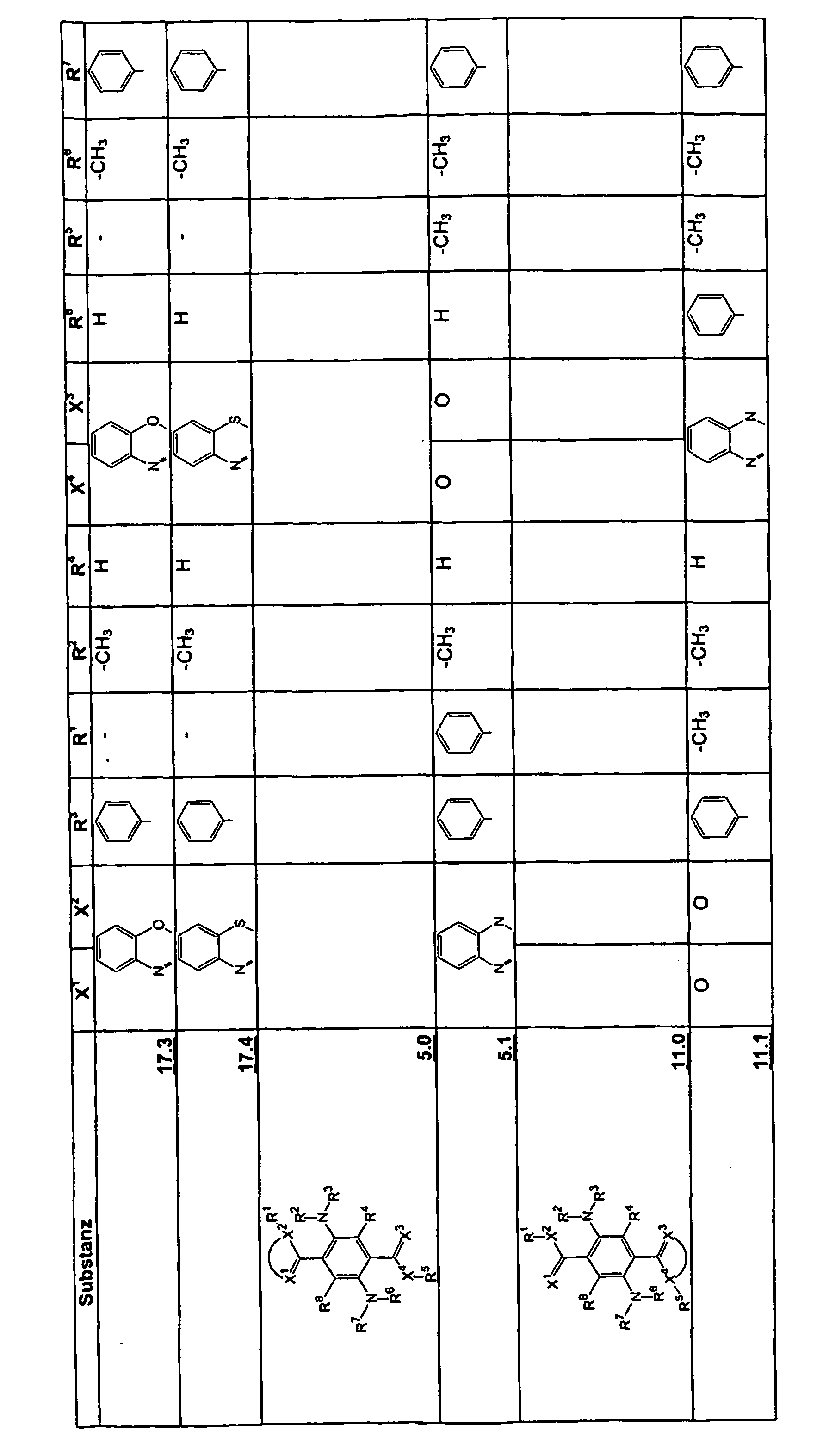

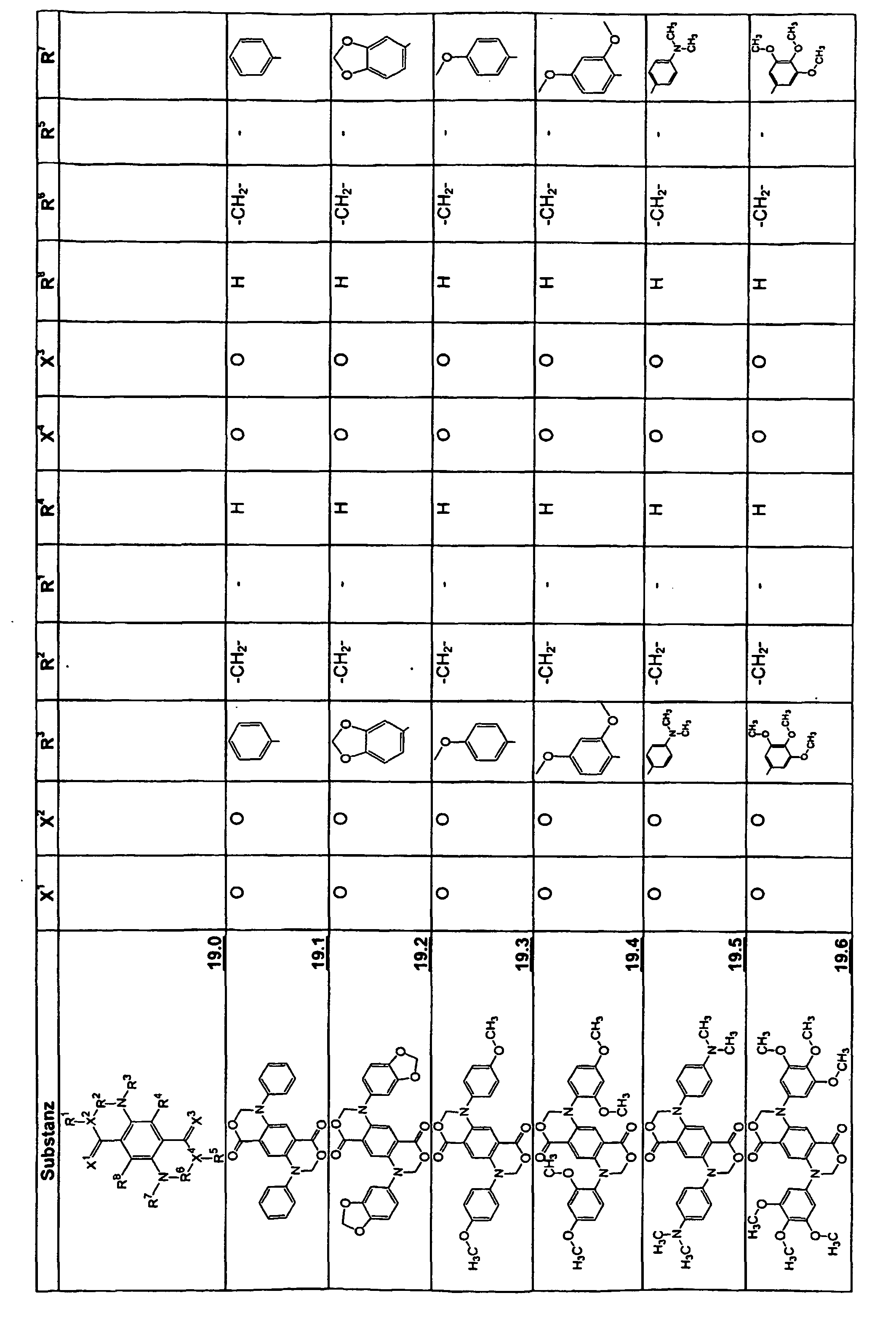

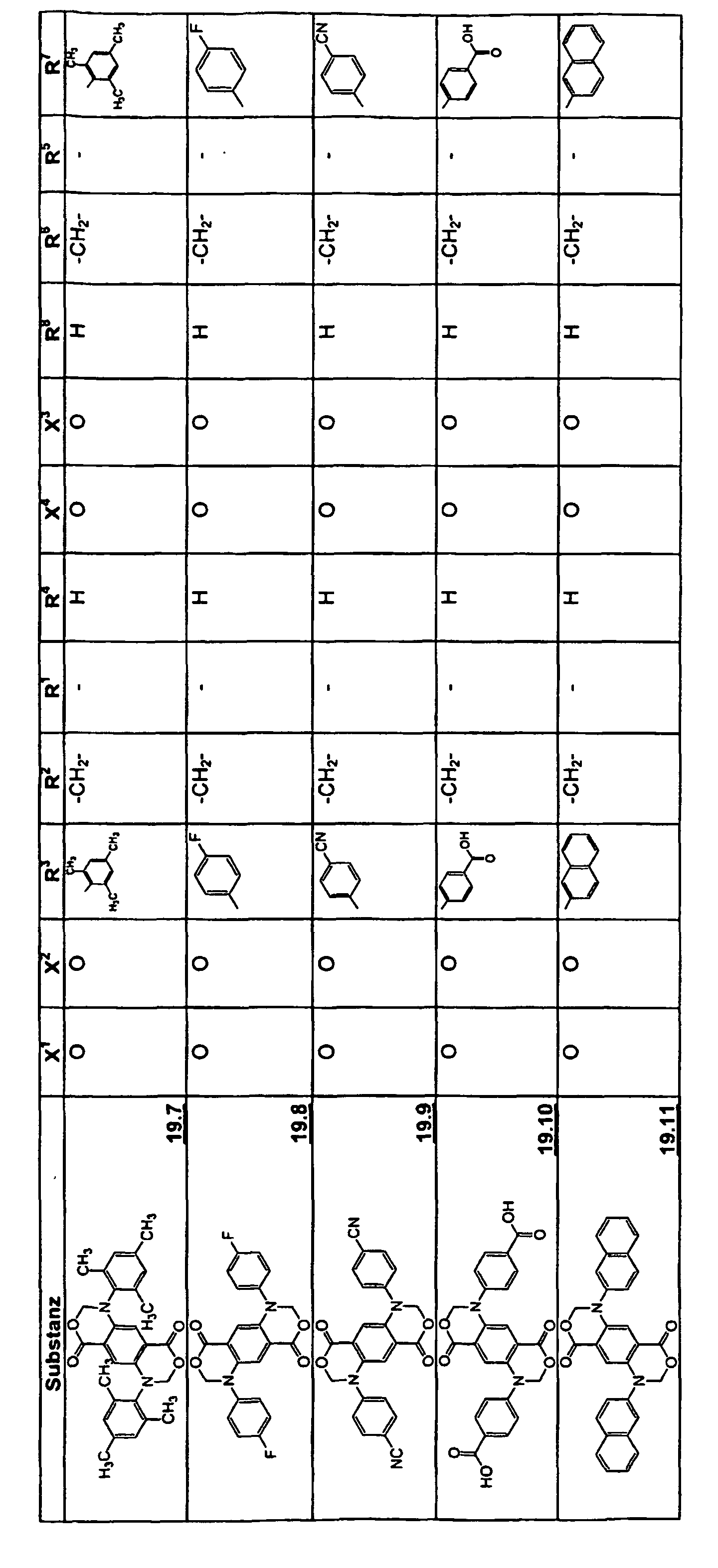

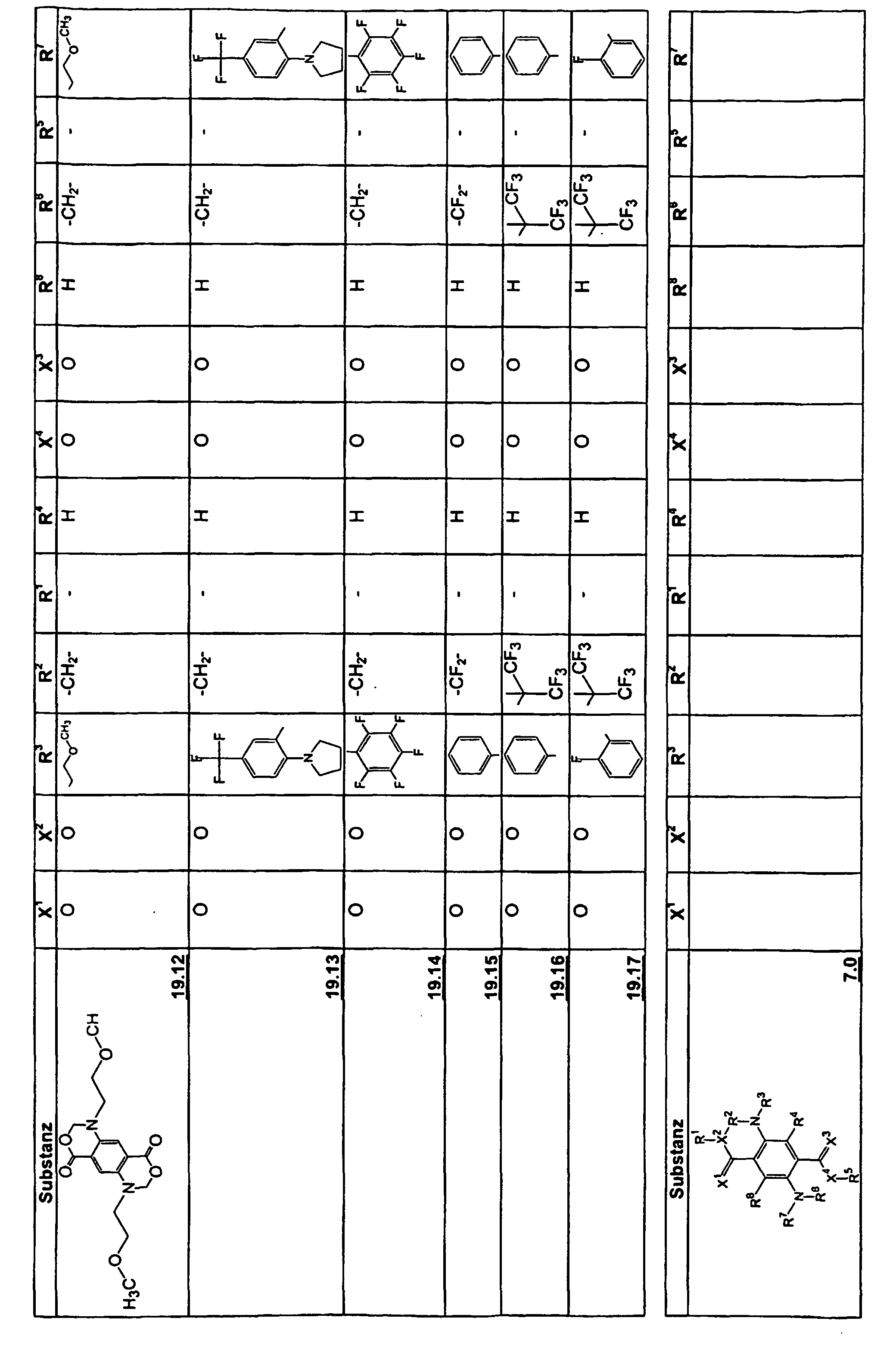

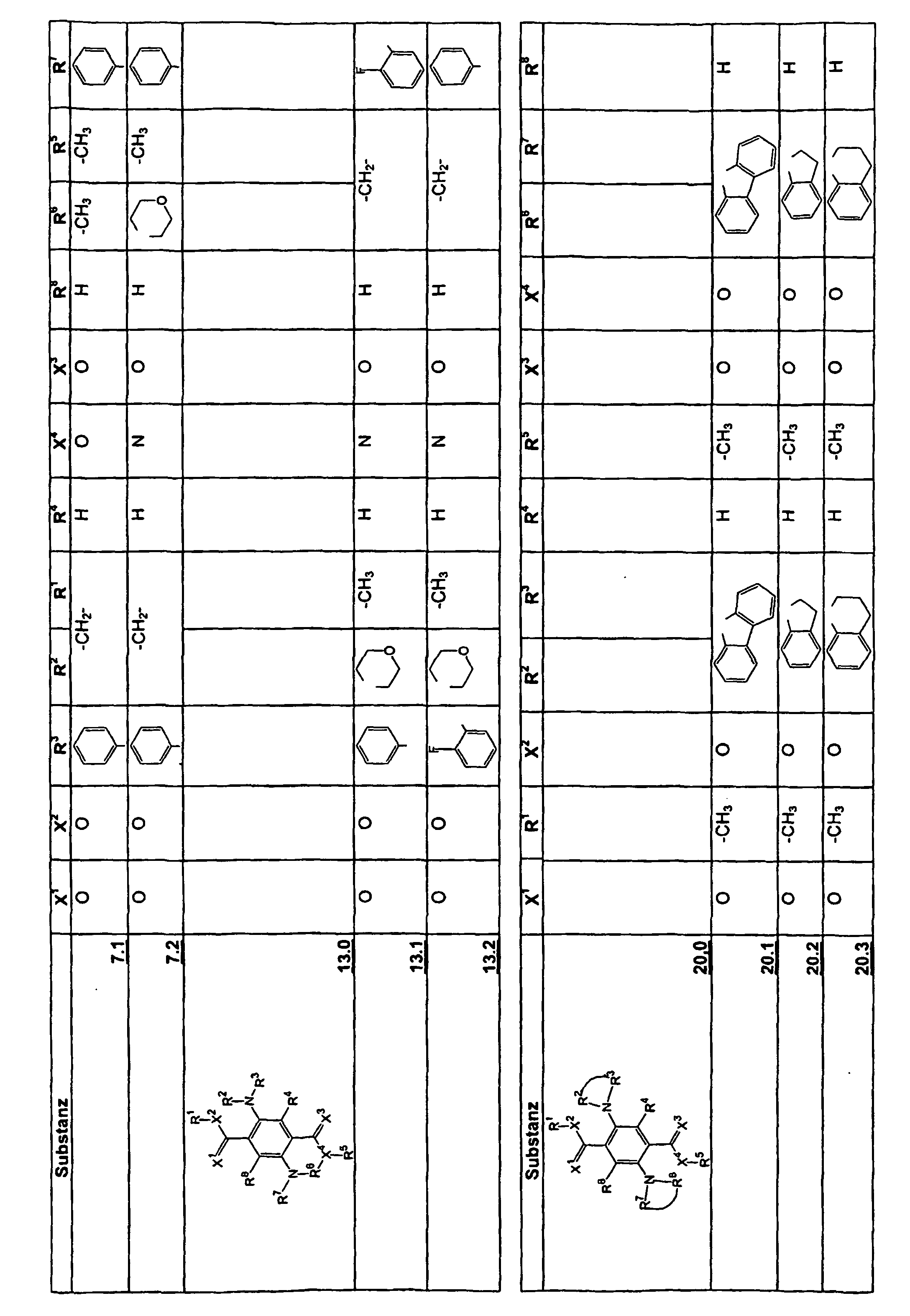

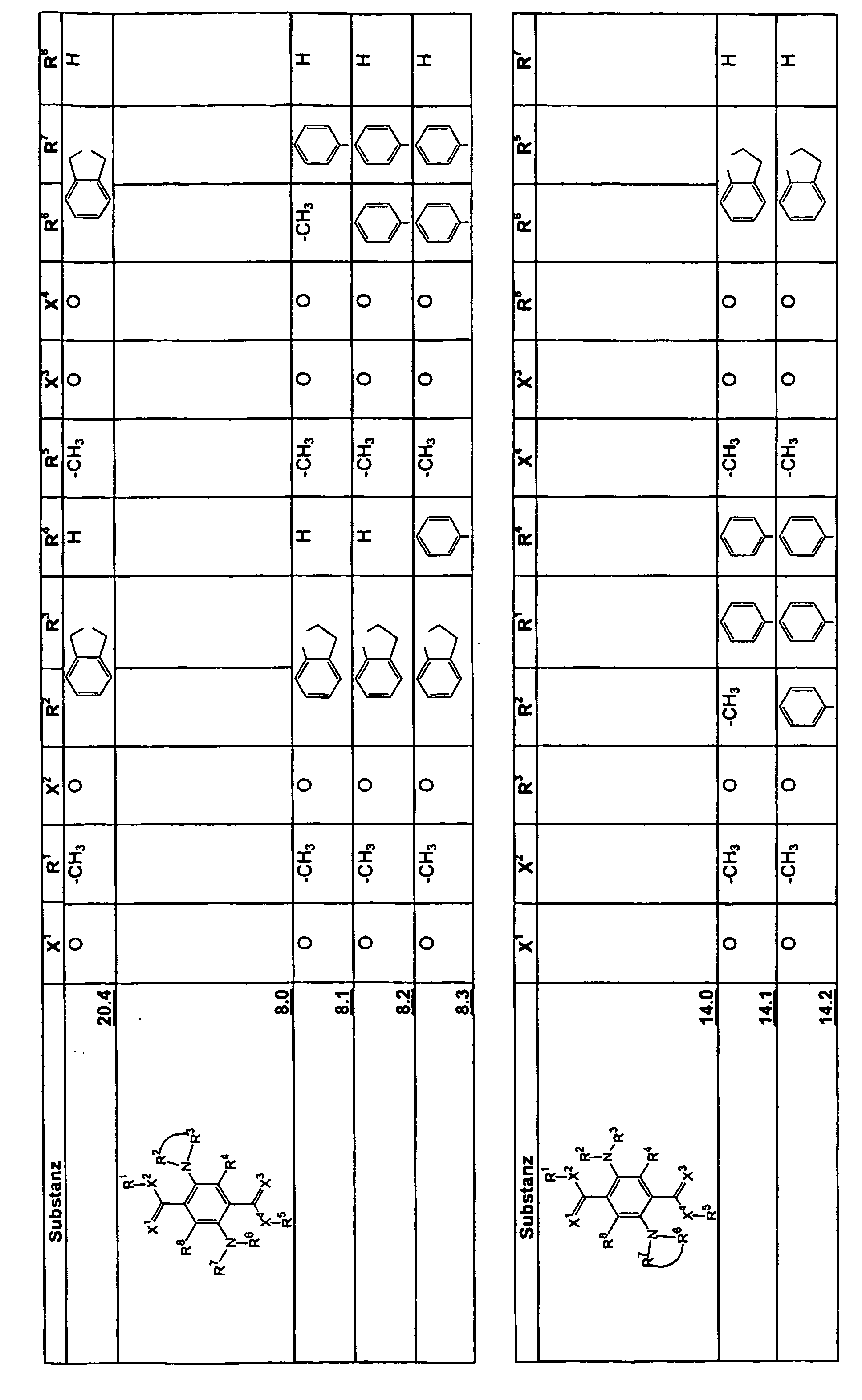

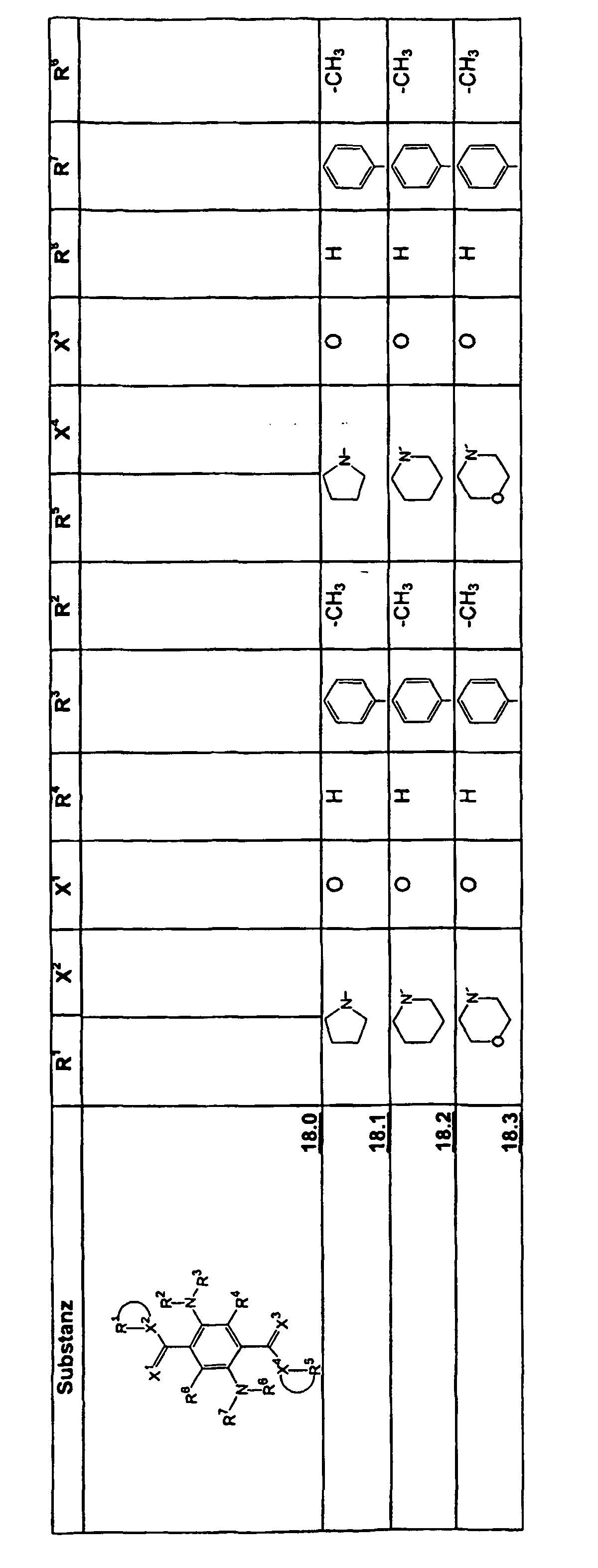

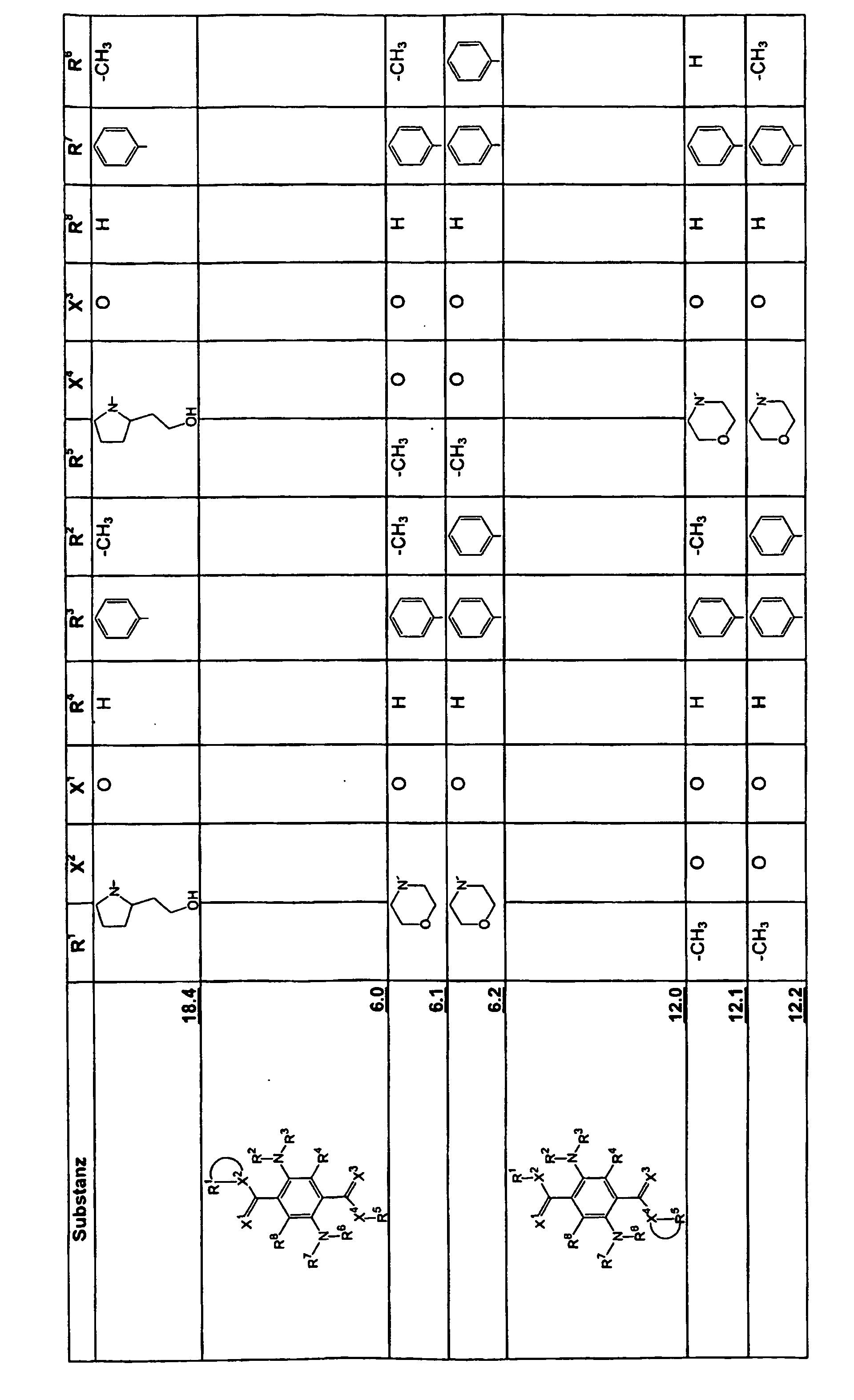

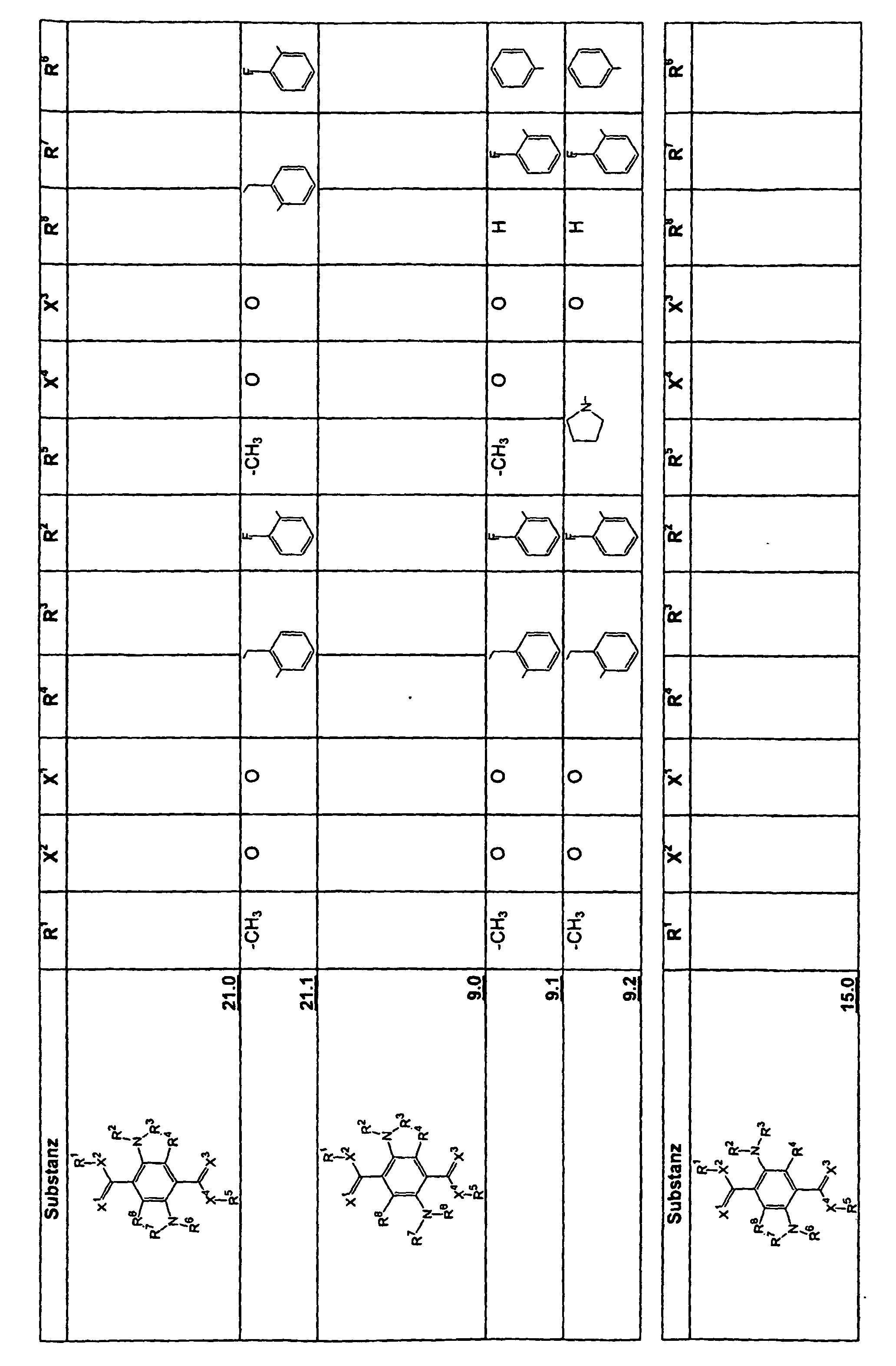

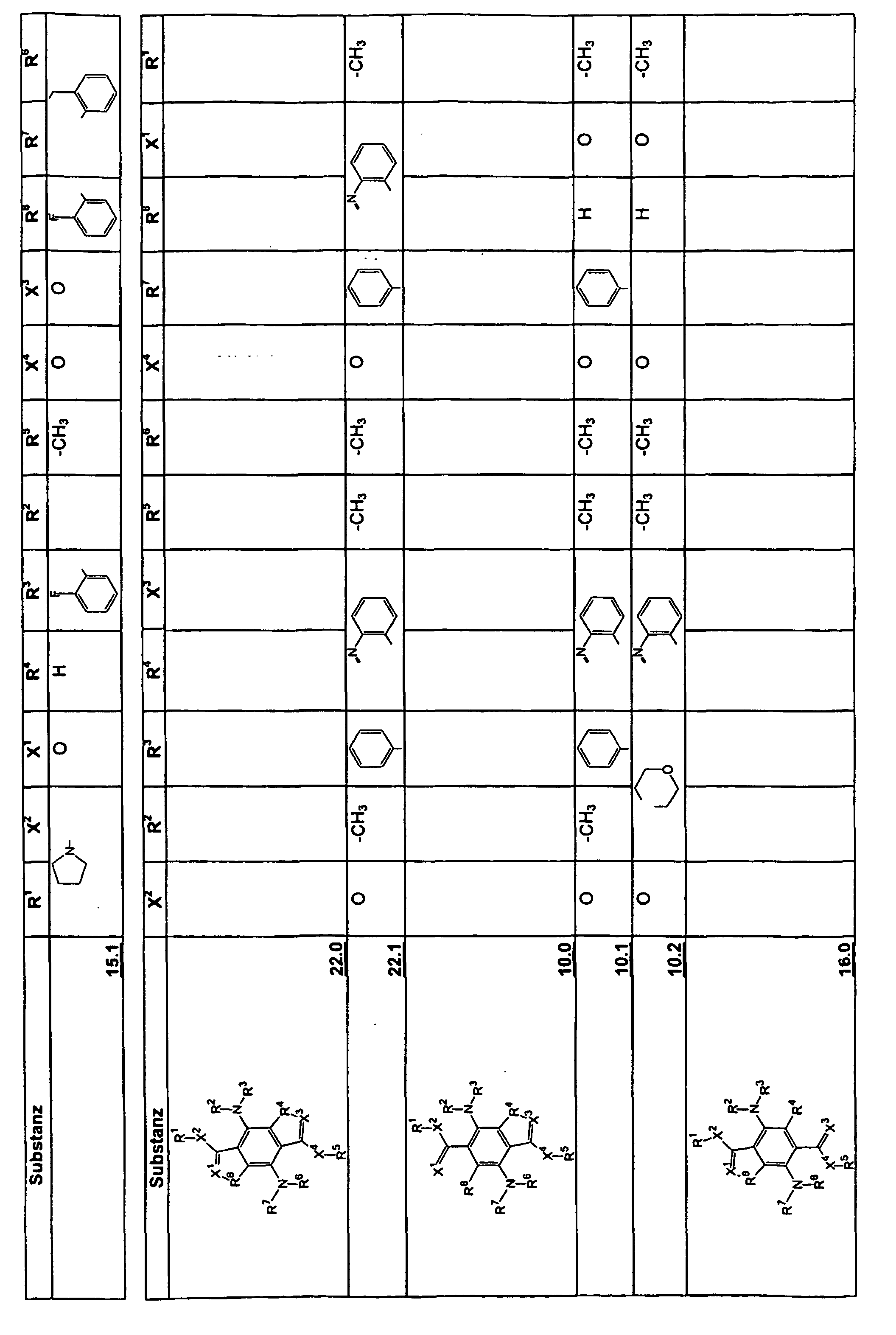

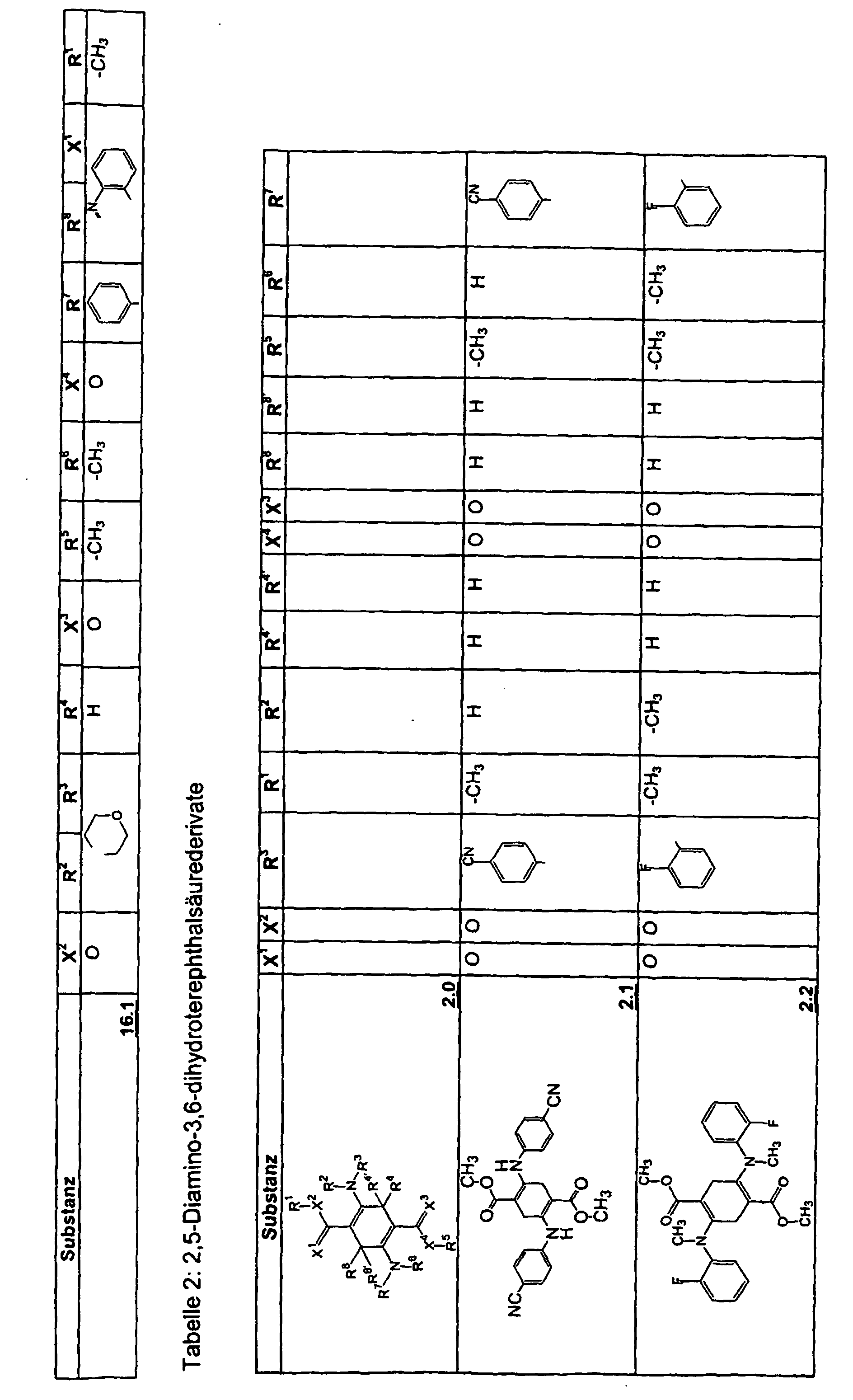

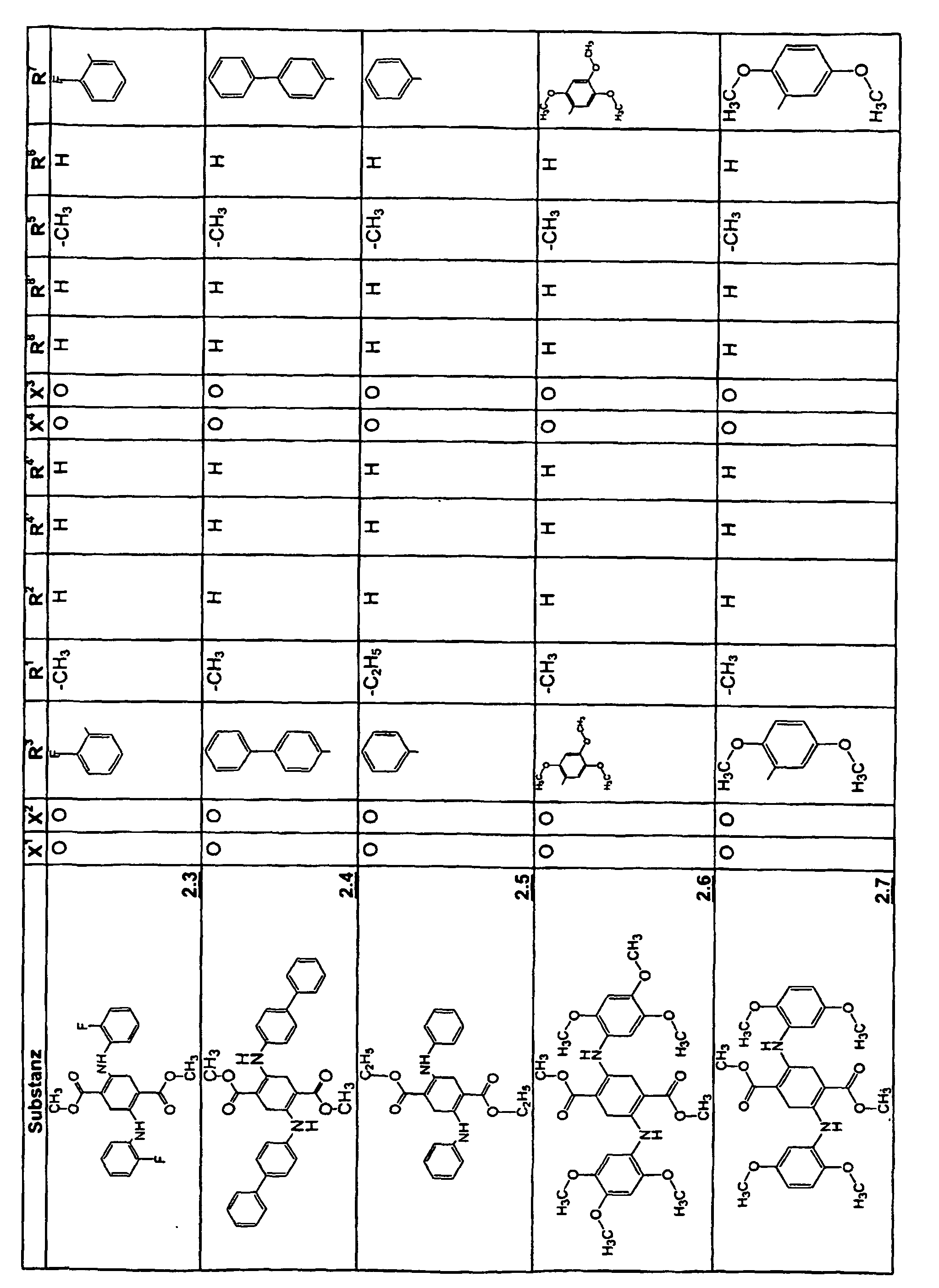

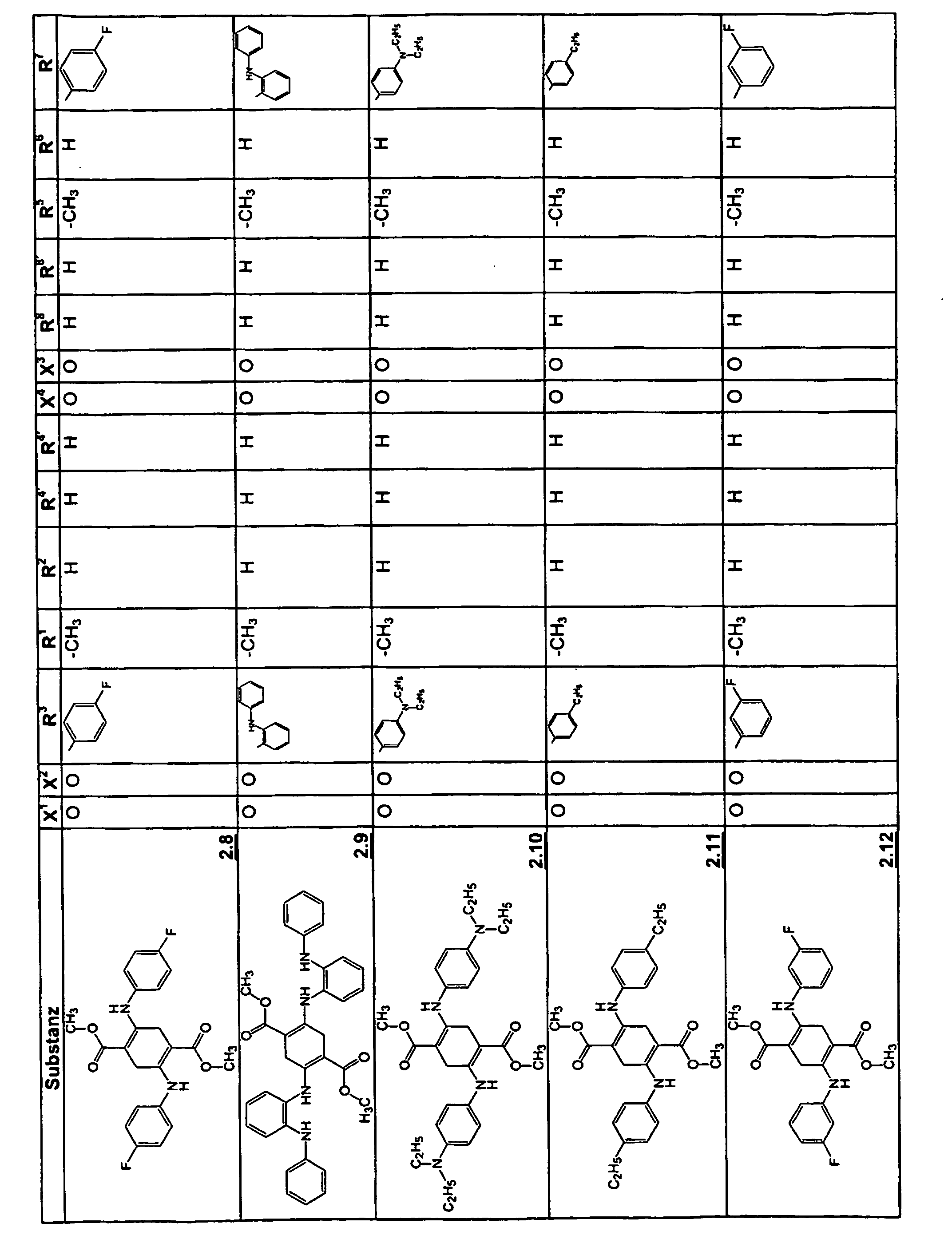

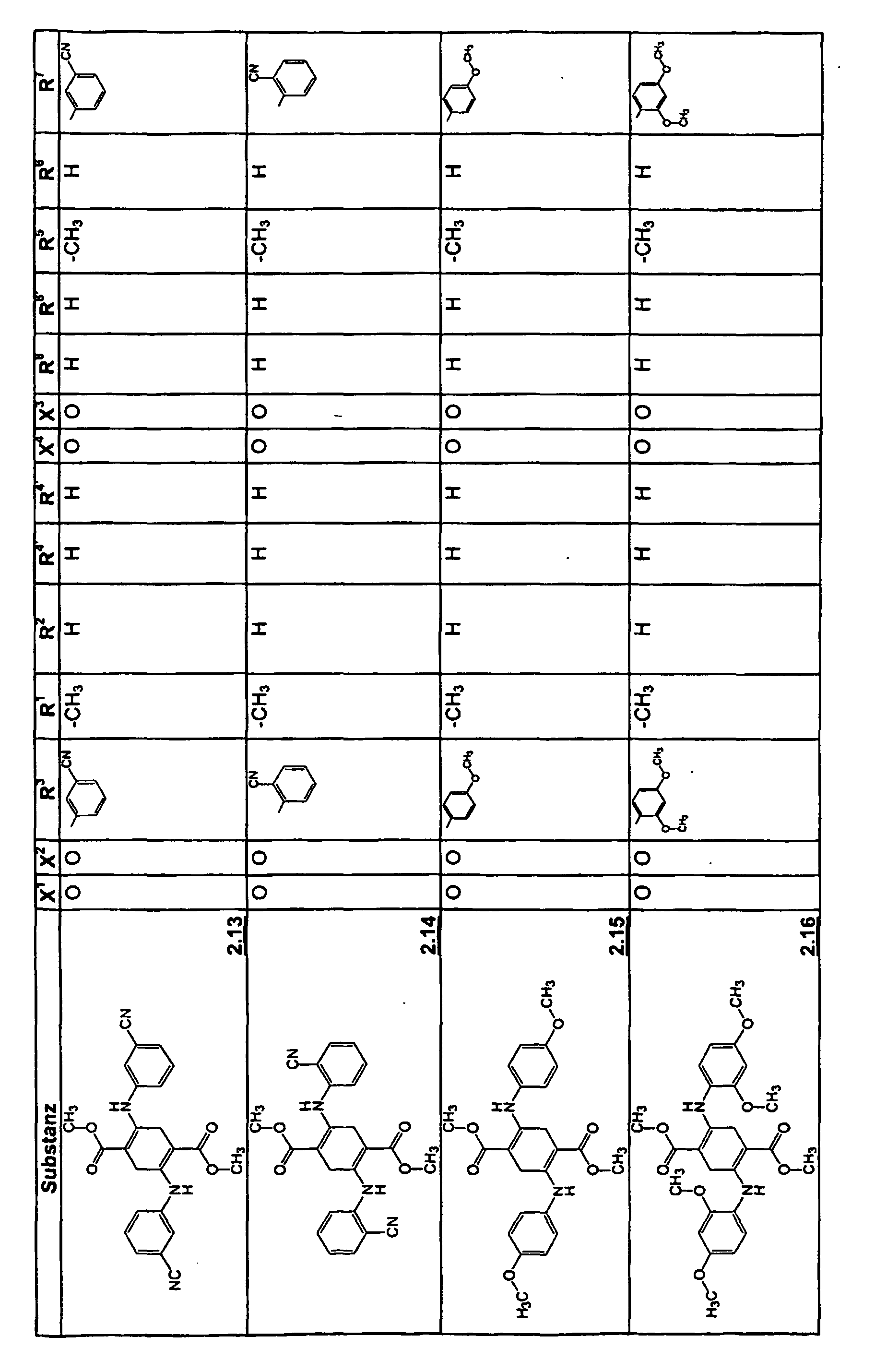

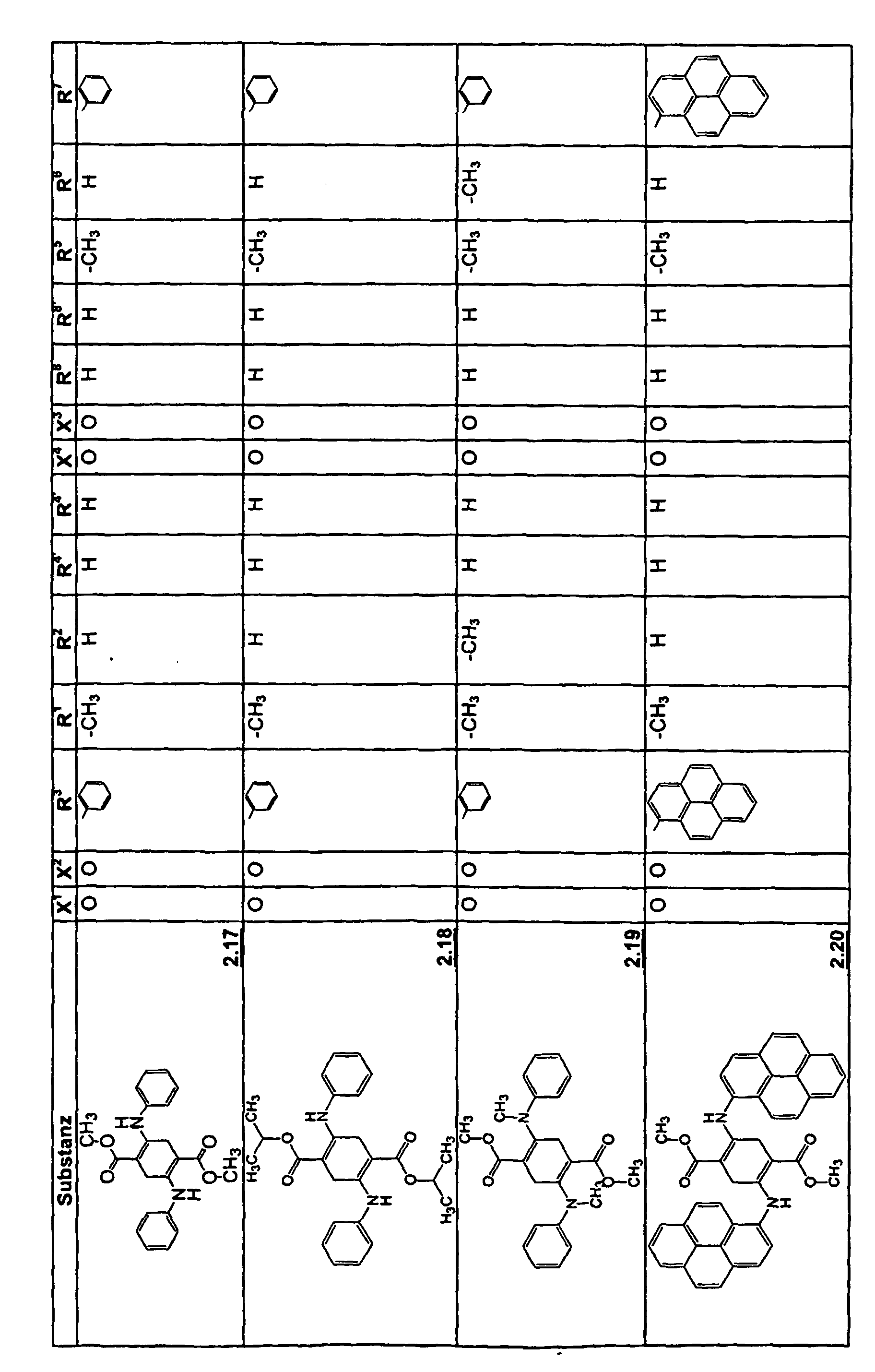

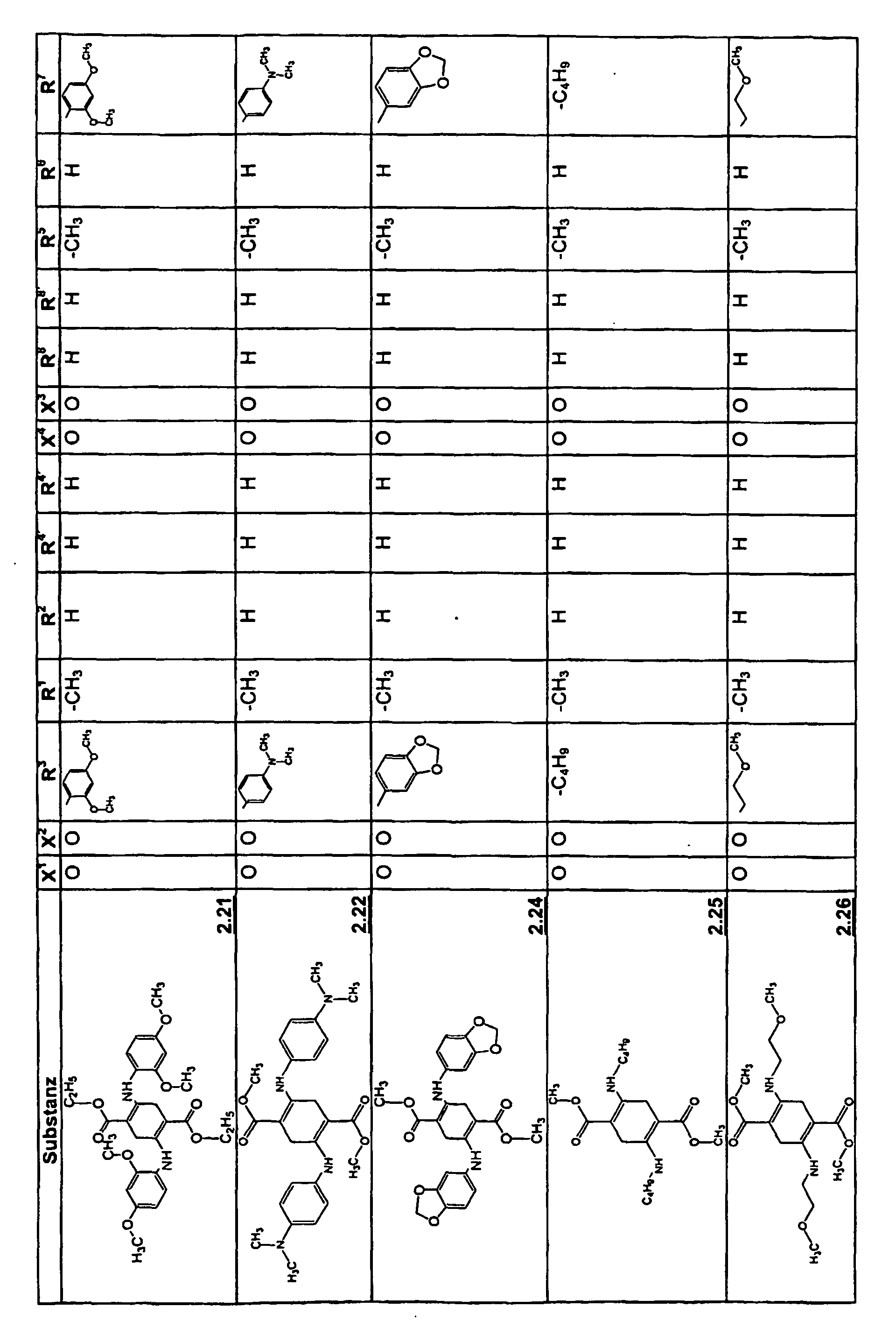

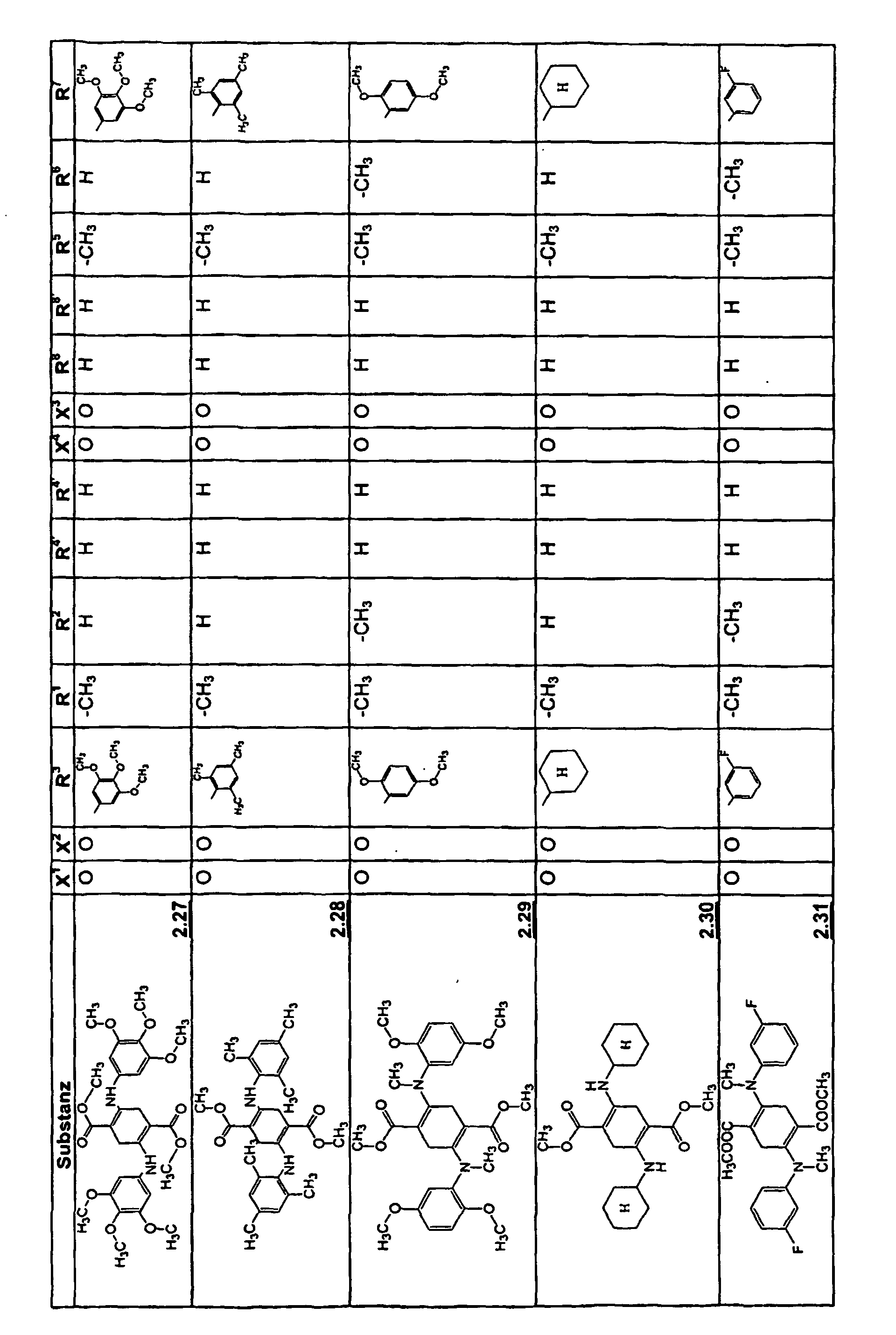

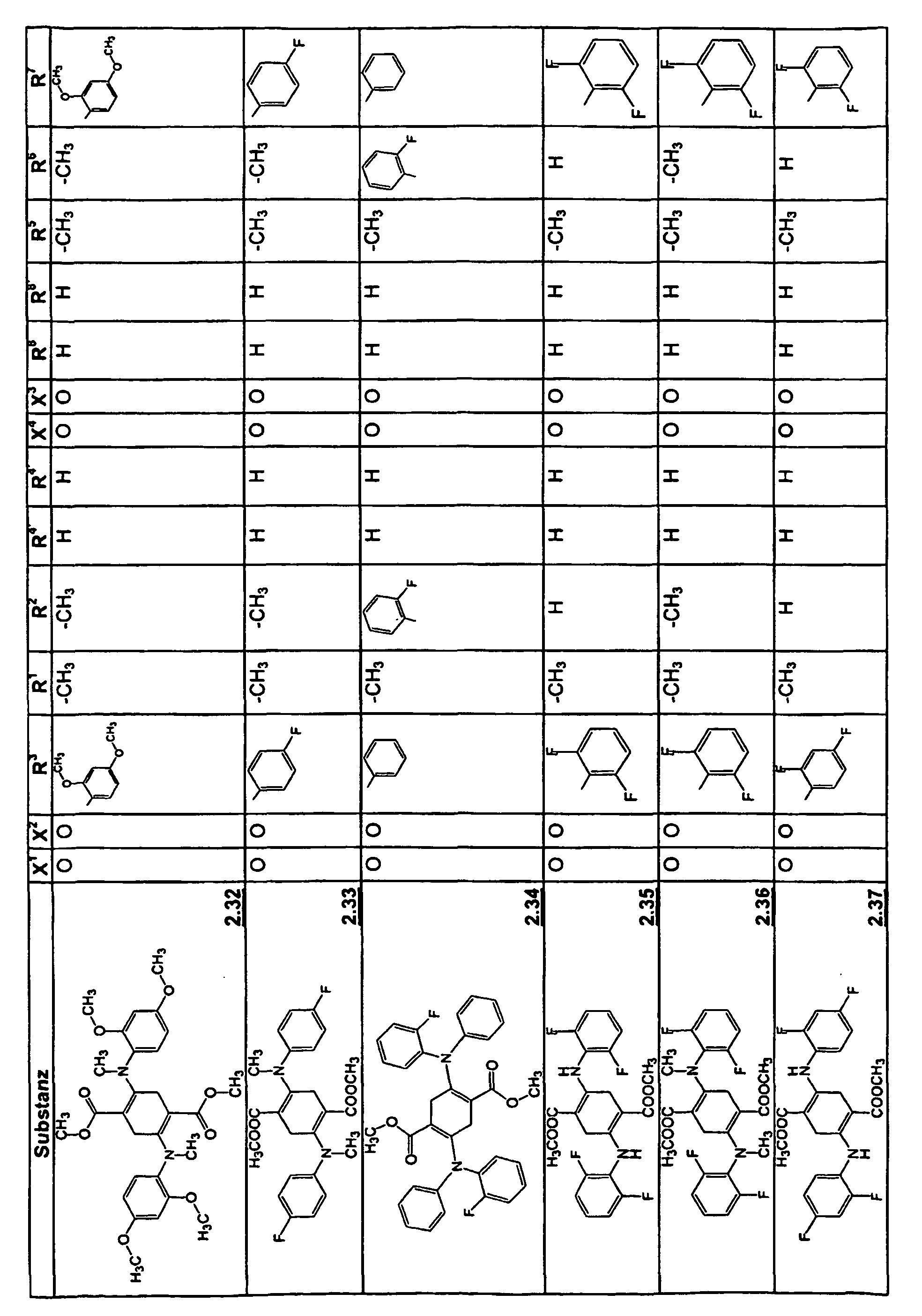

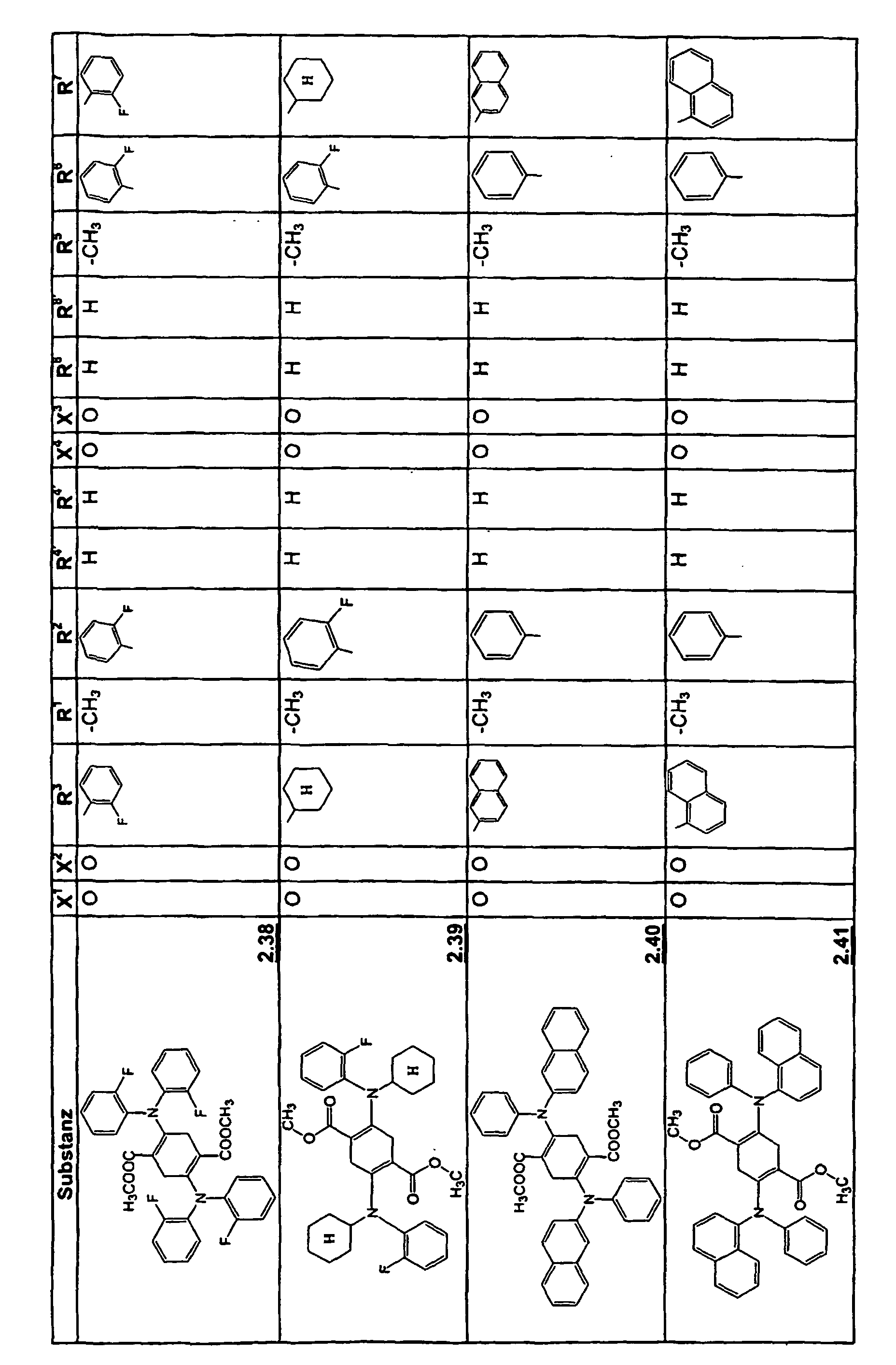

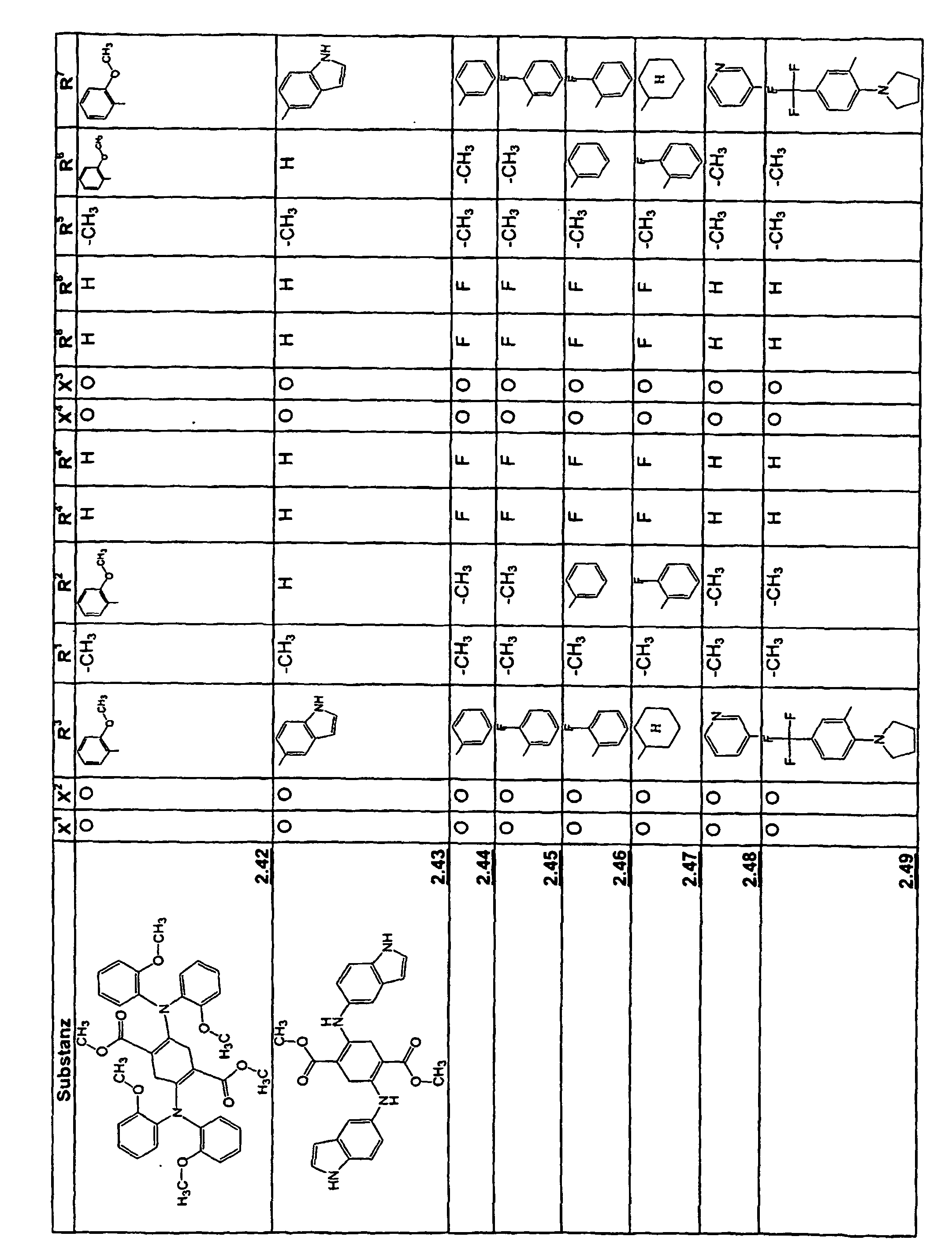

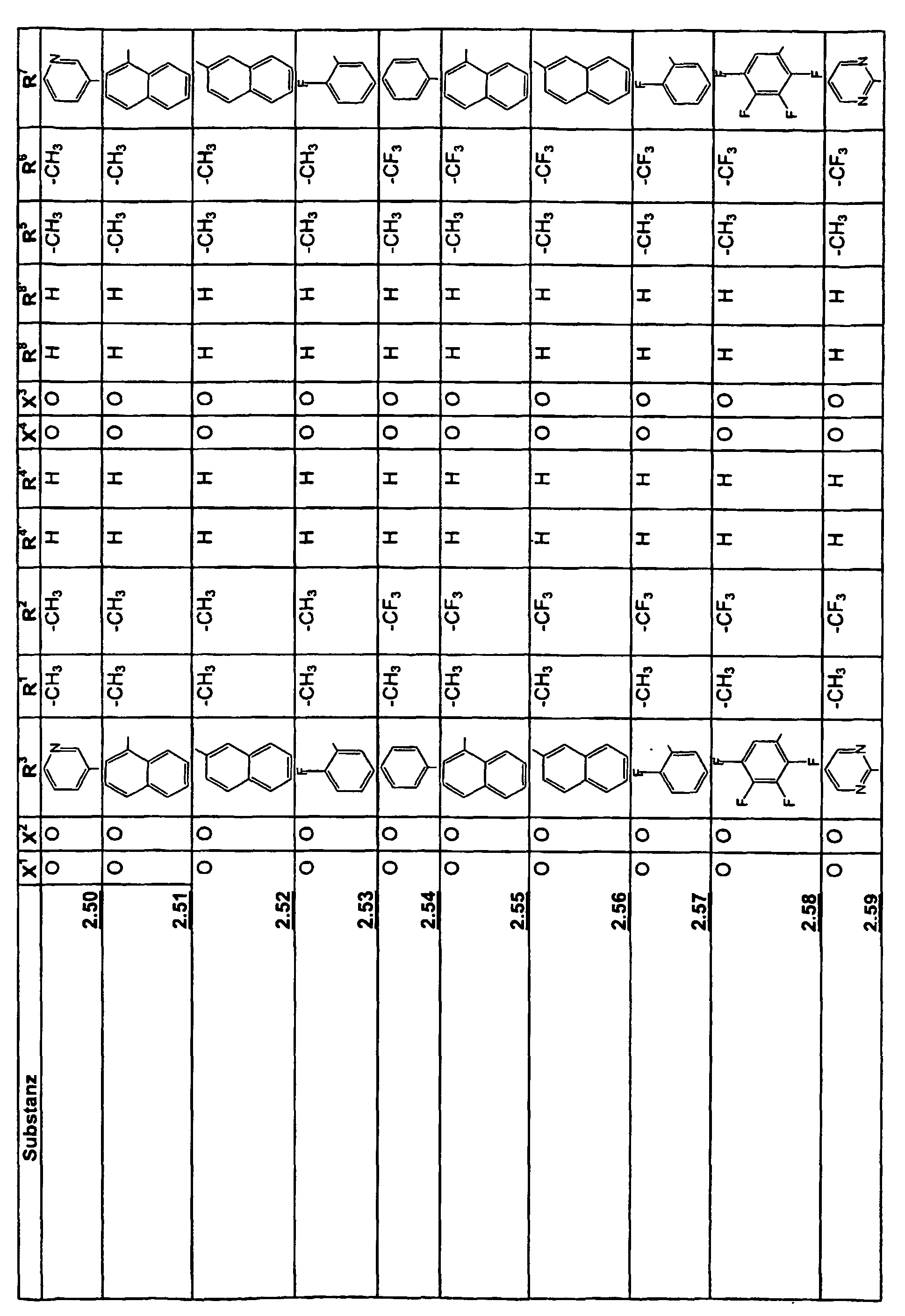

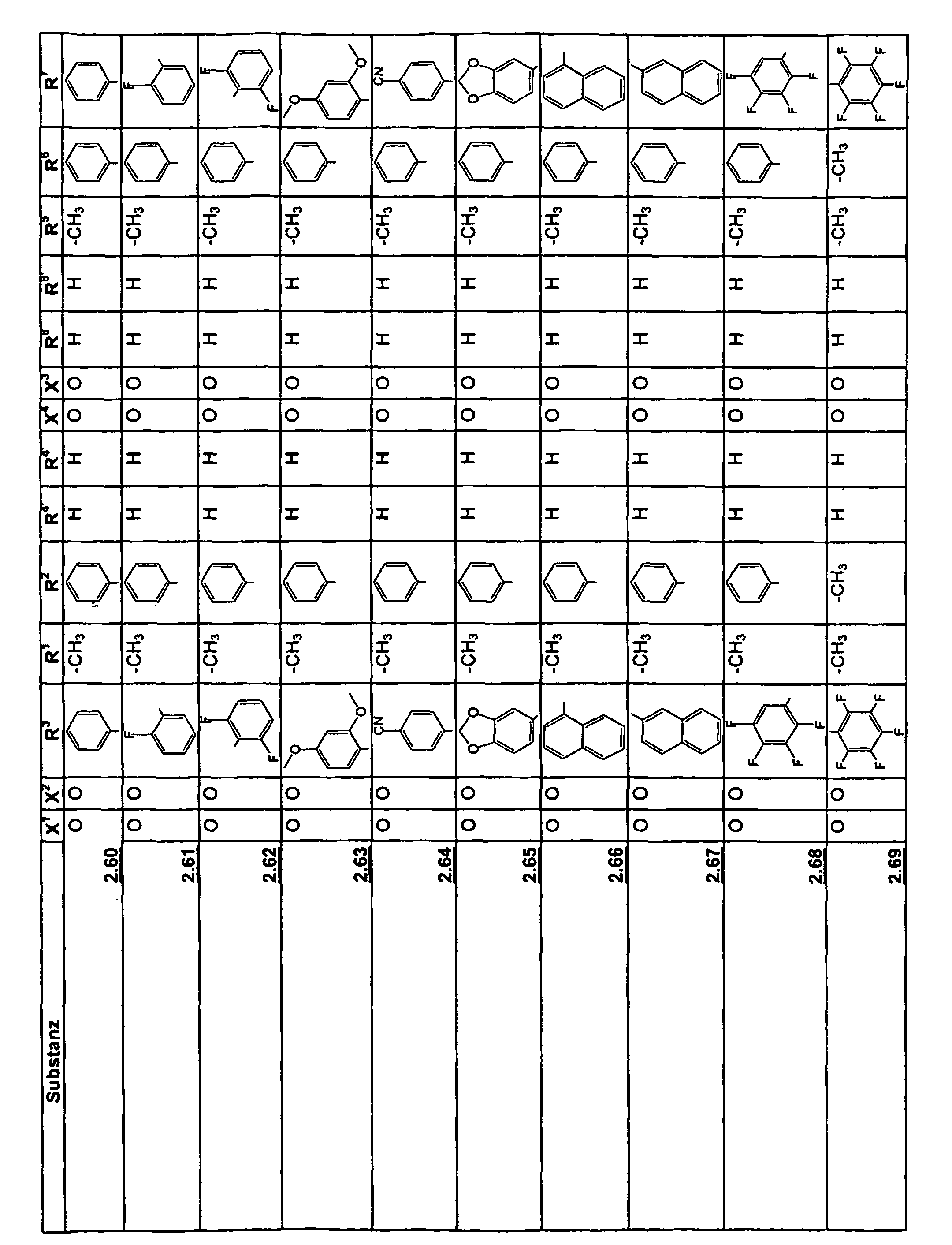

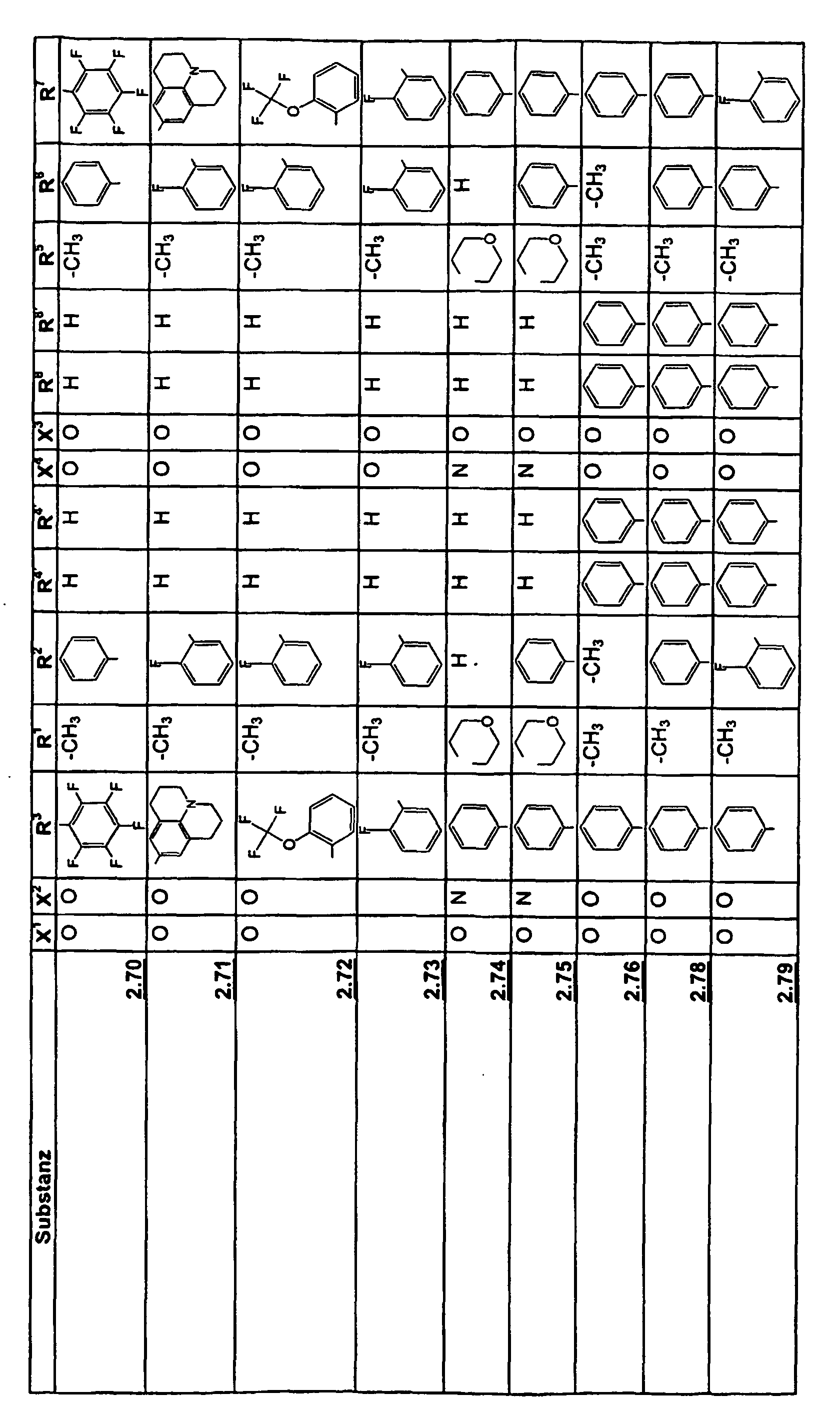

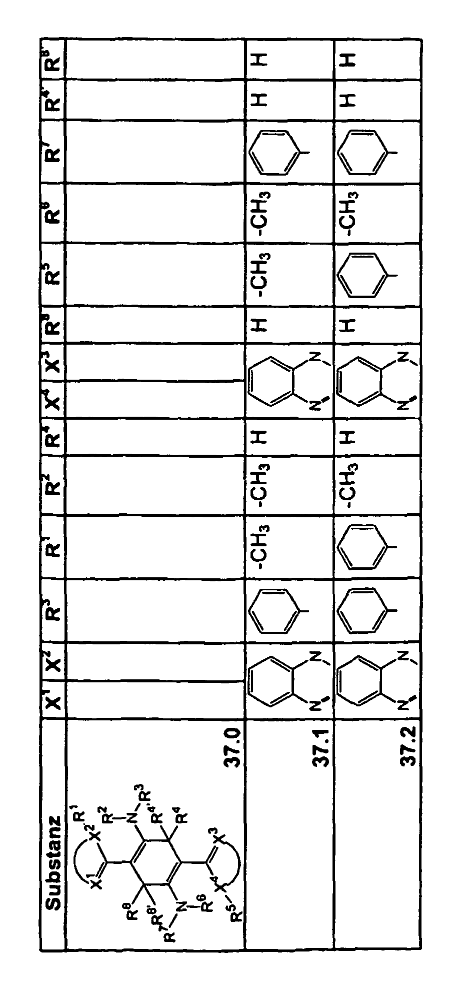

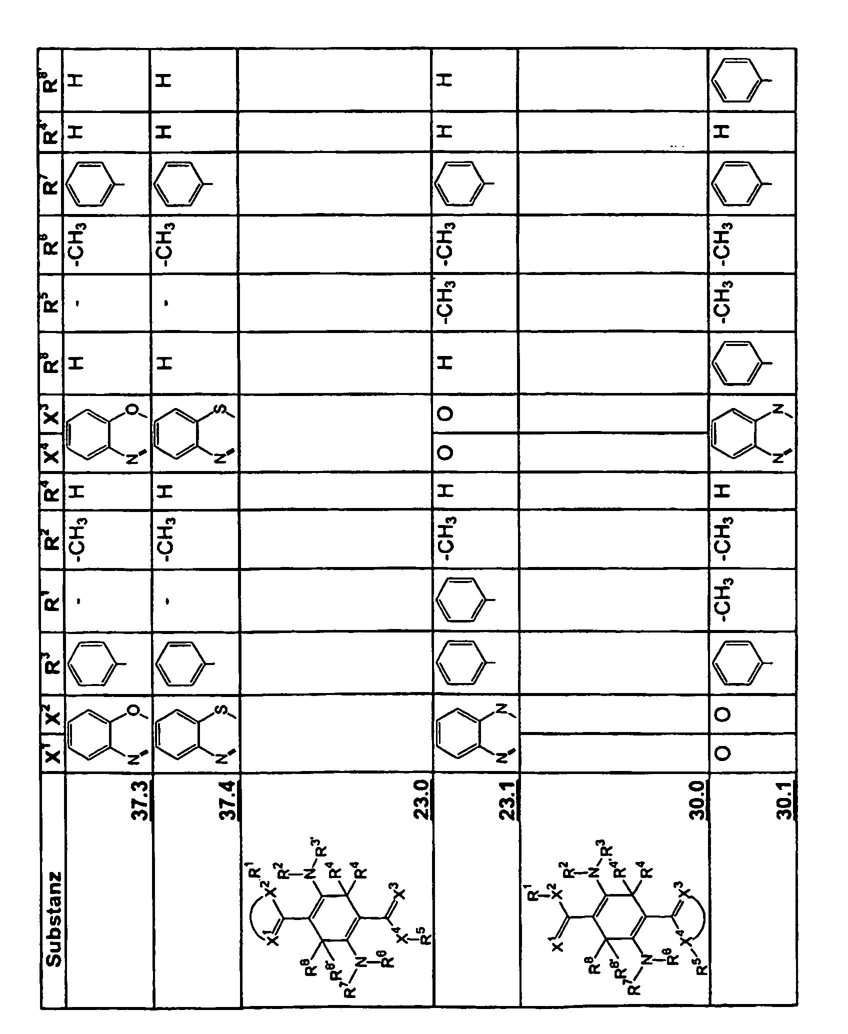

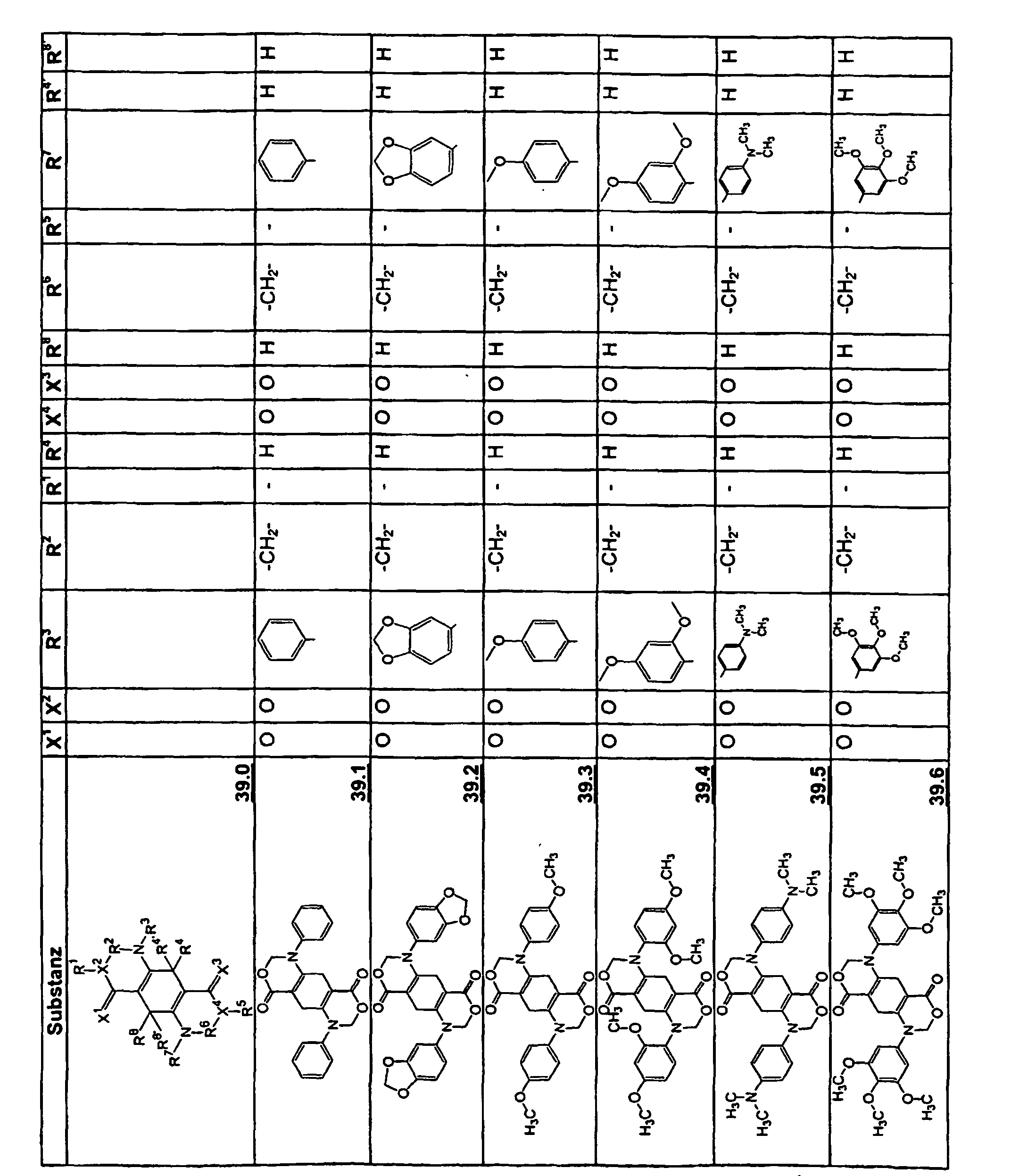

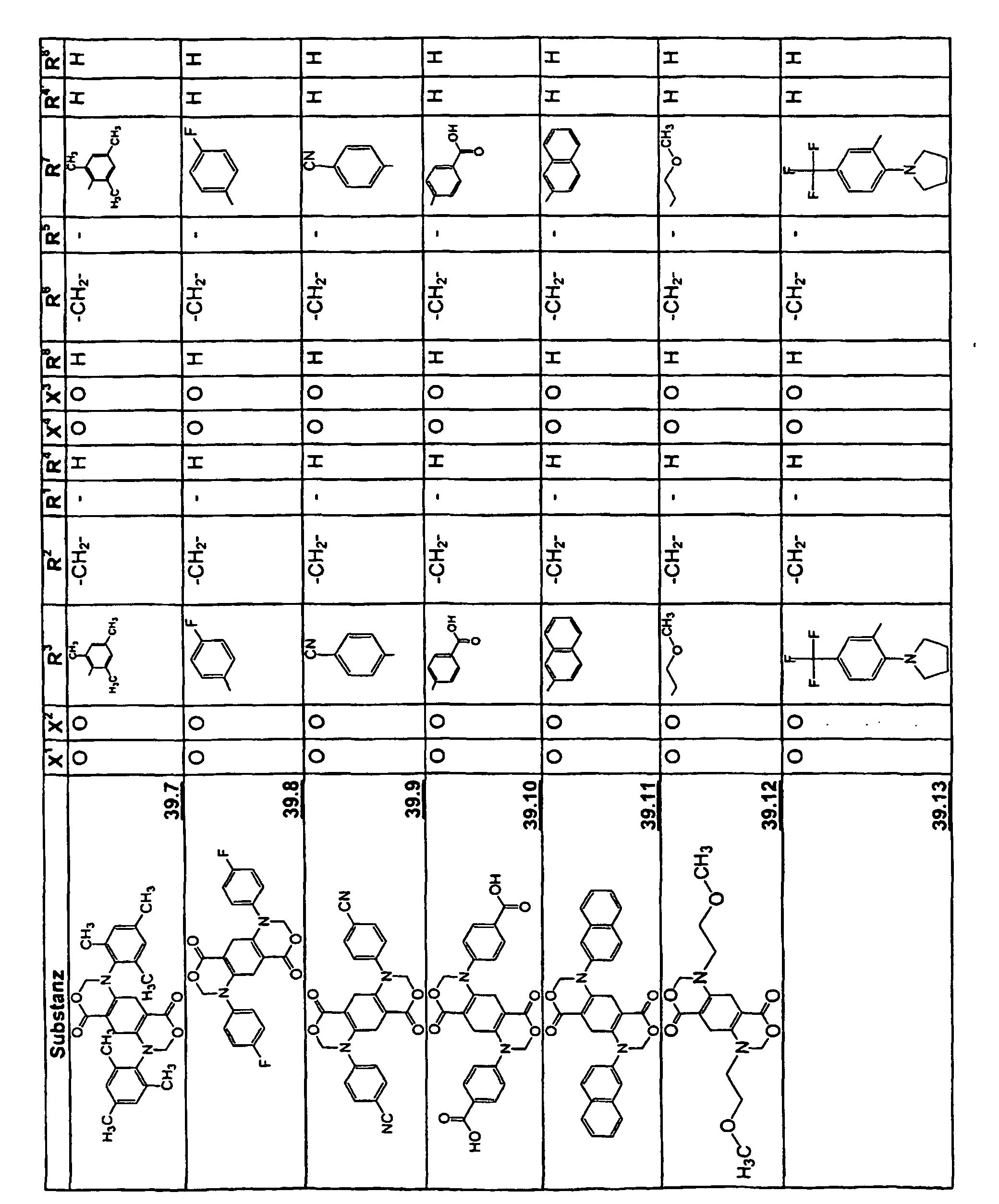

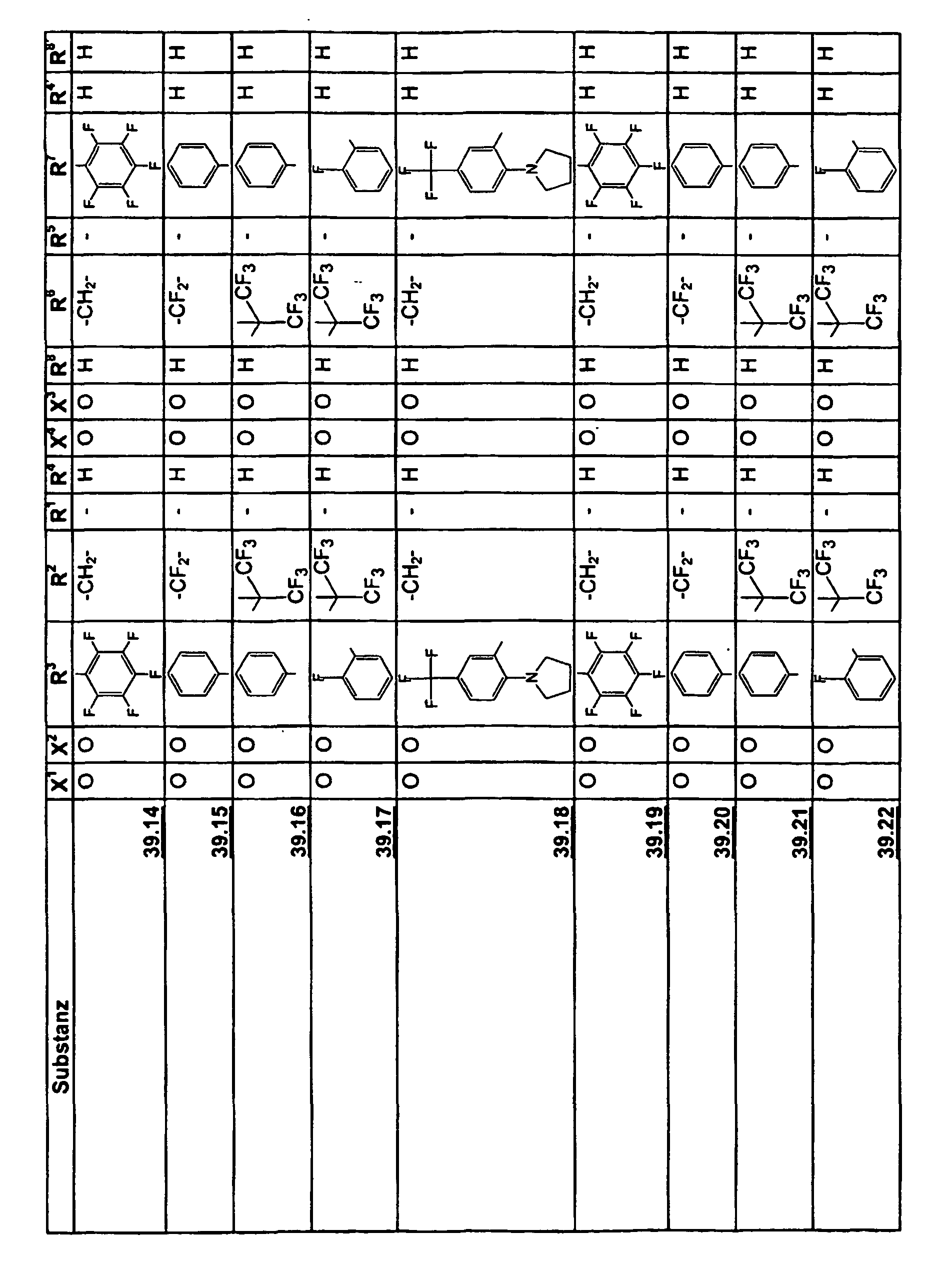

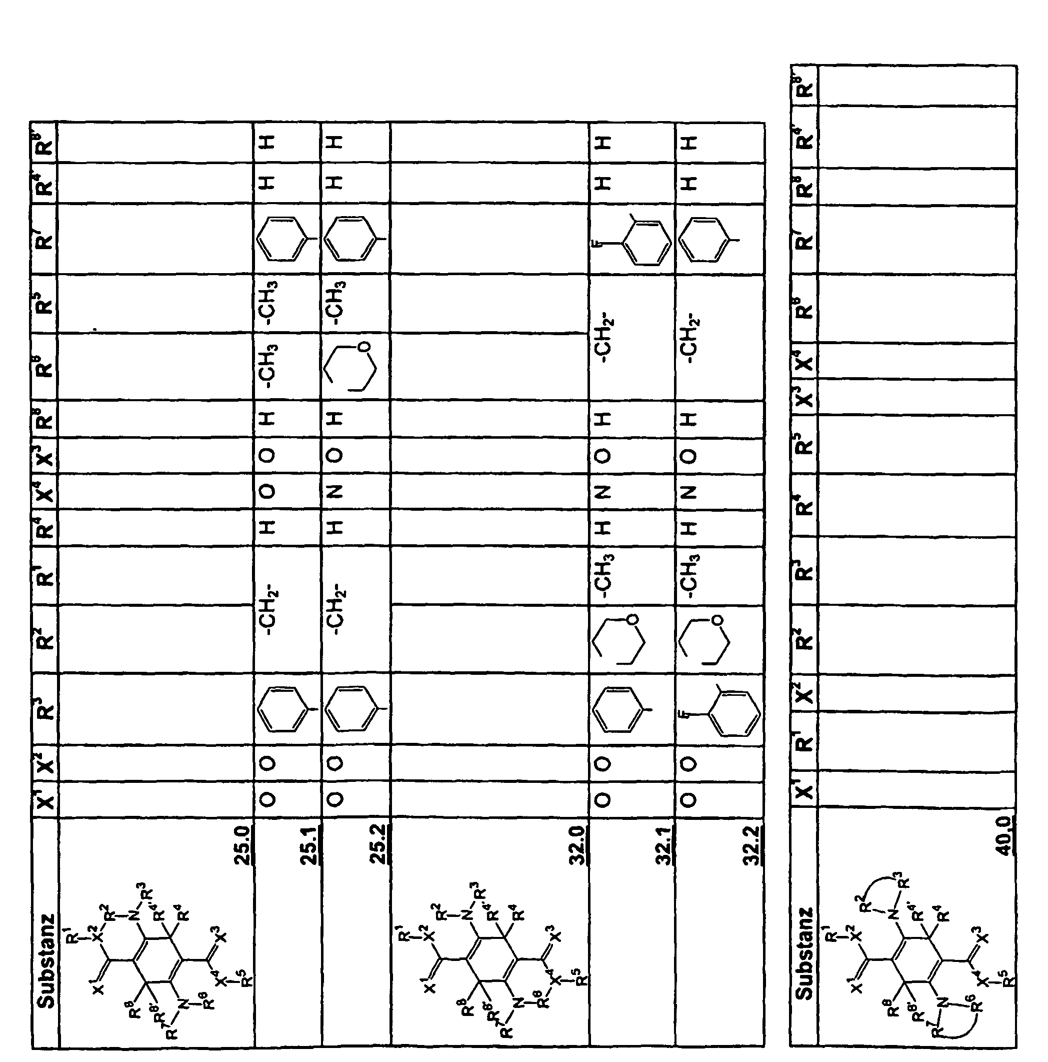

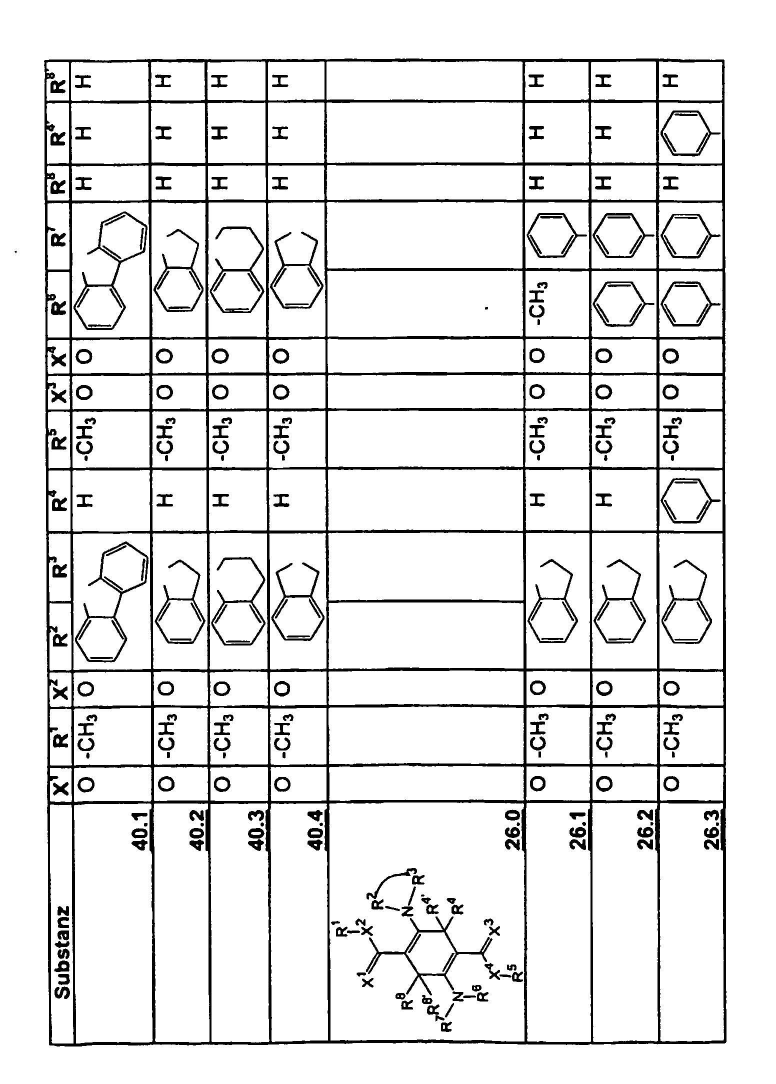

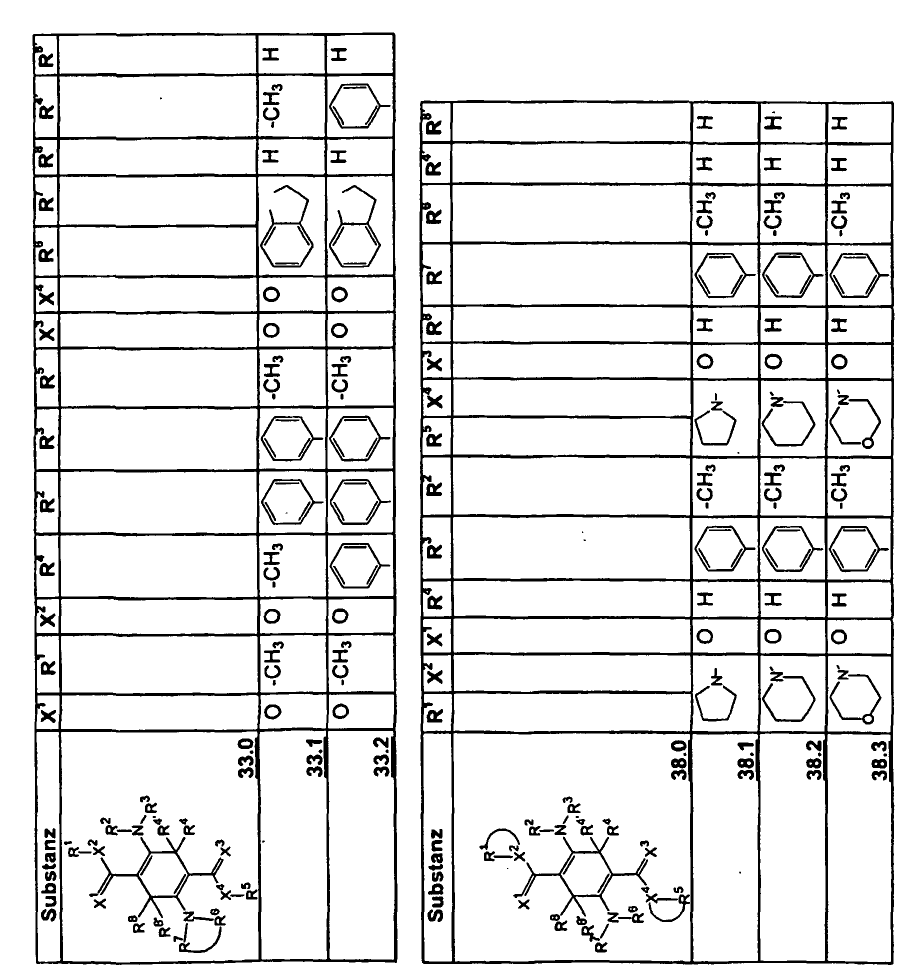

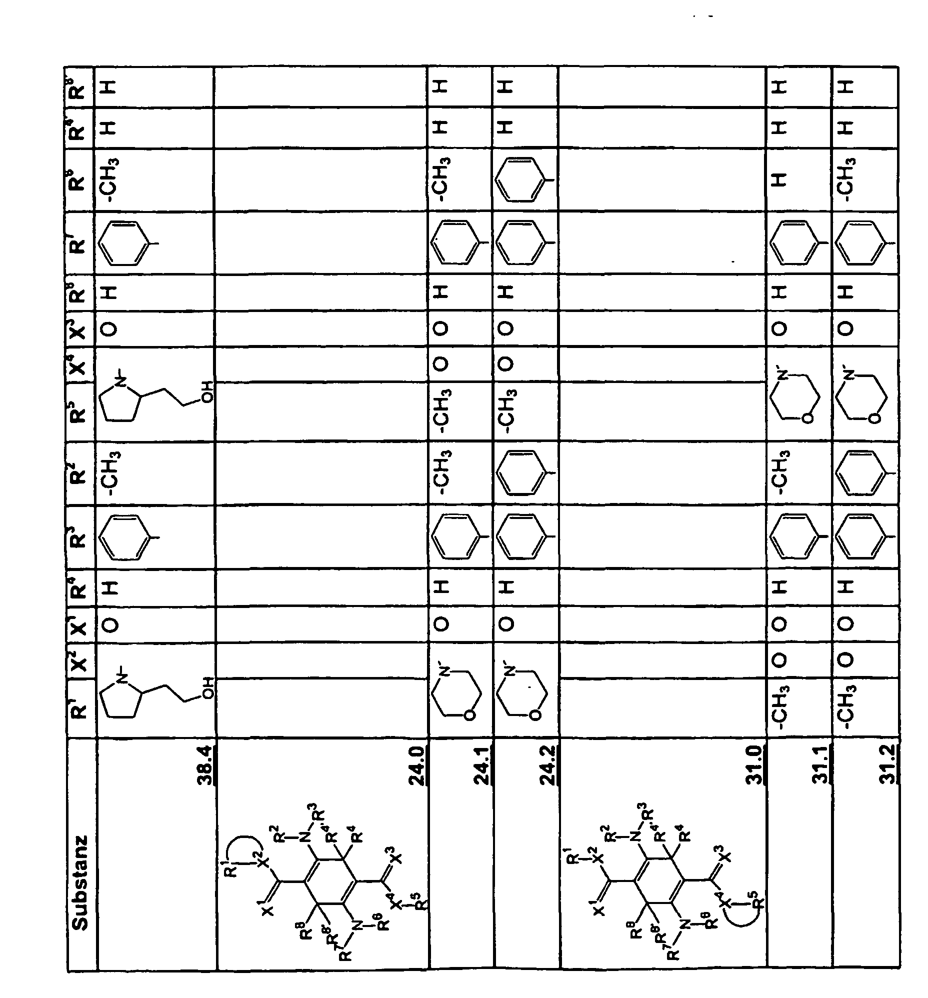

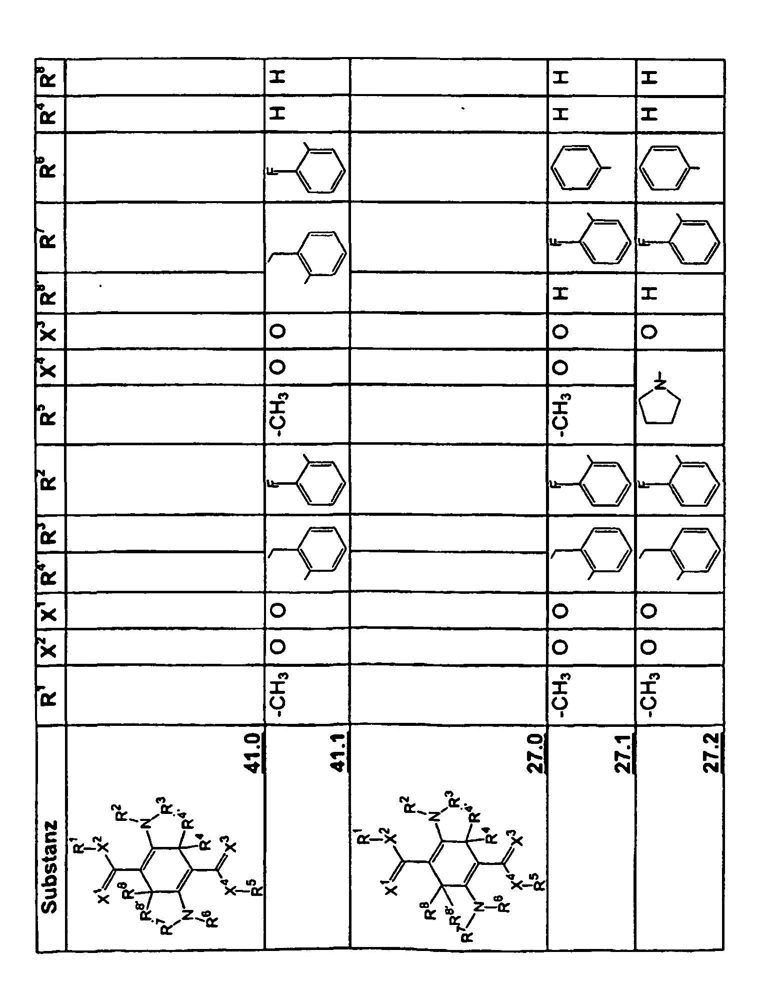

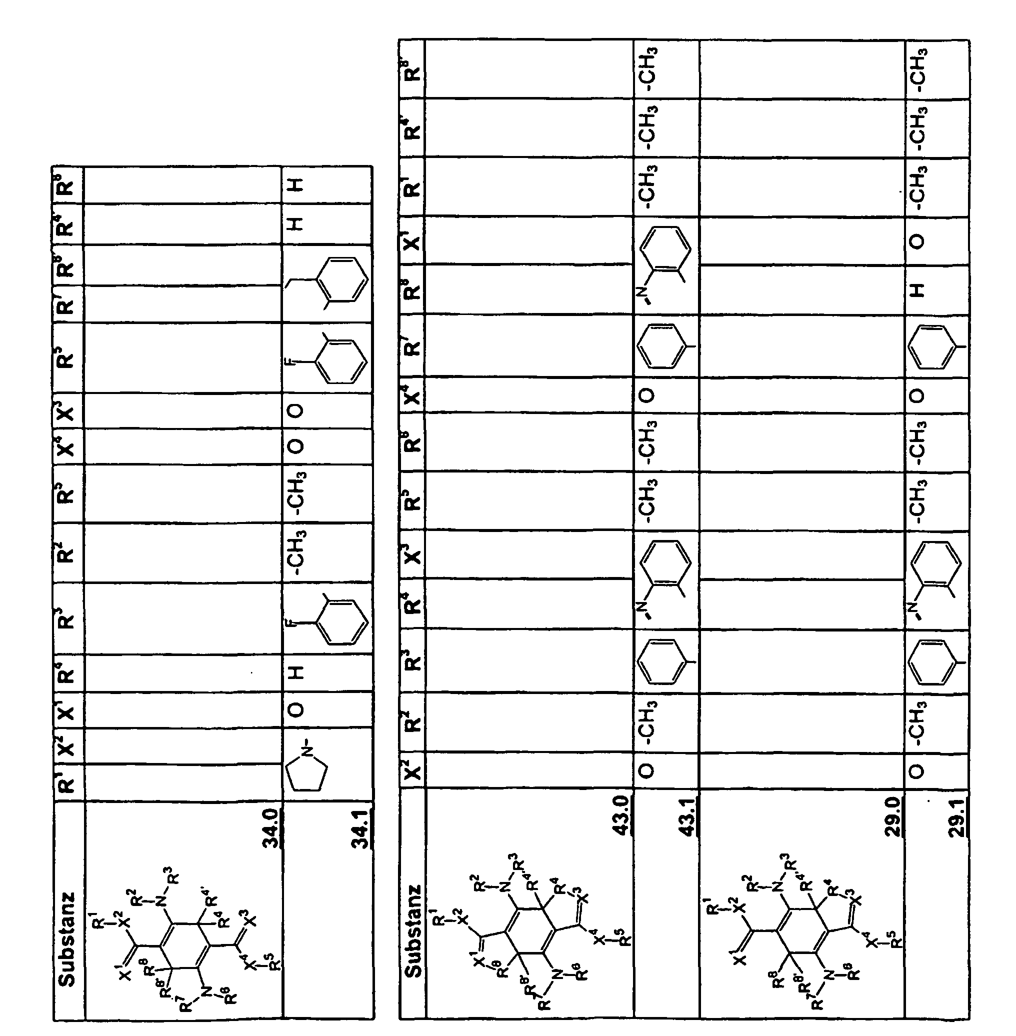

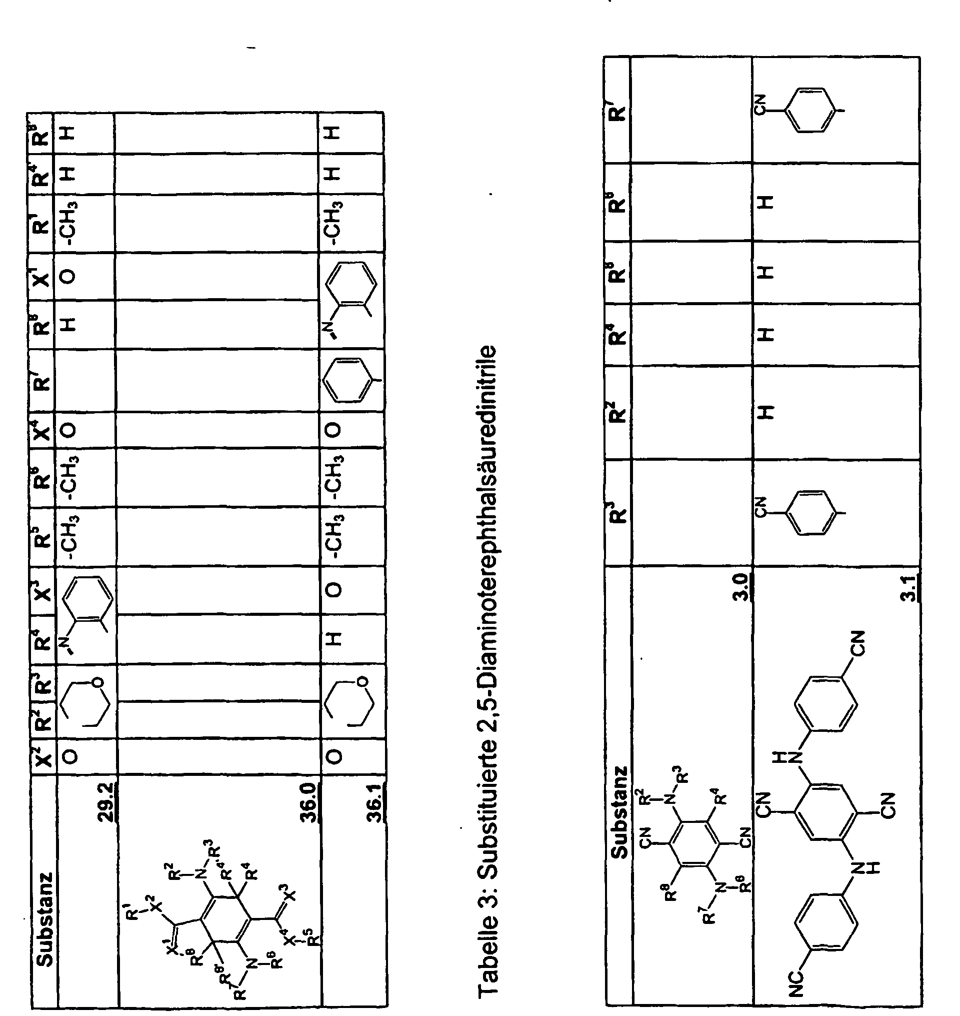

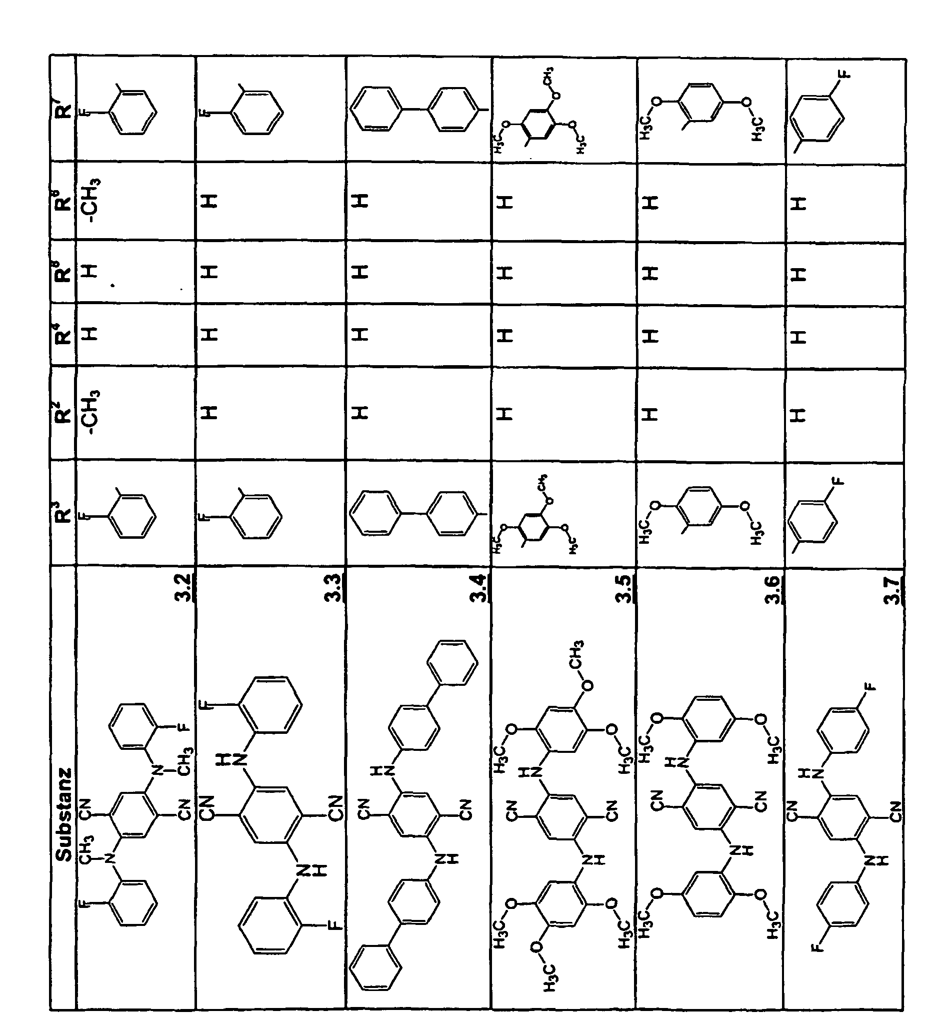

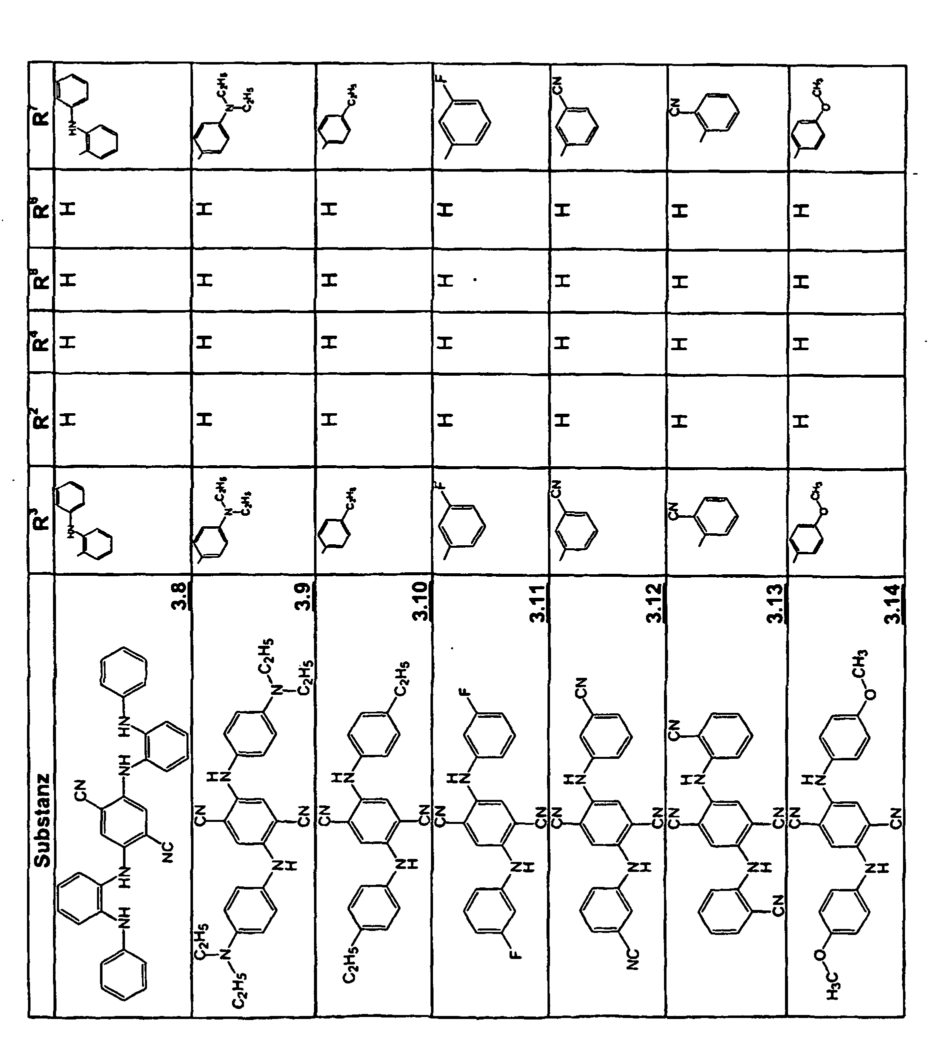

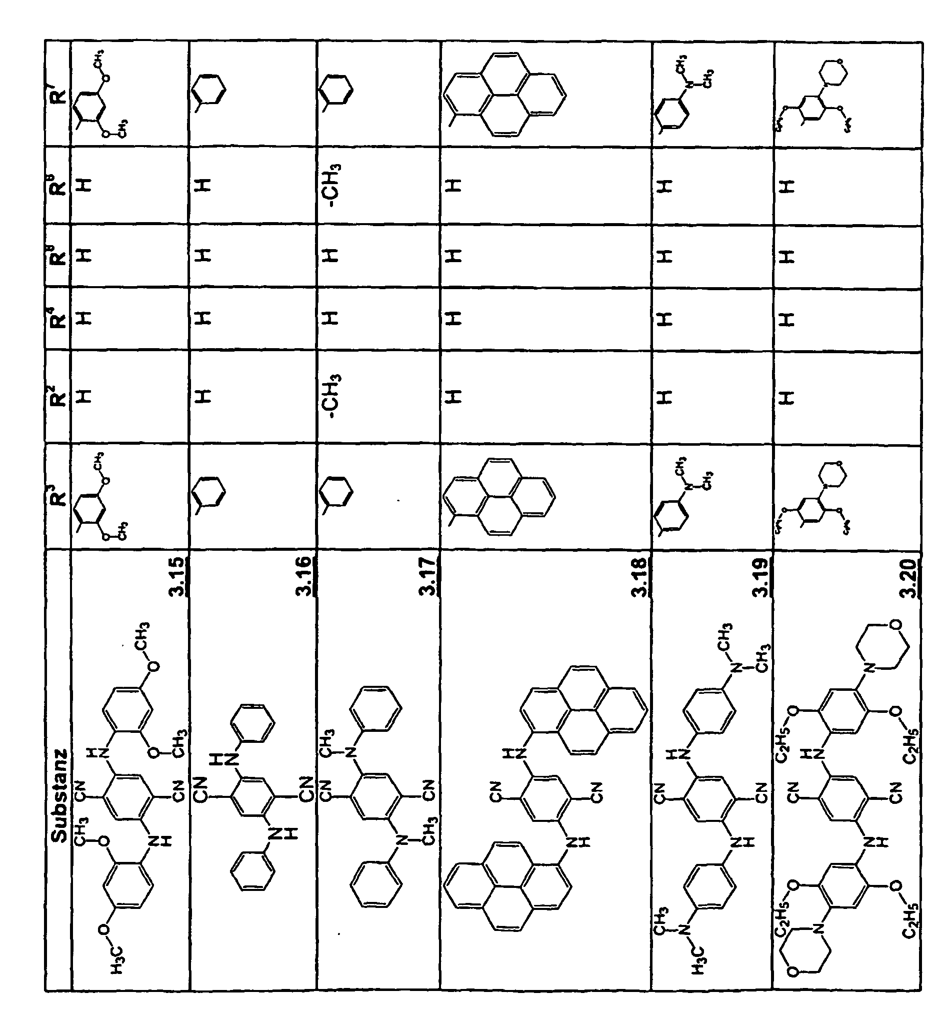

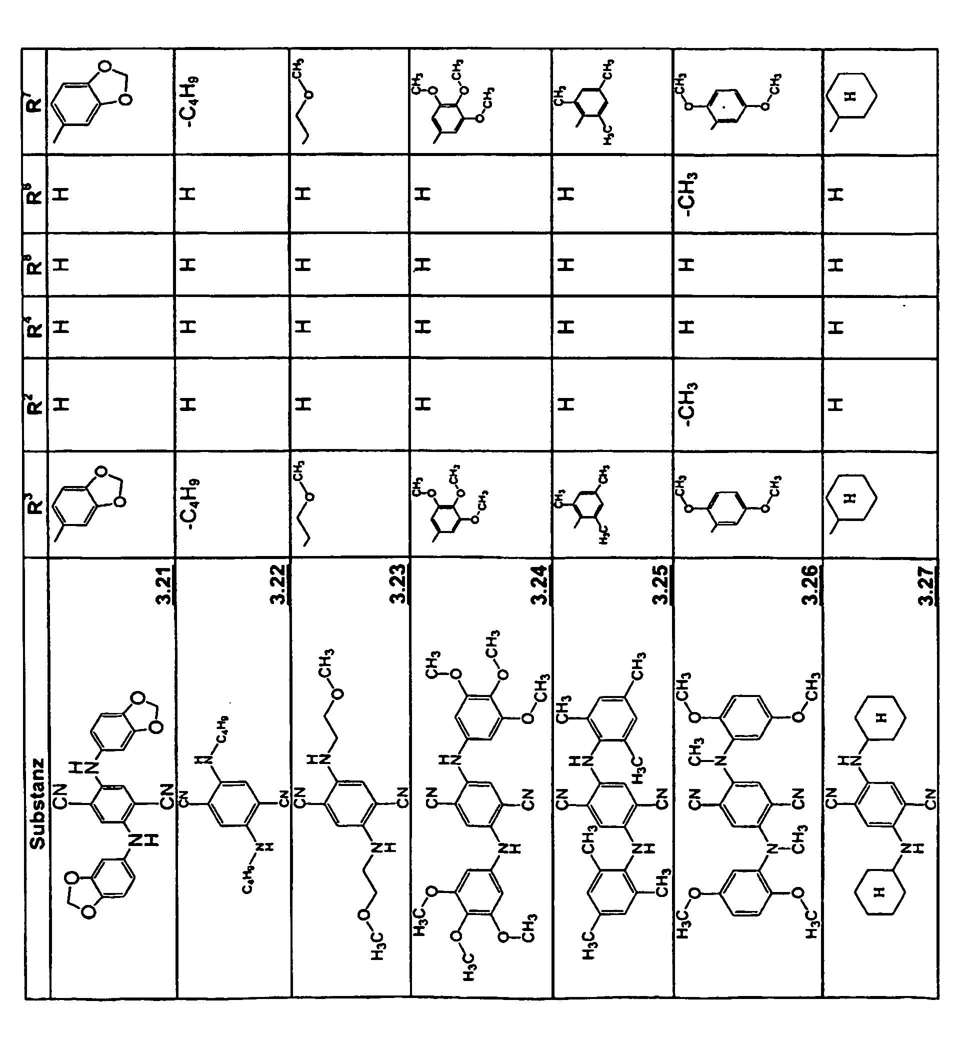

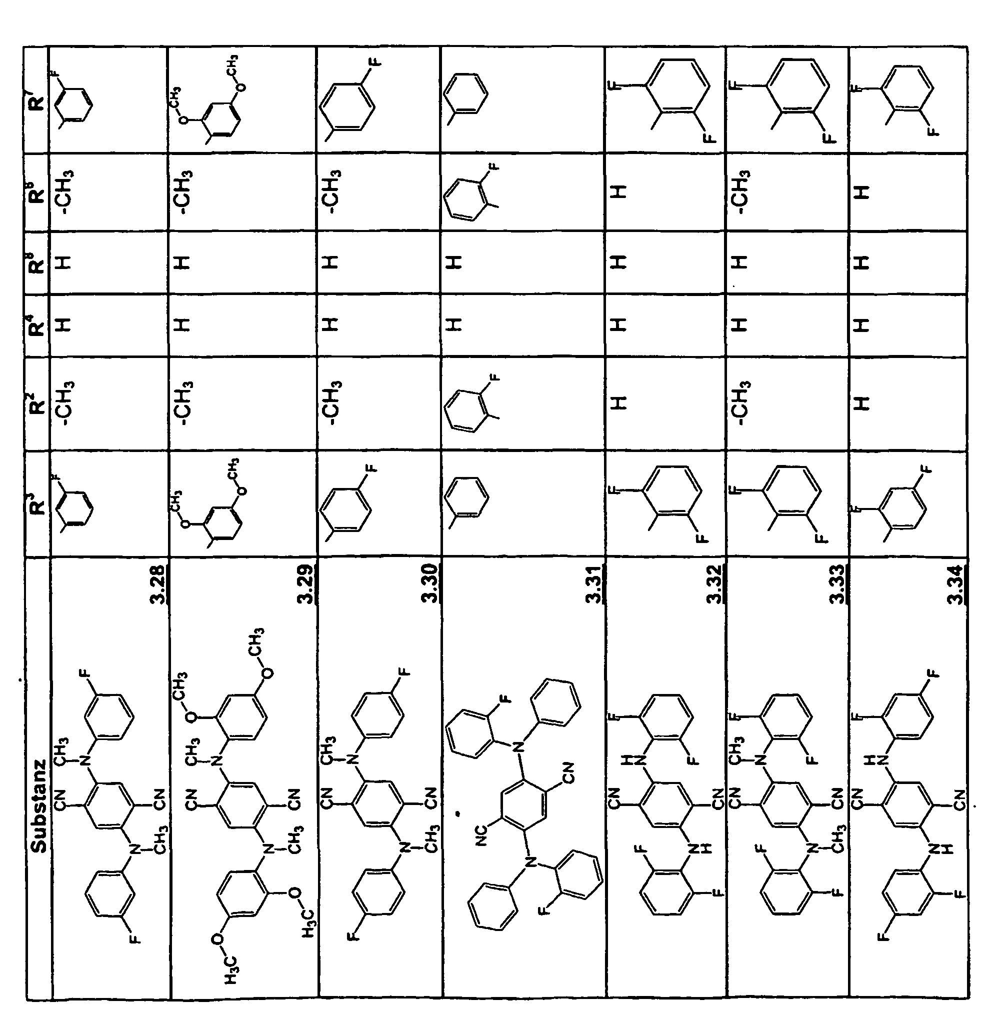

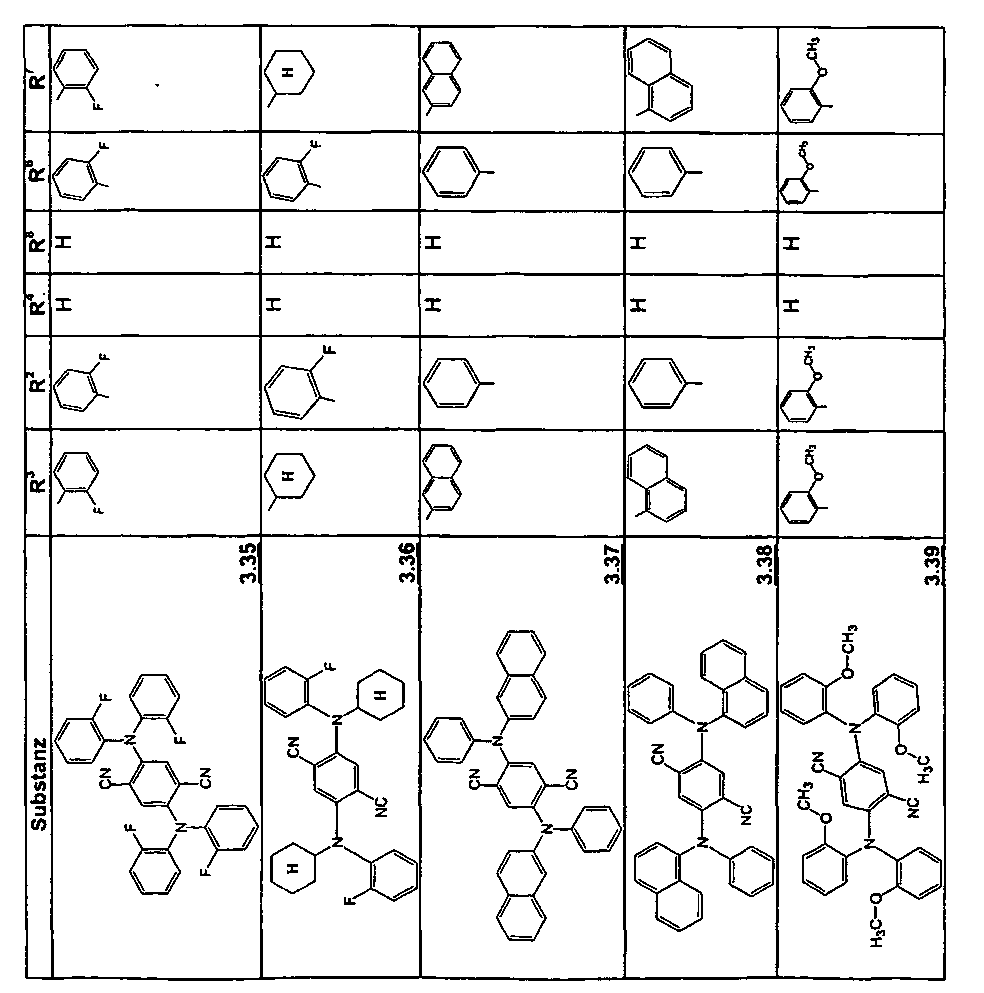

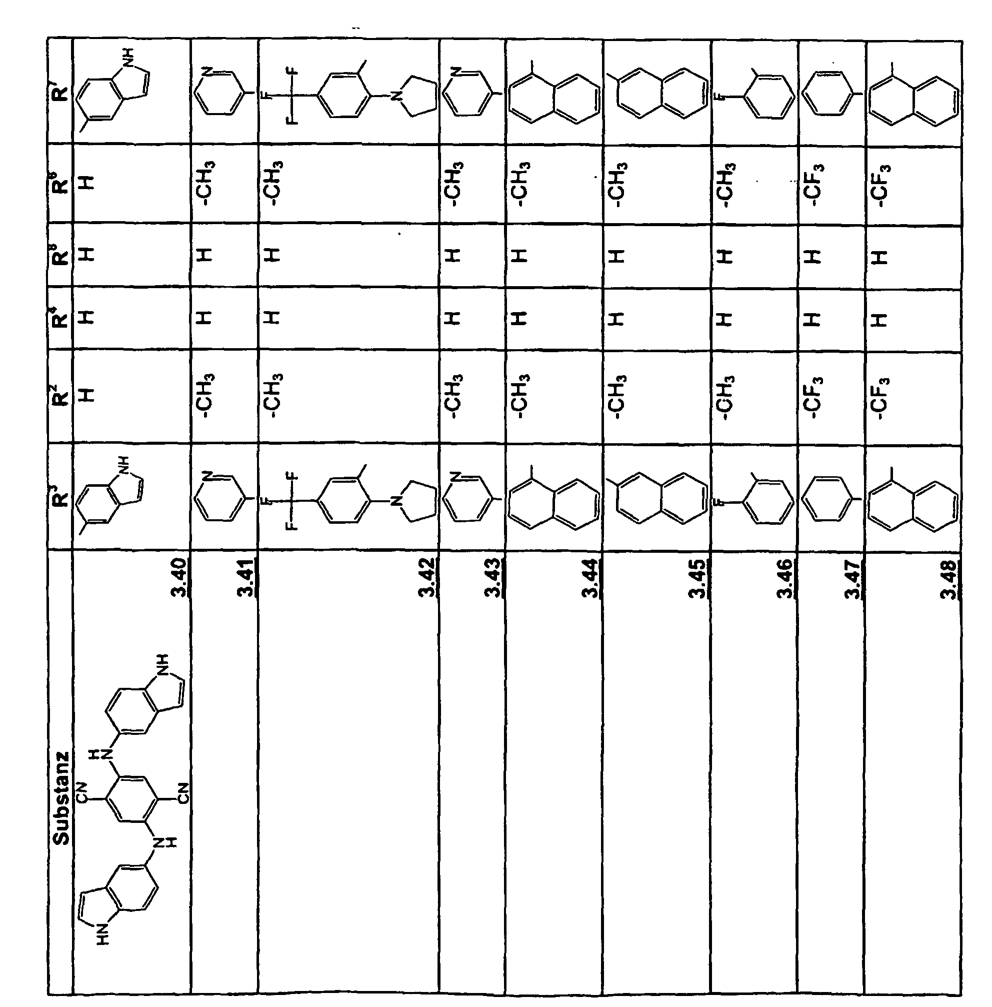

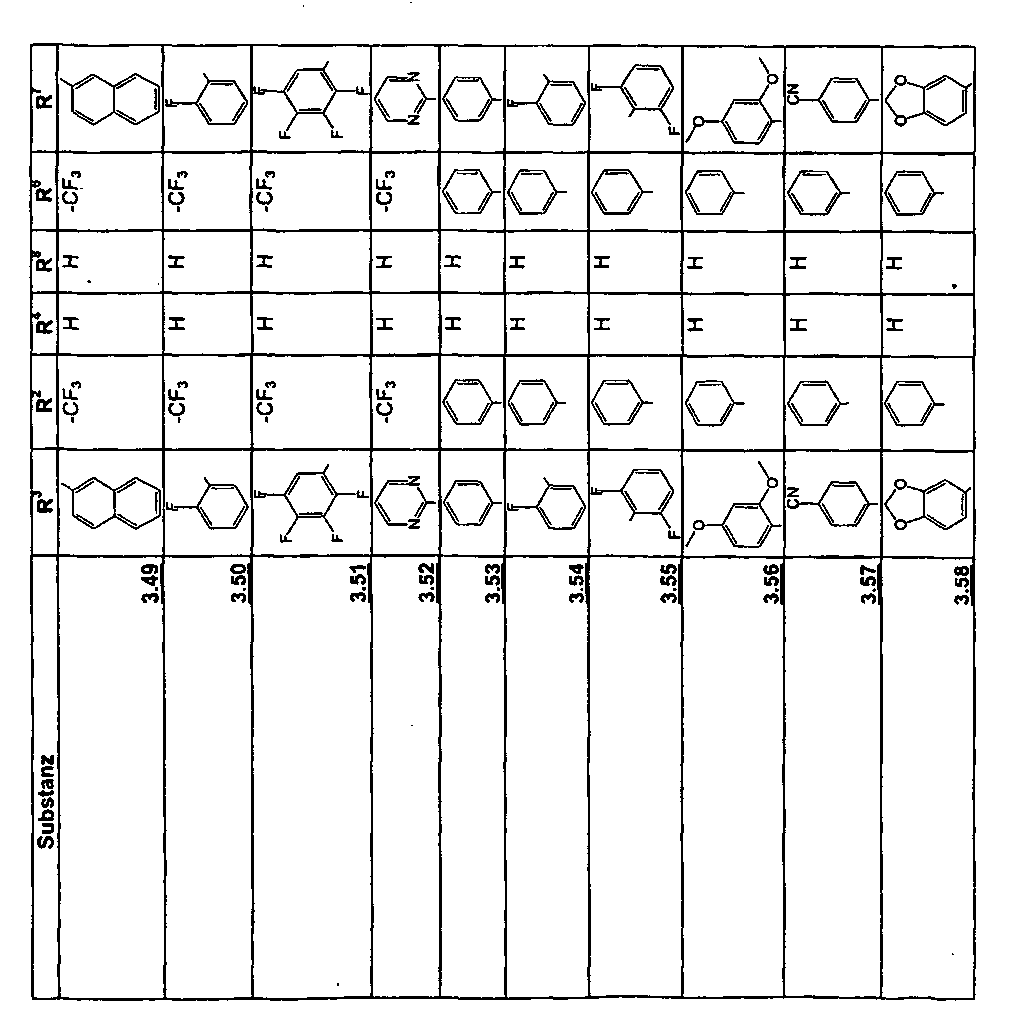

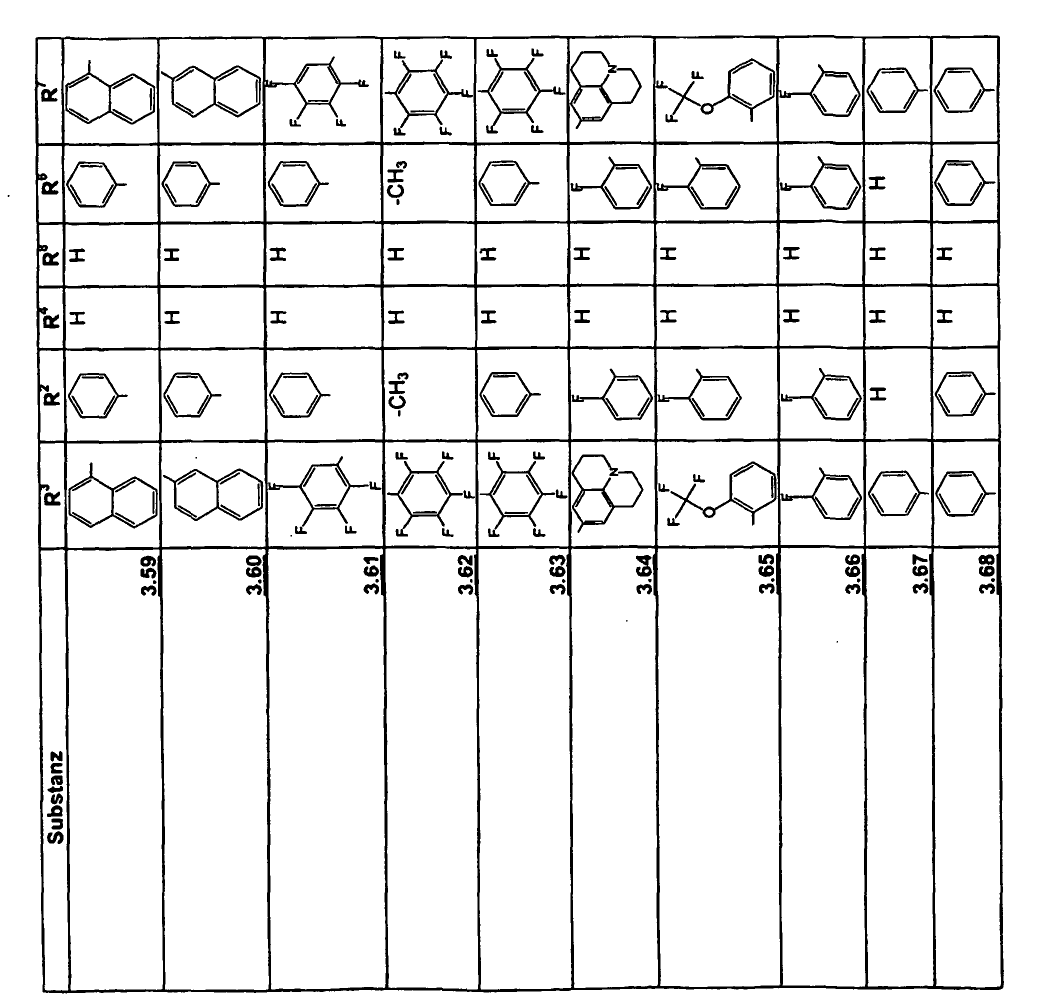

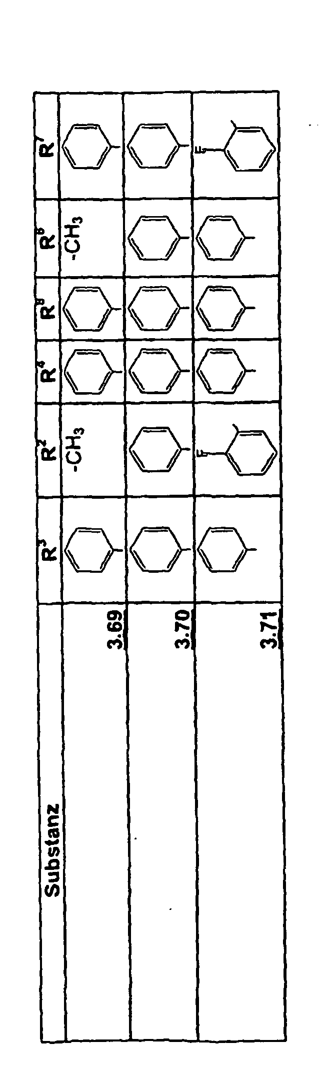

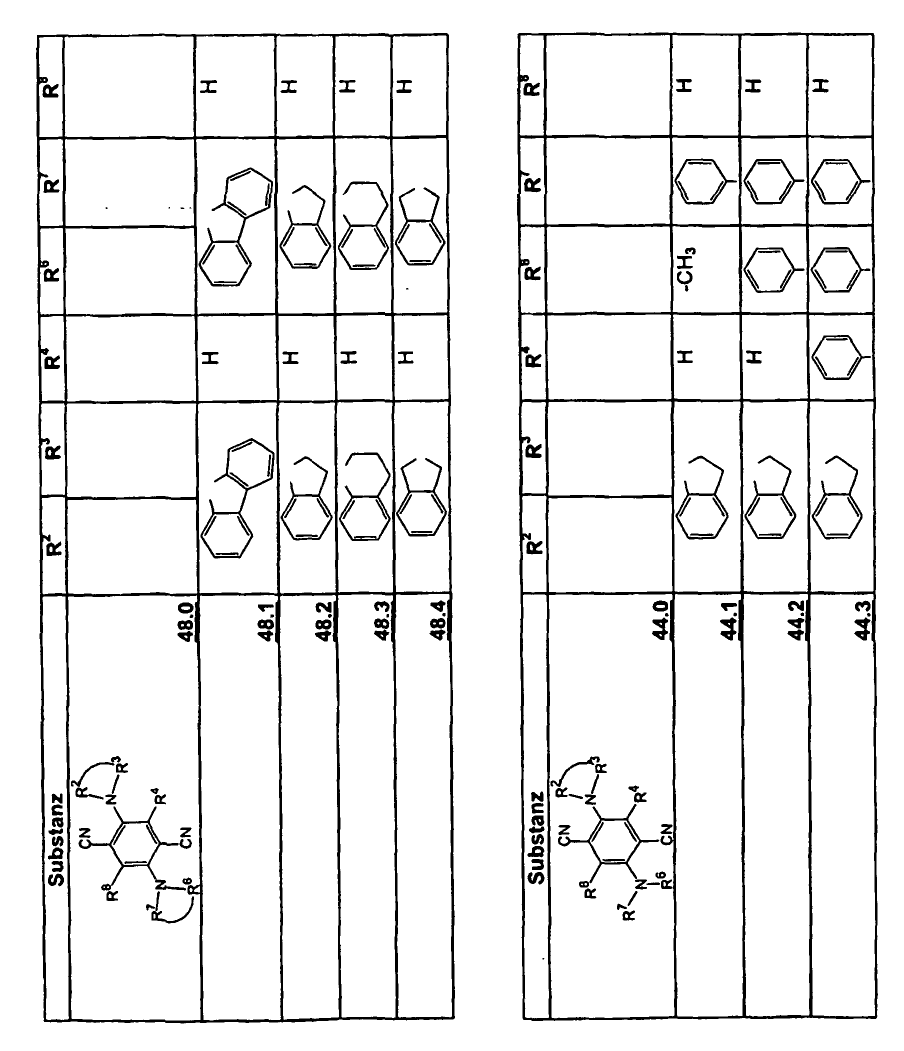

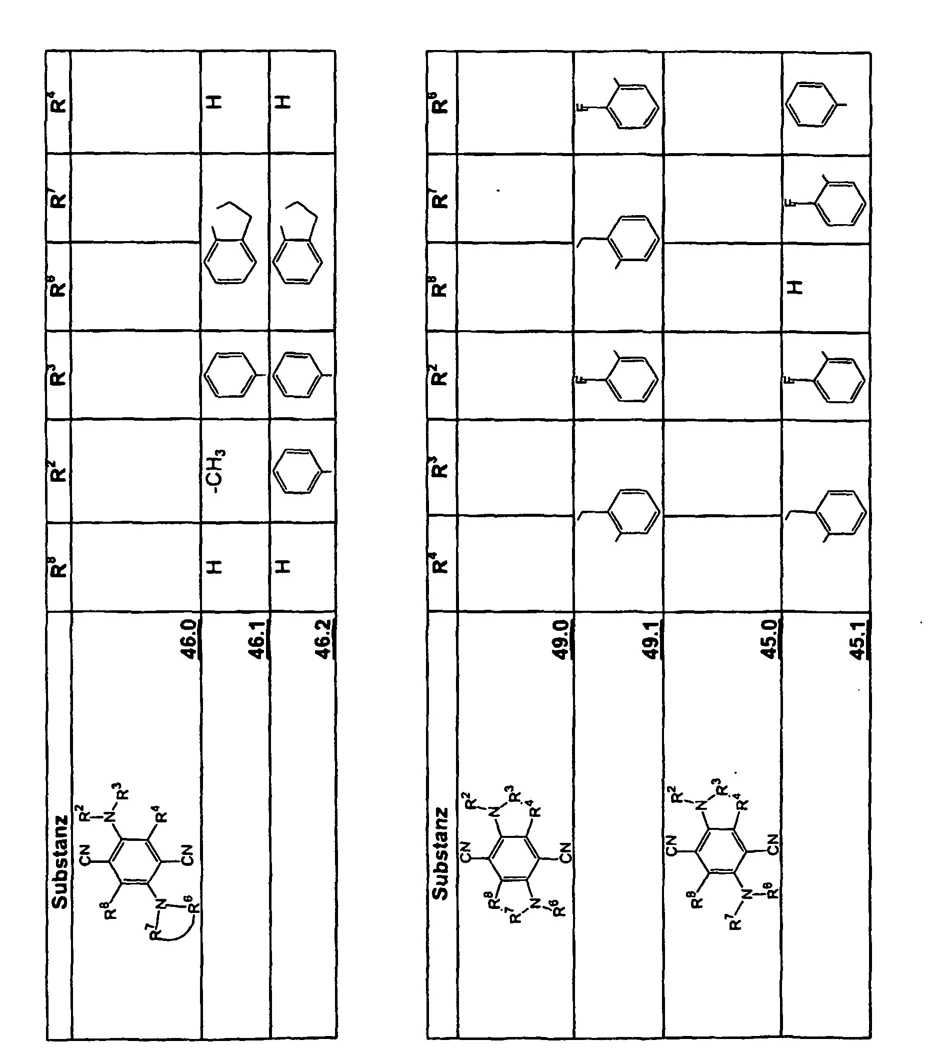

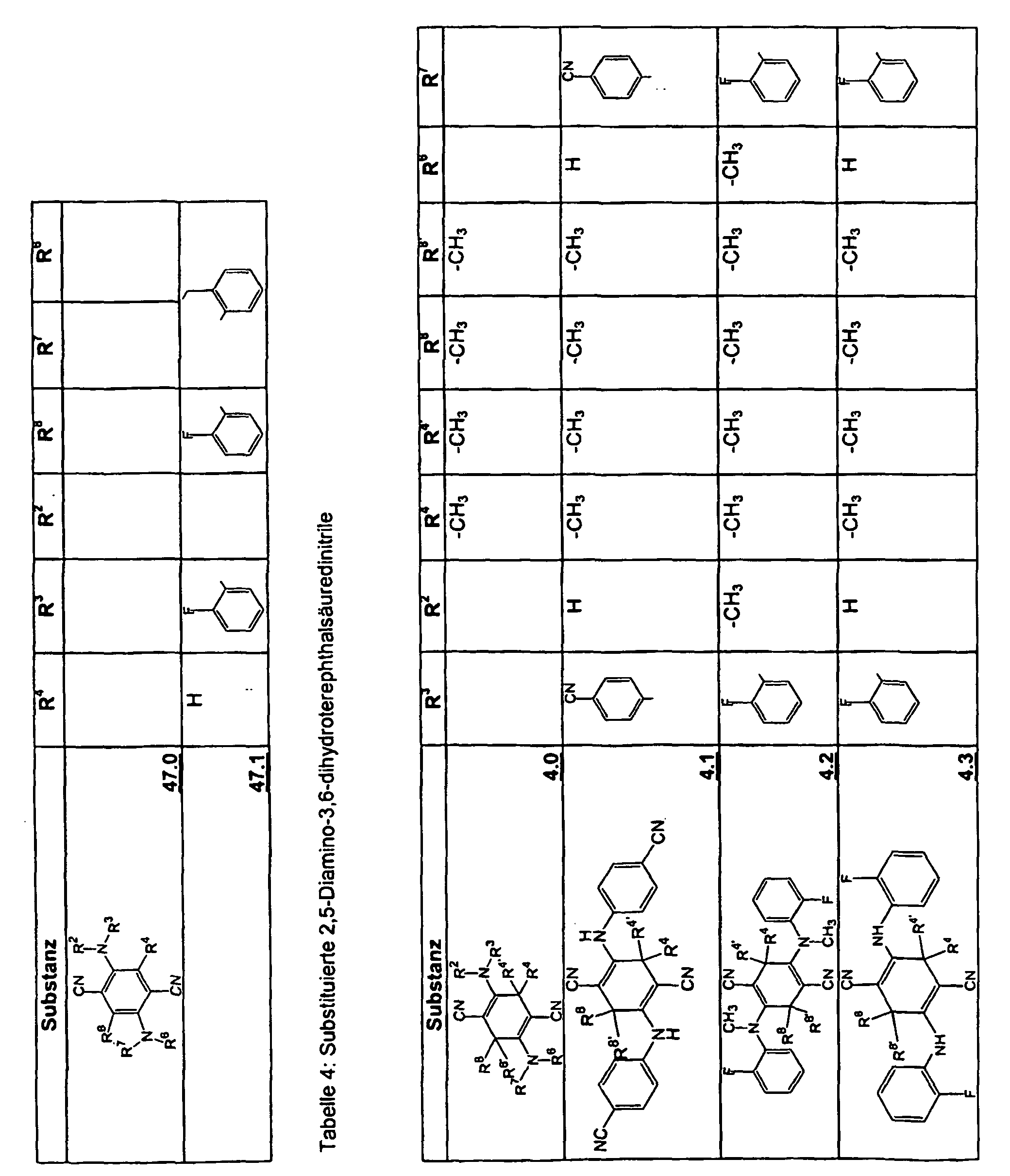

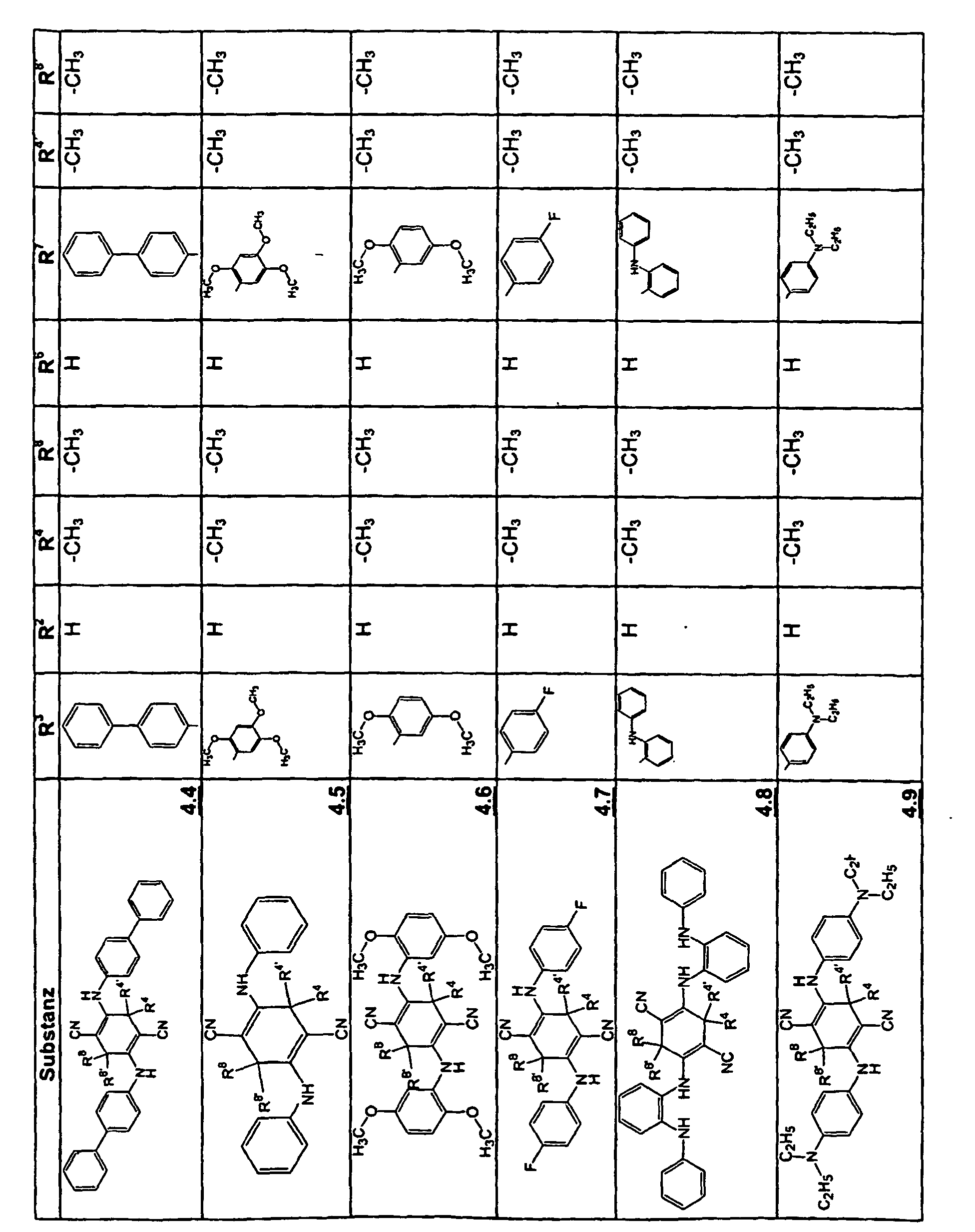

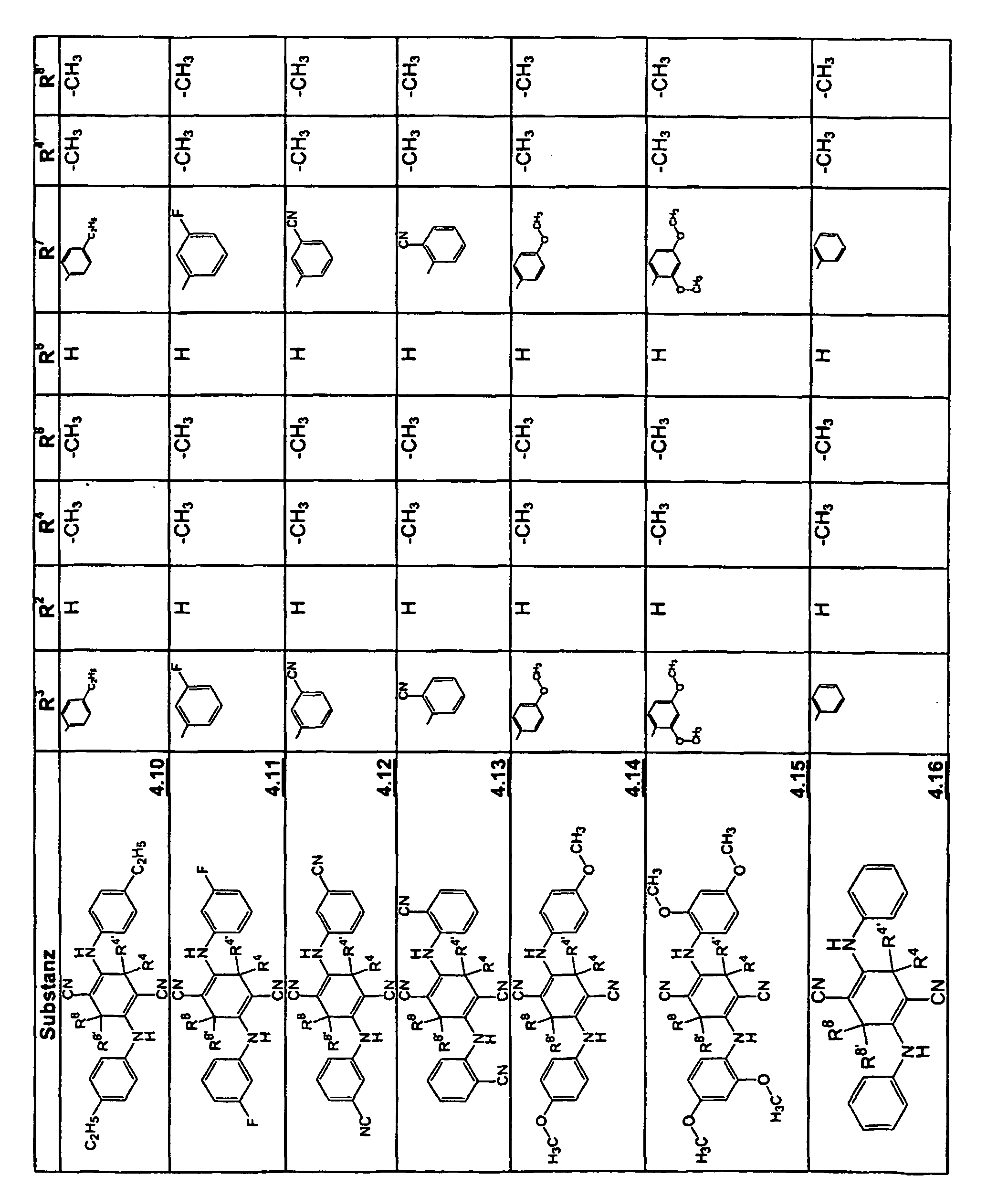

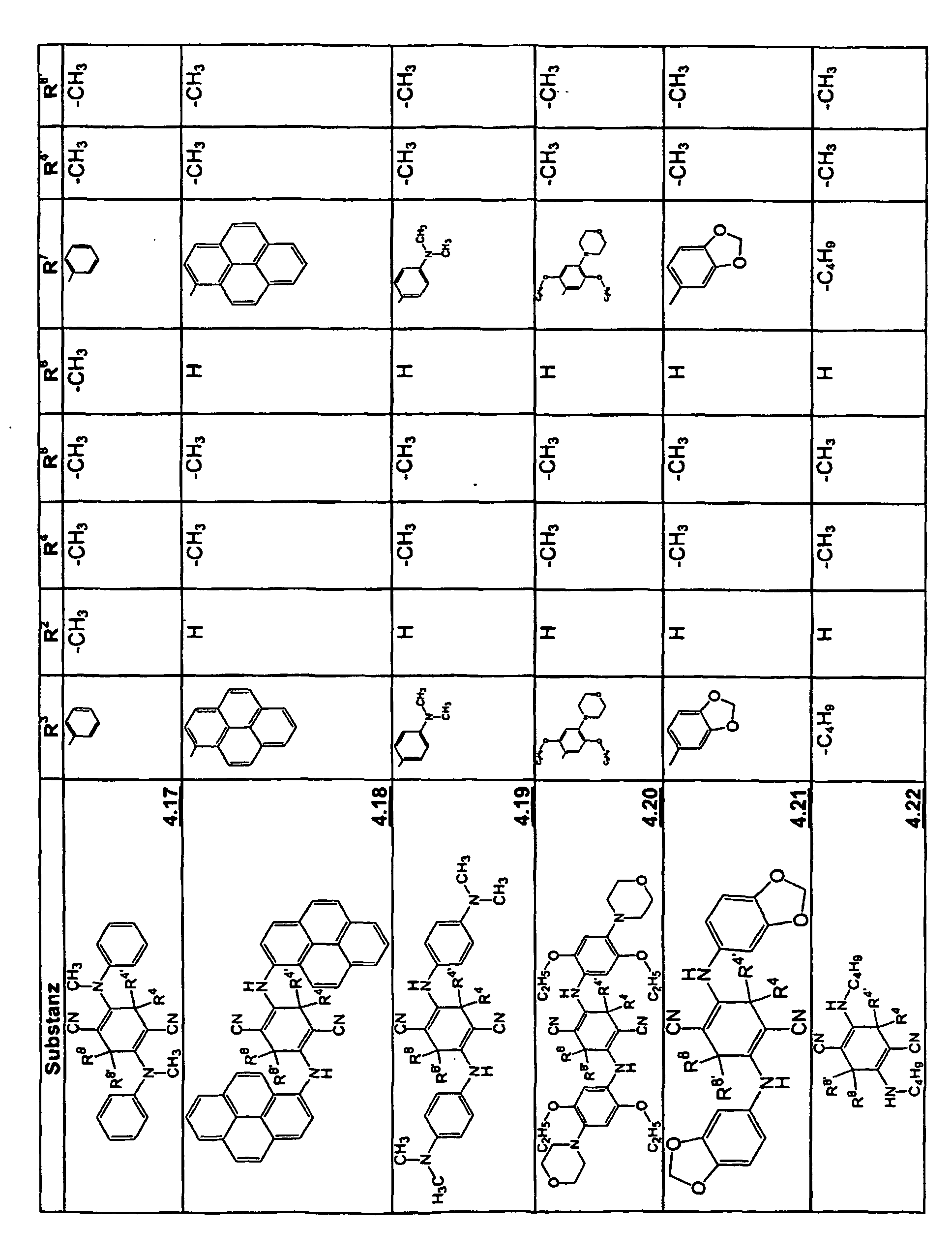

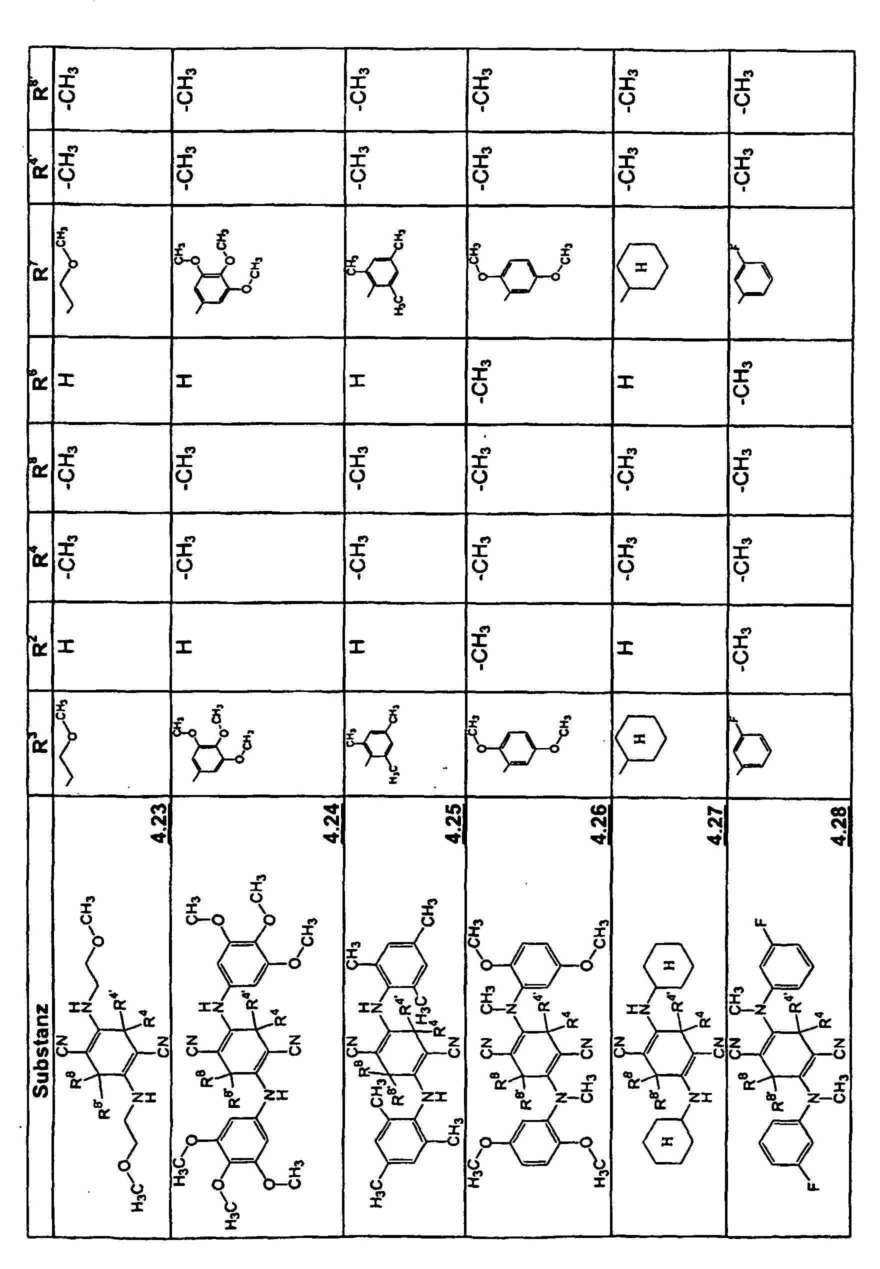

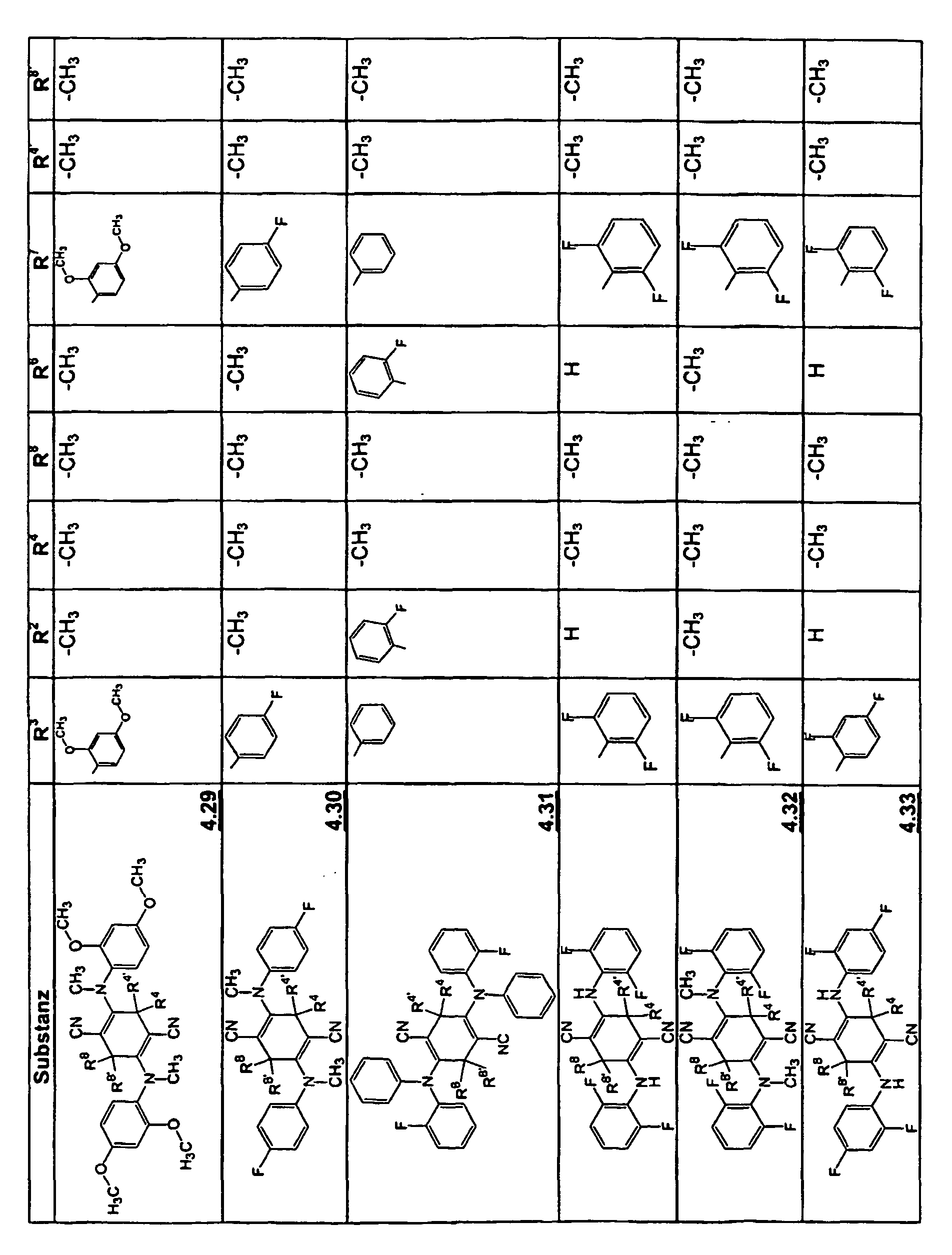

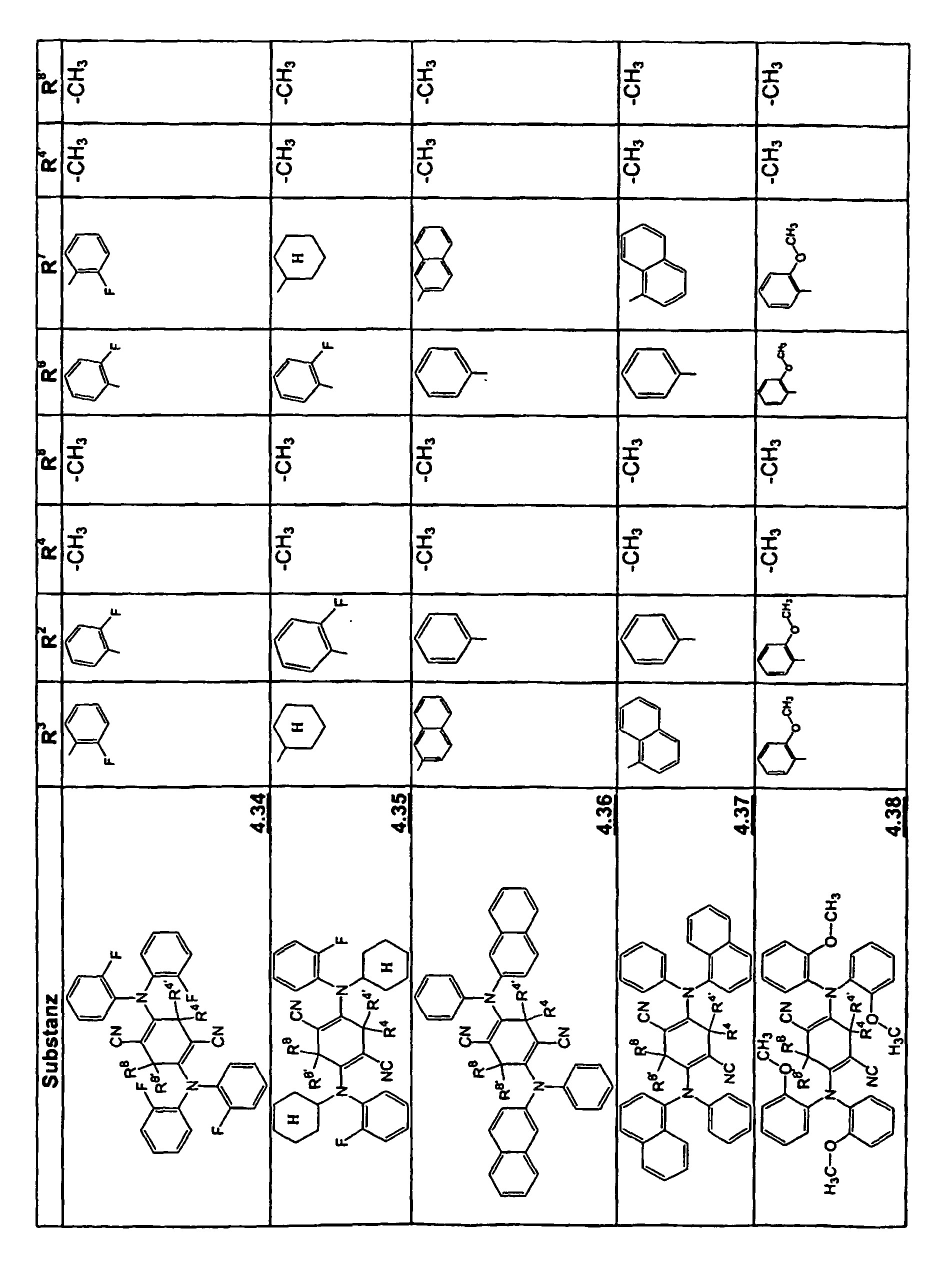

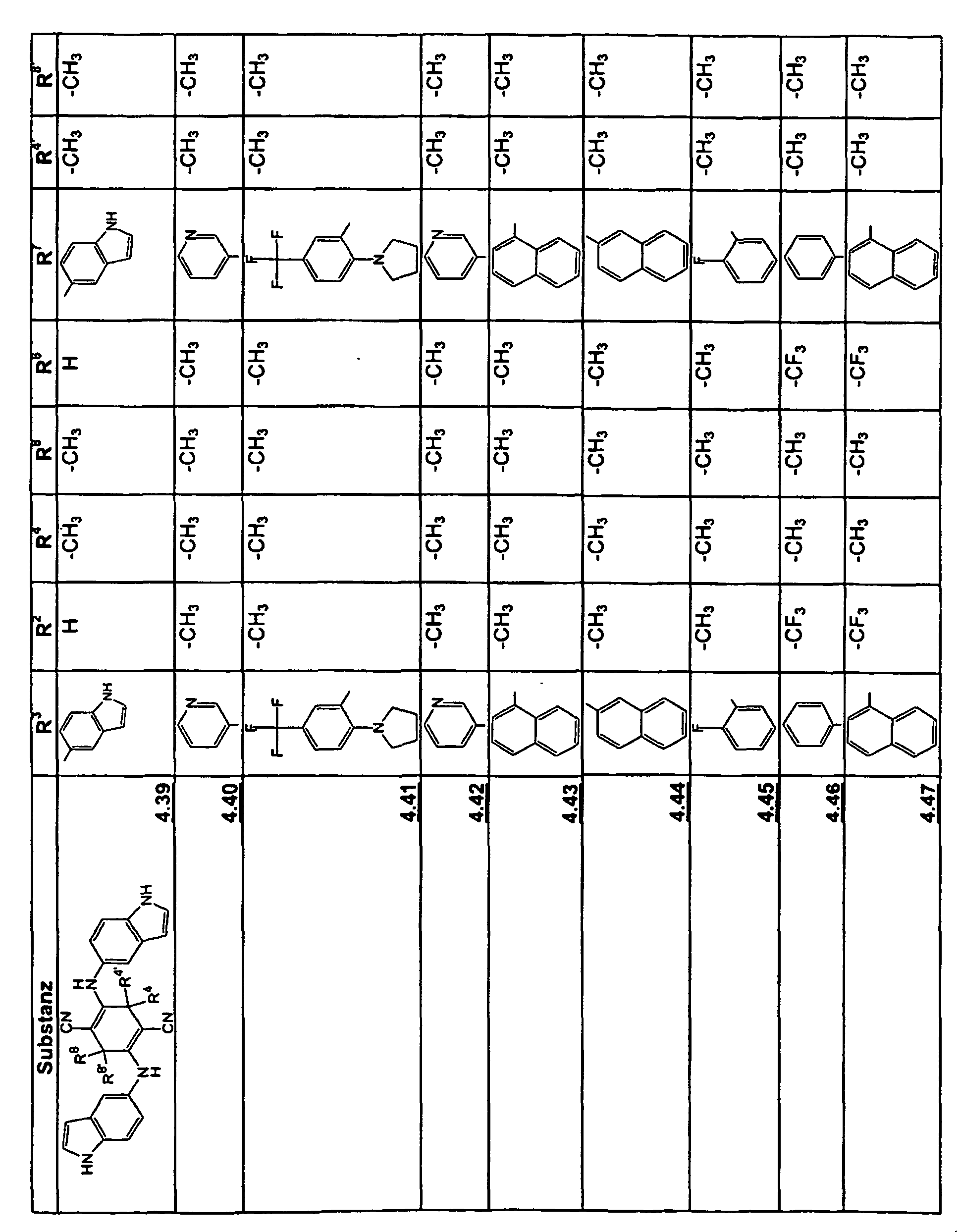

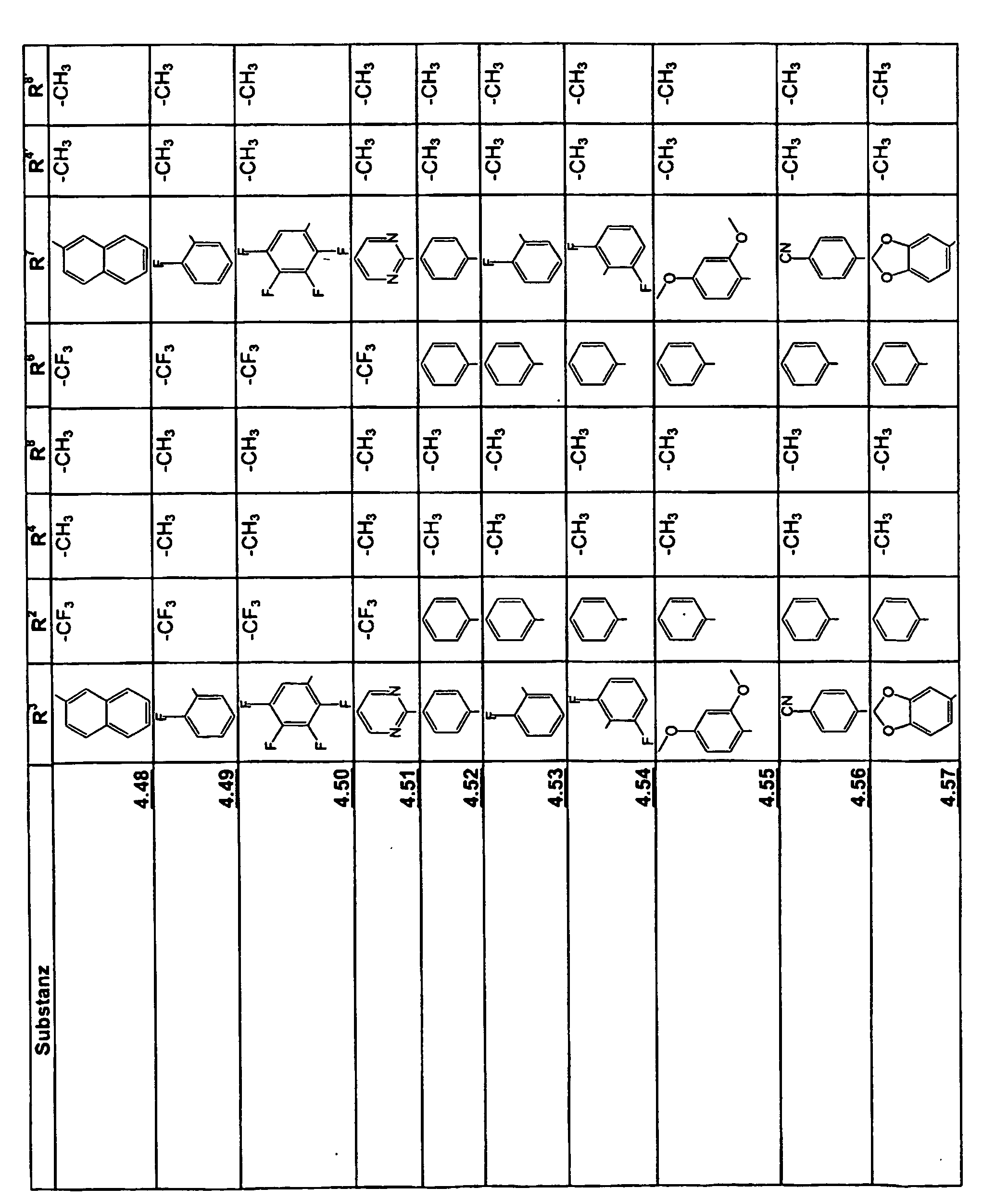

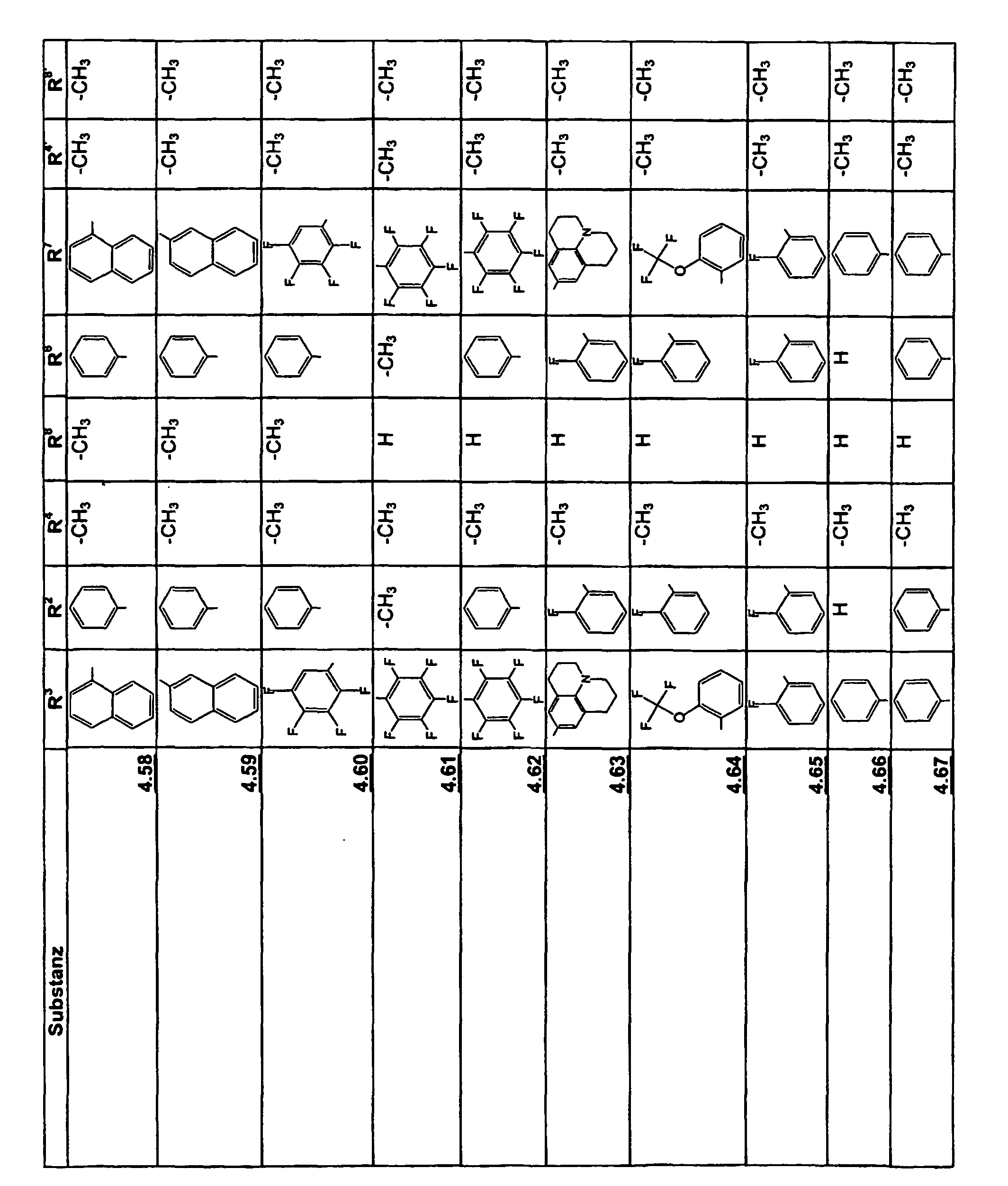

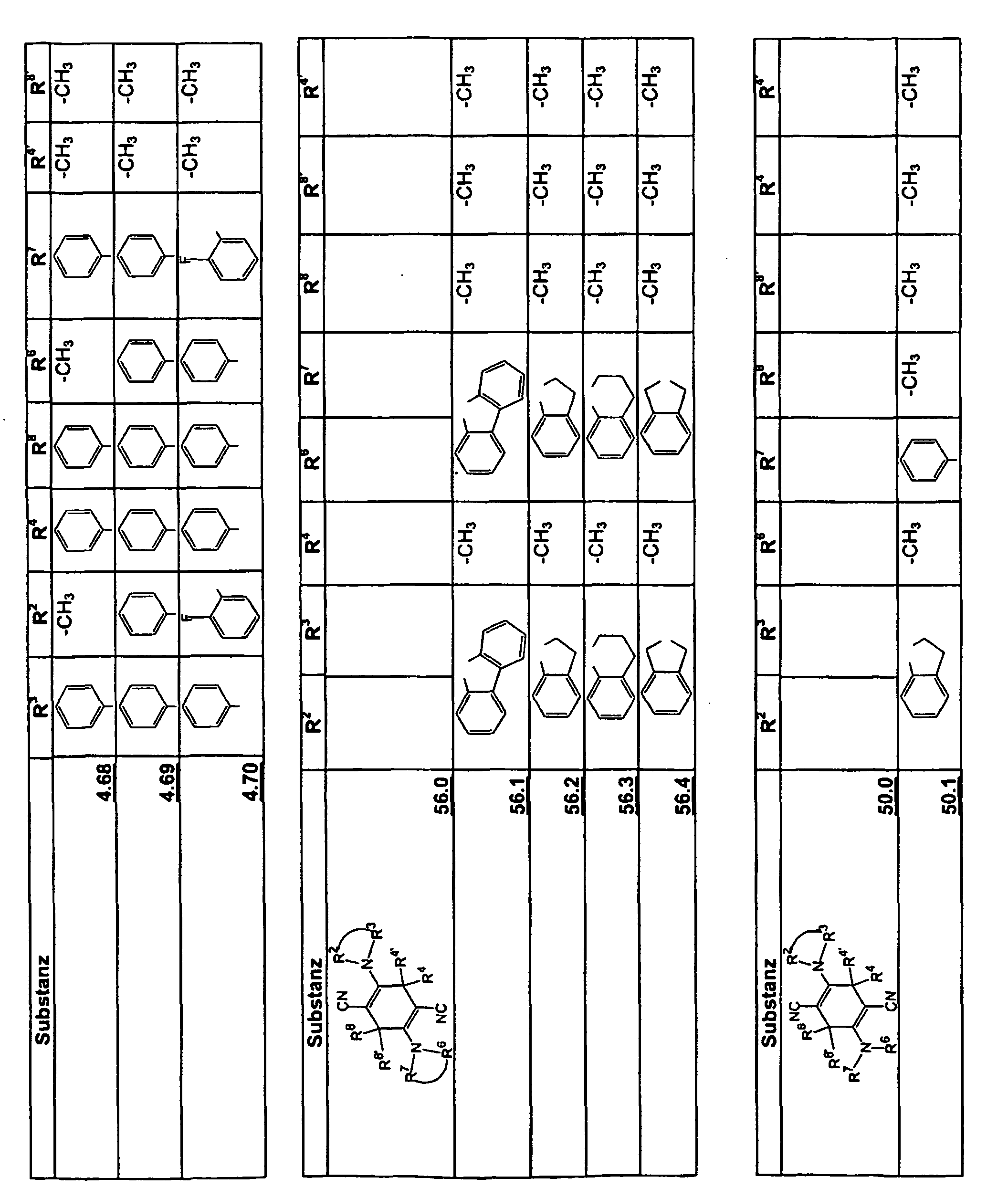

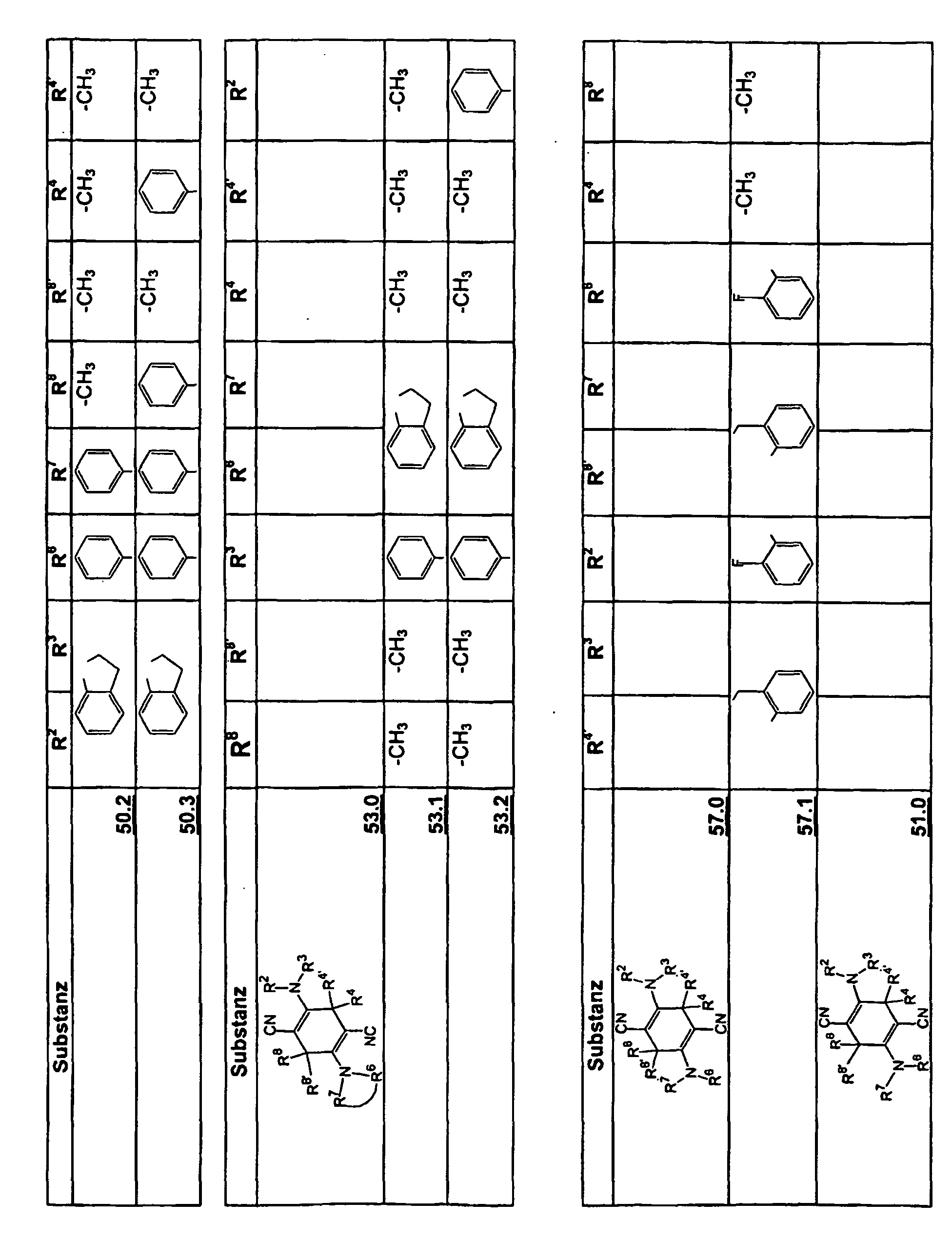

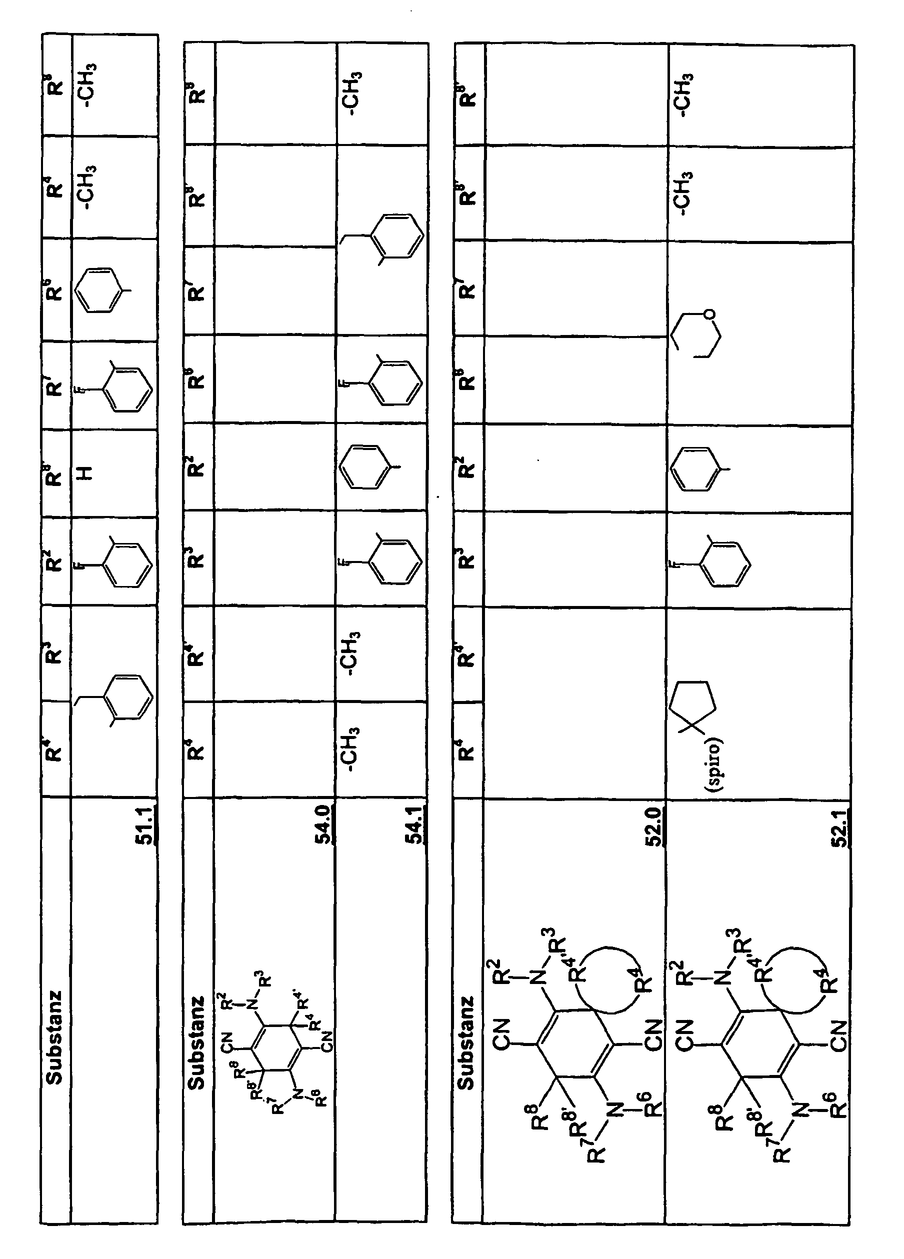

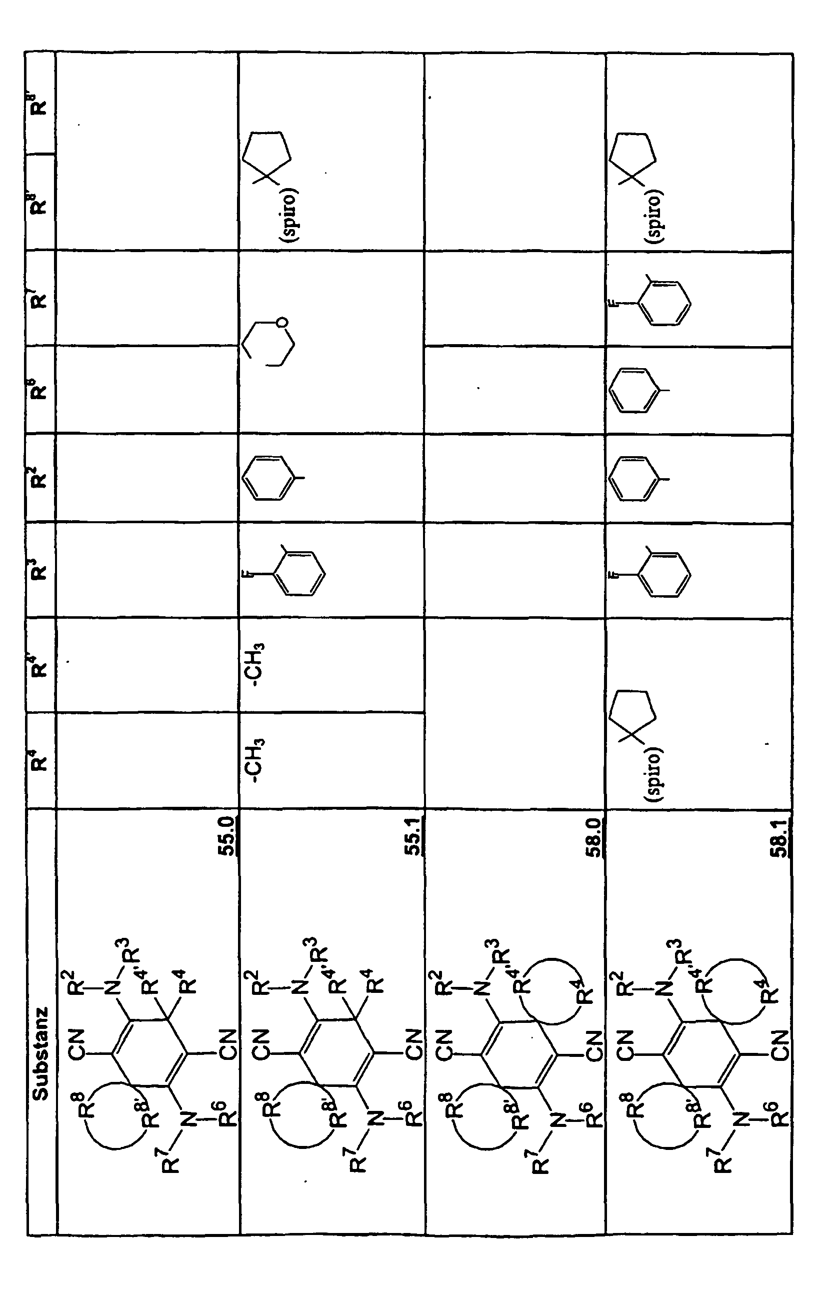

Die neuen Emitter werden in einem Device mit oder ohne einer Elektronentransportschicht verwendet, wobei die Schichten in einem Device wie in Fig. 2 angegeben angeordnet sein können:
1. der Träger besteht aus einem transparenten Material, z. B. Glas;
2. die Anode besteht aus ITO, das die Löcher in die Lochtransportschicht injiziert;
3. u. 4. der Lochleiter besteht vorrangig aus Derivaten des Triphenylamins; es können mehrere Lochleiterschichten vorhanden sein, deren Eigenschaften auf das Device abgestimmt sind;
5. zwischen Lochleiter und Elektronenleiter werden eine oder mehrere Emitterschichten aufgebracht;
6. der Elektronenleiter kann z. B. aus Alq3 bestehen und leitet die Elektronen von der Kathode in das Innere des Devices zur Emissionsschicht bzw. dem Lochleiter;
7. die Pufferschicht aus bestimmten Metallsalzen oder deren Oxiden, wie z. B. LiF, verbessert die Elektroneninjektion in die Schicht 6;
8. die Kathode besteht aus einem unedlen Metall oder einer Legierung (z. B. Aluminium oder Calcium).

Typische Schichtdicken für die Emitter sind 3 -10 nm, vorzugsweise 4 - 6 nm. Die Emissionswellenlängen hängen in charakteristischer Weise von der chemischen Struktur ab, wobei offensichtlich elektronische und sterische Faktoren der Moleküle einen Einfluß auf die Wellenlänge des emittierten Lichts und der erreichten Performance haben. Für die in Tab 2 gezeigten Beispiele liegen die Emissionswellenlängen zwischen 538 nm und 618 nm.

Zum Erreichen von Mischfarben können die neuen Emitter der Formel **1.0** - **58.0** in mehreren Schichten der jeweils reinen Materialien übereinander (Fig. 2) oder im Gemisch in einer oder mehreren Schichten angeordnet werden.

Schichten der neuen Emitter der Formel **1.0** - **58.0** können durch bekannte Emittermaterialien dotiert werden, wie in Fig. 1 gezeigt wird.

Die neuen Emitter der Formel **1.0** - **58.0** können in Devices mit an sich bekannten Lochleitern **(****59** u. **60**) und anderen Komponenten verwendet werden. Typische Beispiele zeigen die Fig. 1 und 2.

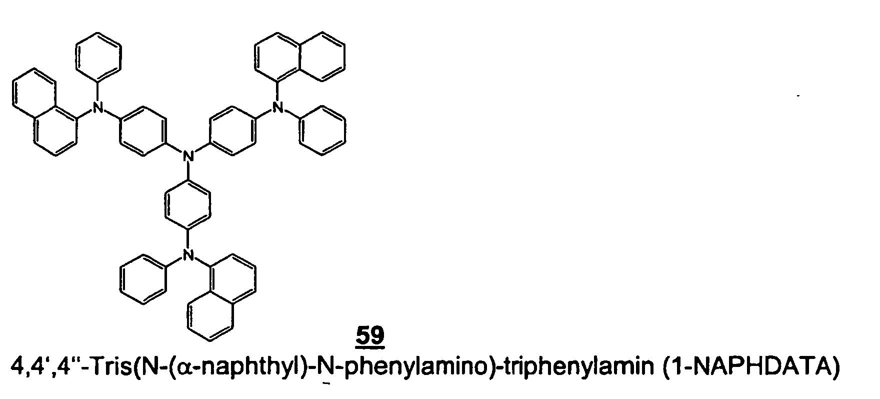

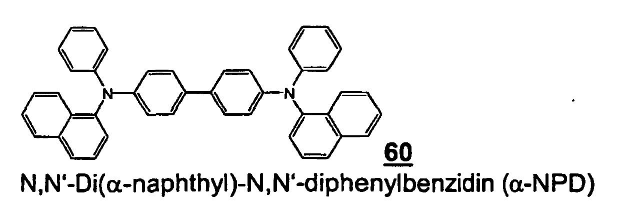

Die Devices auf der Basis der neuen Emitter können in an sich bekannter Weise durch Aufdampfen im Vakuum zwischen 1 und 10⁻⁹ Torr hergestellt werden.
Eine weitere Möglichkeit zur Herstellung der Devices besteht im Beschichten (Coating) aus der Lösung, z. B. Webcoating oder Spincoating. Dabei können die neuen Emitter der Formeln **1.0** - **58.0** sowohl in als reine Substanz oder als Dopand in einem geeigneten Polymer aufgetragen werden.
Überraschend wurde gefunden, daß insbesondere mit fluorsubstituierten Vertretern der Formel **1.0** besonders effiziente Devices hergestellt werden können. Es zeigt sich in diesen Fällen eine bemerkenswert hohe photometrische Effizienz. Mit der Substanz **1.2** wird ein Device realisiert, welches ein spektral nahezu reines grün ausstrahlt.

### Experimenteller Teil

Die folgenden Beispiele sollen die Erfindung näher beschreiben, stellen aber keine Beschränkung dar.

### Beispiel 1 (Substanzen 2.1, 2.3 - 2.5)

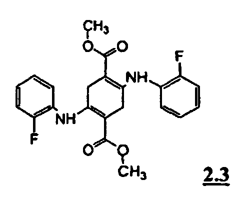

In einem Gemisch aus 200 ml Eisessig und 200 ml Alkohol (entsprechend der Esterkomponente) werden 0.06 mol Cyclohexan-2,5-dion-1,4-dicarbonsäurediester suspendiert. Unter Stickstoff werden 0.135 mol eines primären Amins bzw. Anilins zügig zugesetzt. Das Reaktionsgemisch wird unter intensivem Rühren 5 - 8 h unter Rückfluß erhitzt. Die akzeptorsubstituierten Aniline erfordern eine Verlängerung der Reaktionszeiten.
Zur Isolierung des Rohprodukts genügt es im Falle der Aniline, die abgekühlte Reaktionsmischung abzusaugen, mit Methanol intensiv zu waschen und zu trocknen. Aliphatische Amine bilden Produkte von hoher Löslichkeit, so daß zunächst, sämtliches Lösungsmittel weitestgehend am Rotationverdampfer abgetrennt werden muß. Das Rohprodukt wird in Methanol aufgenommen und nach starker Kühlung abgesaugt und getrocknet.

### Beispiel 2 (Substanzen 1.1, 1.3 - 1.5)

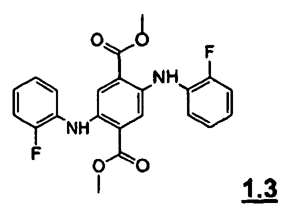

Die in Beispiel 1 erhaltenen Dihydroterephthalsäureester werden oxidiert. Die Isolierung gelingt mit Ausbeuten bis zu 95%. Das abgetrennte Rohprodukt kann zur Reinigung aus DMF, Toluol, Chloroform oder Methanol umkristallisiert werden. Die Substanzen sind sublimierbar.

### Beispiel 3 (Substanz 19.1 - 19.4)

Die nach Beispiel 2 erhaltenen Ester werden in Gemischen aus n-Propanol und Wasser verseift. Vom Terephthalsäurediester werden 0.01 mol in etwa 50 ml n-Propanol suspendiert und mit 50 ml Wasser, das 0.03 mol Kaliumhydroxid enthält, versetzt. Die Suspension wird unter Rückfluß erhitzt bis eine klare Lösung entstanden ist. Nach weiteren 2 h wird abgesaugt. Zur Neutralisation werden in die Lösung etwa 5 ml Eisessig getropft. Die anfallende Säure wird mit Methanol gewaschen und getrocknet. Zur Herstellung der Substanzen **19.1** **-****19.4** wurden 0.01 mol der hier hergestellten Terephthalsäure in 100 ml Eisessig unter Zusatz von 15 ml Formaldehydlösung (37%) 2 h unter Rückfluß erhitzt.
Die Reaktionsprodukte werden abgetrennt und mit Methanol gewaschen. Es wird aus Acetonitril oder Chloroform umkristallisiert. Die Substanzen können durch Sublimation gereinigt werden.

### Beispiel 4 (Substanz 1.2)

Um Verbindungen dieses Typs herzustellen kann der entsprechende Terephthalsäureester (Beispiel 2) alkyliert werden. In 350 ml wasserfreien DMSO werden 0.05 mol Terephthalsäureester suspendiert und mit 18.63 g (0.131 mol) Methyliodid versetzt. Bei einer Temperatur zwischen 20 und 23°C werden portionsweise unter starkem Rühren 6.1 g (0.152 mol) 60%iges Natriumhydrid in Paraffin hinzugefügt. Nach ca. 5 h Reaktionszeit hat sich ein Farbumschlag der festen Bestandteile von orange nach rein gelb vollzogen. Zu diesem Gemisch werden nun ca. 200 ml Methanol gegeben, was die Filtrierbarkeit wesentlich verbessert.
Das abgetrennte gelbe Reaktionsprodukt wird mit Methanol intensiv gewaschen und getrocknet. Ein reines Produkt wird durch Umkristallisation aus Toluol erhalten.

### Beispiel 5 (Device: Substanz 19.4)

Auf einen strukturierten ITO-Glasträger von 50 × 50 mm² wurde eine Schicht von 55 nm 4,4',4"-Tris(N-(α-naphthyl)-N-phenylamino)-triphenylamin und eine weitere von 5 nm Stärke aus N,N'-Di(α-naphthyl)-N,N'-diphenylbenzidin aufgedampft. Auf diesen Lochtransportschichten werden 5 nm 1,6-Bis(2,4-dimethoxyphenyl)-benzo[1,2-d;4,5-d']-1,2,6,7-tetrahydro-bis[1,3]oxazin-4,9-dion (**19.4**) abgeschieden.
Über dieser Emitterschicht wird nun zusätzlich Tris-(8-hydroxychinolinato)-aluminium mit einer Schichtdicke von 30 nm aufgebracht und darüber zunächst eine sehr dünne Pufferschicht (0.5 nm) Lithiumfluorid und abschließend Aluminium aufgedampft.
Der Test dieser Anordnung erfolgte bei einer regelbaren Spannung zwischen 0 und 15 V. Diese Vorrichtung emittiert bei 578 nm (gelb). Eine Leuchtdichte von 100 cd/m² wurde bei 5.0 V erreicht. Maximal wurde eine Leuchtdichte von 11400 cd/m² erreicht.

### Beispiel 6 (Device: Substanz 1.21)

Entsprechend Beispiel 5 wurde ein Device hergestellt, wobei als Emittersubstanz 2,5-Bis-(N-(2,4-dimethoxyphenyl)amino)terephthalsäurediethylester als 5 nm starke Schicht zwischen Loch- und Elektronenleiter eingebracht wurde.
Der Test dieses Device erfolgte ebenfalls bei einer regelbaren Spannung zwischen 0 und 15 V. Diese Vorrichtung emittiert bei 618 nm (rot). Eine Leuchtdichte von 100 cd/m² wurde bei 9.5 V erreicht. Maximal wurde eine Leuchtdichte von 644 cd/m² erreicht.

### Beispiel 7 (Device: Substanz 1.5)

Das Device folgt dem Aufbau in den Beispielen 5 und 6. Als Emittersubstanz wurde 2,5-Bis-(N-phenylamino)-terephthalsäurediethylester eingesetzt.
Für den Test wurde wiederum eine Spannung zwischen 0 und 15 Volt angelegt. Das Device leuchtet gelb (578 nm). Die Leuchtdichte von 100 cd/m² wurde bei 5.6 V erreicht. Maximal wurde eine Leuchtdichte von 5300 cd/m² registriert.

### Beispiel 8 (Device: Substanz 1.2)

In Analogie zu den Beispielen 5 - 7 wurde bei gleichem Bauprinzip auf den Lochtransportschichten eine Schicht von 5 nm N,N'-Dimethyl-2,5-bis-(N-(2-fluorphenyl)amino)terephthalsäuredimethylester abgeschieden.
Der Test dieser Anordnung (Fig. 2) erfolgte bei einer regelbaren Spannung zwischen 0 und 15 V. Das Device leuchtet grün (λₘₐₓ= 547 nm). Eine Leuchtdichte von 100 cd/m² wurde bei 5.4 V erreicht. Maximal wurde eine Leuchtdichte von 17700 cd/m² erreicht.
1. der Träger besteht aus Glas;
2. die Anode besteht aus ITO;
3. als Lochleiter wird 1-Naphdata aufgebracht;
4. eine weitere Lochleiterschicht besteht aus α-NPD;
5. zwischen Lochleiter und Elektronenleiter werden eine oder mehrere Emitterschichten aufgebracht;
6. der Elektronenleiter kann z. B. aus Alq3 bestehen;
7. die Pufferschicht aus besteht aus LiF;
8. die Kathode besteht aus einem unedlen Metall oder einer Legierung (z. B. Aluminium oder Calcium).

Typische Schichtdicken für die Emitter sind 3 -10 nm, vorzugsweise 4 - 6 nm.

### Photometrische Kenngrößen ausgewählter Emittersubstanzen

**Tab. 2**

| **#** | ^{**1)**}**V** | ^{**2)**}**nm** | **Farbe** | ^{**3)**}**cd/m**^{**2**} ^{**4)**} | **cd/A** | ^{**5)**} **Im/W** |
|---|---|---|---|---|---|---|
| **1.21** | 9,2 | 629 | rot-weiß | 1980 | 0,12 | 0,07 |
| **1.16**^{*)} | 9,3 | 634 | rot-weiß | 3990 | 0,14 | 0,10 |
| **1.16** | 14,0 | 618 | rot | 144 | 0,09 | 0,07 |
| **1.30** | 5,6 | 612 | orange-rot | 12100 | 2,17 | 2,27 |
| **19.4** | 5,0 | 578 | gelb | 11400 | 2,04 | 1,72 |
| **1.5** | 5,6 | 578 | gelb | 5300 | 1,59 | 1,42 |
| **1.4** | 8,0 | 577 | gelb | 1410 | 0,81 | 0,37 |
| **19.3** | 6,5 | 565 | gelb-grün | 4530 | 0,72 | 0,49 |
| **1.3** | 8,1 | 577 | gelb-grün | 4330 | 2,77 | 1,52 |
| **19.7** | 10,2 | | gelb-grün | 474 | 0,26 | 0,10 |
| **1.34** | 3,5 | 550 | grün | 36500 | 1,00 | 9,21 |
| **1.36** | 5,7 | 546 | grün | 18100 | 6,60 | 4,34 |
| **1.2** | 5,4 | 547 | grün | 17700 | 7,70 | 4,93 |
| **1.38** | 6,4 | 546 | grün | 11300 | 4,62 | 2,47 |
| **19.2** | 6,6 | 564 | grün | 6010 | 0,89 | 0,66 |
| **19.1** | 6,7 | 540 | grün | 4680 | 3,05 | 1,70 |
| **19.6** | 8,6 | 545 | grün | 2610 | 0,52 | 0,36 |
| **1.29** | 11,1 | 564 | grün | 1330 | 1,59 | 0,47 |
| **1.1** | 7,1 | 538 | grün | 1300 | 0,48 | 0,22 |
| **1.33** | 10,3 | 563 | grün | 1100 | 1,53 | 0,54 |
| **1.31** | 10,8 | 566 | grün | 754 | 1,60 | 0,53 |
| **19.8** | 13,4 | | grün | 273 | 1,20 | 0,70 |
| **19.11** | 14,4 | 532 | grün | 144 | 0,03 | 0,01 |
| **19.5** | >20,0 | 540 | grün | 8 | 0,30 | 0,28 |
| **19.9** | >15,0 | 544 | grün | 64 | 0,58 | 0,13 |

| | | | | | | |
|---|---|---|---|---|---|---|
| 1) Spannung bei 100 cd/m² | | | | | | |
| 2) λₘₐₓ der Elektrolumineszenz | | | | | | |
| 3) max. Leuchtdichte | | | | | | |
| 4) max. photometrische Effizienz | | | | | | |
| 5) max. Leistungseffizienz | | | | | | |

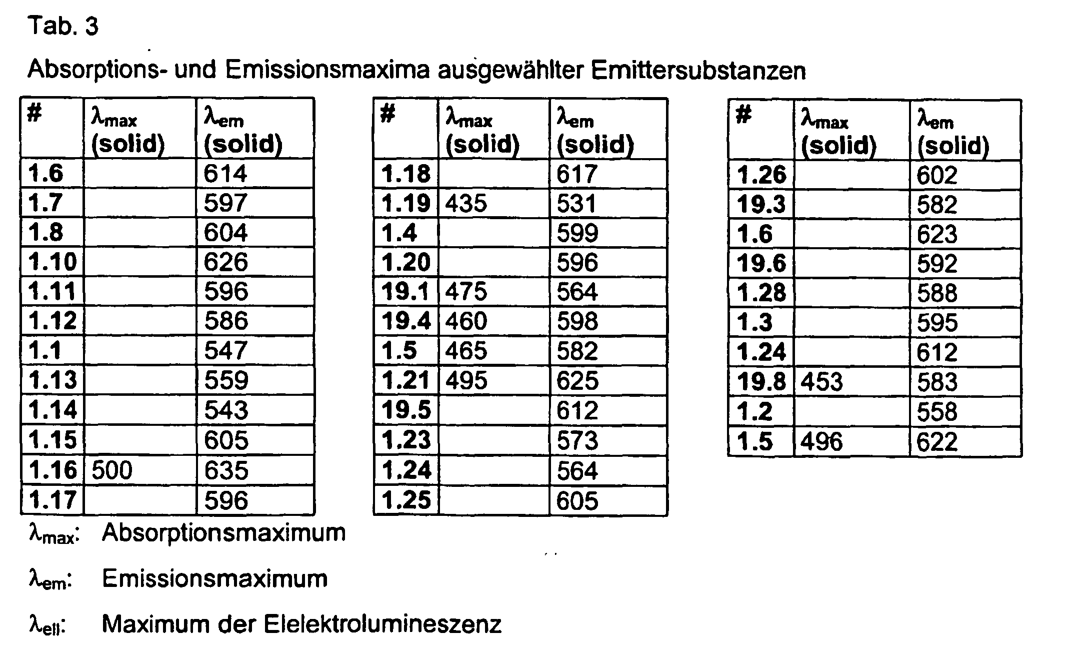

**Tab. 4**

| Extinktionskoeffizienten ε ausgewählter Emittersubstanzen | | | |
|---|---|---|---|
| **#** | **λ**_{**max**} **(nm)** | **ε (I.mol**^{**-1**}**. cm**^{**-1**}**)** | **Lösungsmittel** |
| **1.16** | 489 | 6000 | CHCl₃ |
| **1.5** | 469 | 6640 | CHCl₃ |
| **1.34** | 403 | 4744 | NMP |
| **19.6** | 452 | 5250 | CHCl₃ |
| **19.5** | 474 | 4670 | CHCl₃ |
| **19.7** | 433 | 5450 | NMP |
| **1.17** | 472 | 6410 | CHCl₃ |
| **1.15** | 486 | 5930 | CHCl₃ |
| **1.12** | 460 | 5930 | CHCl₃ |
| **1.11** | 481 | 6840 | CHCl₃ |
| **1.8** | 472 | 6450 | CHCl₃ |
| **1.7** | 474 | 6550 | CHCl₃ |
| **19.1** | 434 | 4700 | NMP |
| **1.30** | 493 | 5450 | NMP |
| **1.27** | 482 | 6800 | CHCl₃ |

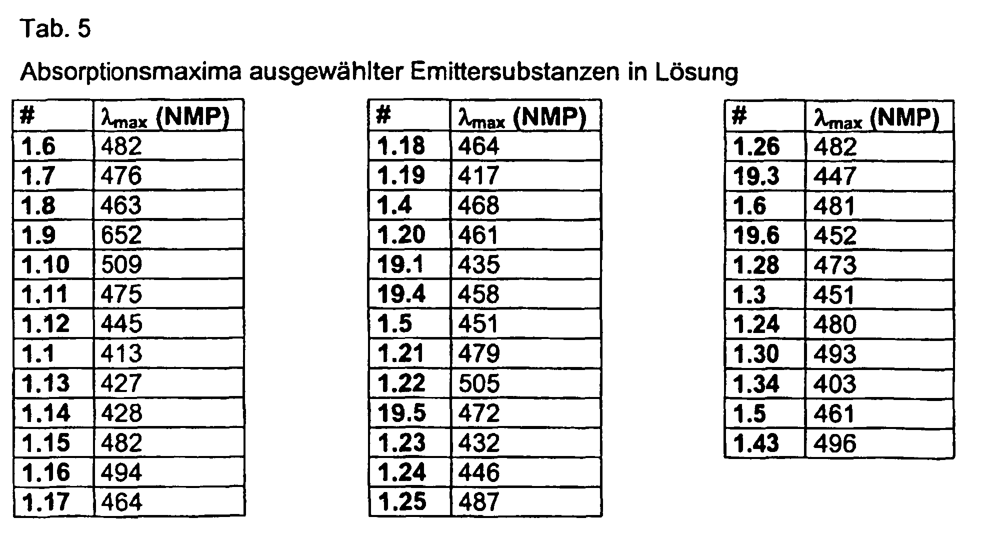

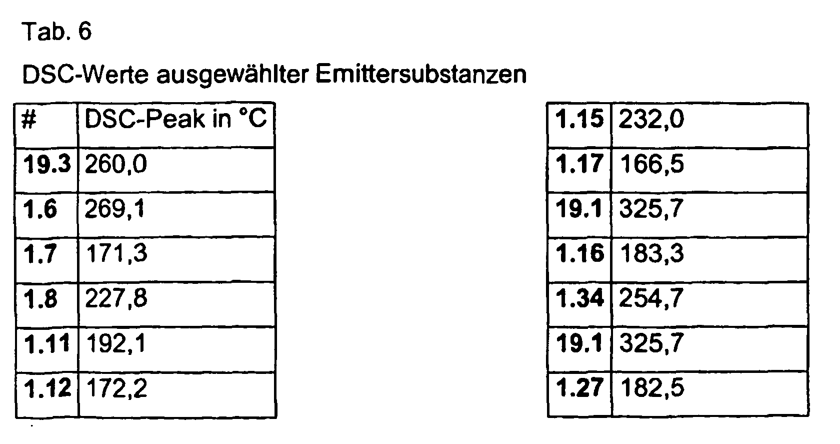

### Präparations- und Messbedingungen

a) Substrat: 125nm ITO mit ca. 13Ω/sq und 85% Transmission 50×50mm²-Glassubstrate (1,1mm dickes poliertes Soda Lime-Floatglas mit SiO₂-Schicht und 8 einzelnen ITO-Anoden (aktiven Fläche: 2x2mm²))
   2 x 20 min im Ultraschallbad mit Aceton selectopur und mit Methanol selectopur gereinigt,
   3 x Snow Jet Cleaning (CO₂-Eiskristalle)
   O2-Plasmabehandlung (5 min bei 450 W und 0,12 mbar)
b) Druck (2-4)x10⁻⁵mbar beim Bedampfen
   Aluminiumoxidkeramik-Tiegel
   Bedampfungsrate: 0,06nm/s
   Kontrolle der Schichtdicke über Schwingquartz-Mikrobalance-Messgerät
   Maskenwechsel mit Zwischenbelüftung der Bedampfungskammer zuerst mit Stickstoff und dann mit Luft
   Kathoden: jeweils 0,5nm Lithiumtluorid (isolierend) und 100nm Aluminium
c) Das Device nach Fig. 2 wurde in eine Glovebox eingeschleust, aktive OLED-Fläche in einer abgedunkelten Meßeinrichtung über kalibrierten V_{λ}-Silizium-Photodioden positioniert und die ausgeführten Anoden (ITO) - und Kathoden (Al) - Kontakte mit vergoldeten Feder-Elektroden kontaktiert;
   programmierbare Spannungsquelle (SMU) und Digitalmultimeter zur Aufnahme und Verarbeitung der OLED-Kennlinien am PC über GPIB-BUS und LabView-Programm Spannungs-Impulsbetrieb (Pulsdauer 1s) von -10V bis +15V (Schrittweite 0,5V): Stromdichte-Spannungs-Kennlinie und Leuchtdichte-Spannungs-Kennlinie sowie die berechneten Werte von photometrische Effizienz (in cd/A) und Leistungs-Effizienz (in Im/W) gegen U
d) Wellenlänge des Maximums durch Aufzeichnung des Elektrolumineszenz-Spektrums am Xdap-Diodenarray-Spektrometer;

## Patentansprüche

1. Organische elektrolumineszierende Vorrichtung, **dadurch gekennzeichnet, daß** sie 2,5-Diaminoterephthalsäurederivate der nachfolgenden Formel **1a** in einer oder mehreren Emitterschichten rein oder dotiert in einem Device enthält 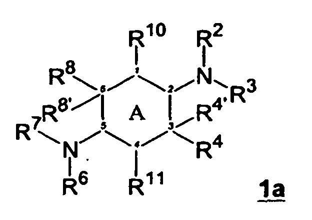 worin der Ring A ein dreifach ungesättigter Benzolring ist, in dem R^{4'} und R^{8'} entfallen, oder der Ring A ist ein ungesättigter Ring mit zwei isolierten Doppelbindungen in 1,2- und 4,5-Position, und
worin R¹⁰ einen Nitrilrest -CN oder einen Rest -C(=X¹)-X²R¹ bedeutet,
R¹¹ ist ein Nitrilrest -CN oder ein Rest -C(=X³)-X⁴R⁵ ,
X¹ und X³, die gleich oder verschieden sind, sind Sauerstoff, Schwefel oder Imino,
X² und X⁴, die gleich oder verschieden sind, sind Sauerstoff, Schwefel oder gegebenenfalls substituiertes Amino,
R¹ bis R⁸, R^{4'} und R^{8'}, die gleich oder verschieden sein können, sind Wasserstoff, C₁-C₂₀-Alkyl, Aryl, Heteroaryl, wobei Aryl und Heteroaryl durch gleiche oder verschiedene Reste Di-C₁-C₃-amino, C₁-C₁₀-Alkoxy, C₁-C₄-Alkyl, Cyan, Fluor , Chlor und Brom sowie Phenyl ein- oder mehrfach substituiert sein können,
R⁴ und R⁸ können auch Halogen, Nitro, Cyan oder Amino sein,
R² bis R⁴ , R⁶ bis R⁸ , R^{4'} und R^{8'} können auch Trifluormethyl, 2-Fluorphenyl, 3-Fluorphenyl, 4-Fluorphenyl, 2,4-Difluorphenyl, 2,6-Difluorphenyl, 2,3,4,5-Tetrafluorphenyl oder Pentafluorphenyl sein,
und worin die folgenden Reste einen gesättigten oder ungesättigten Ring bilden können X¹ und X², R¹ und R², R² und X², R² und R³, R³ und R⁴, R⁴ und X³, X³ und X⁴, R⁵ und X⁴, R⁶ und X⁴, R⁶ und R⁷, R⁷ und R⁸, R⁸ und X¹, R³ und R^{4'}, R⁷ und R^{8'}, R⁴ und R^{4'} und R⁸ und R^{8'}, an die weitere Ringe ankondensiert sein können.

2. Vorrichtung nach Anspruch 1, worin Alkyl C₁-C₈-Alkyl bedeutet, Aryl ist Phenyl oder Naphthyl , und Heteroaryl ist Cumaryl, Pyridyl, Chinolyl, Indolyl, Carbazolyl, Imidazolyl, Thienyl, Thiazolyl, Furyl oder Oxazolyl.

3. Vorrichtung nach Anspruch 1 oder 2 , worin R², R³, R⁶ und R⁷ Trifluormethyl, 2-Fluorphenyl, 3-Fluorphenyl, 4-Fluorphenyl, 2,4-Difluorphenyl, 2,6-Difluorphenyl, 2,3,4,5-Tetrafluorphenyl oder Pentafluorphenyl sind, R⁴ und R⁸ sind Halogen, Nitro, Cyan oder Amino, und die anderen Substituenten haben die in Anspruch 1 genannte Bedeutung.

4. Vorrichtung nach Anspruch 1 oder 2, worin R⁴ und R⁸ Trifluormethyl, 2-Fluorphenyl, 3-Fluorphenyl, 4-Fluorphenyl, 2,4-Difluorphenyl, 2,6-Difluorphenyl, 2,3,4,5-Tetrafluorphenyl oder Pentafluorphenyl sind, und die anderen Substituenten haben die in Anspruch 1 genannte Bedeutung.

5. Vorrichtung nach einem der Ansprüche 1 bis 4, worin der Alkylrest in Alkoxy die Bedeutung C₁-C₄ hat.

6. Vorrichtung nach einem der Ansprüche 1 bis 5, worin X¹ und X³ Sauerstoff darstellen.

7. Vorrichtung nach Anspruch 1, worin Verbindungen der nachfolgenden allgemeinen Formel **1** in der einen oder in den mehreren Emitterschichten, rein oder dotiert, in dem Device enthalten sind, 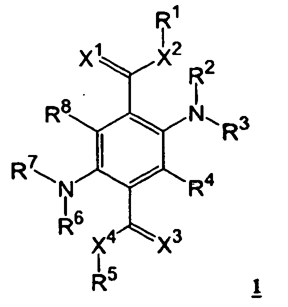 wobei
X¹ und X³ gleiche oder ungleiche Atome oder Gruppen, Sauerstoff, Schwefel oder Imino sein können;
X² und X⁴ gleiche oder ungleiche Atome oder Gruppen, Sauerstoff, Schwefel oder Amino, wobei der Aminostickstoff substituiert sein kann, sein können;
R¹ bis R⁸ gleiche oder ungleiche Substituenten, Wasserstoff, Alkyl mit 1 bis 20 C-Atomen; Aryl, wie Phenyl oder Naphthyl, Heteroaryl, wie Pyridyl, Thienyl, Furyl sein können und diese Reste durch Atome oder Gruppen wie Dialkylamino, Alkoxy, Cyano, Fluor oder Chlor substituiert sein können;
R⁴ und R⁸ auch gleich oder ungleich Halogen, Nitro, Cyano oder Amino sein können.

8. Vorrichtung nach einem der Ansprüche 1 bis 7, worin die Verbindungen der nachfolgenden
Formel **17** enthalten sind 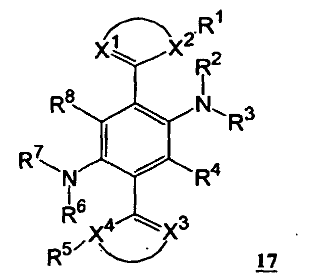 wobei
X¹ und X² sowie X³ und X⁴ Glieder eines 5- bis 6-gliedrigen Ringes sein können,
die einen gesättigten oder ungesättigten Heterocyclus mit oder ohne weitere Annelierung aufbauen und X¹ gleich Imino ist, X², X³, X⁴, R¹ bis R⁸ die in Anspruch 1 genannte Bedeutung haben, und R⁴ und R⁸ auch Halogen, Nitro, Cyano oder Amino sein können.

9. Vorrichtung nach einem der Ansprüche 1 bis 7, worin die Verbindungen der nachfolgenden
Formel **18** enthalten sind 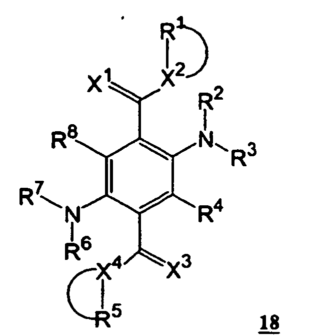 wobei
R¹ und X² sowie R⁵ und X⁴ Glieder eines 5- bis 6-gliedrigen Ringes sein können,
die einen gesättigten oder ungesättigten Heterocyclus mit oder ohne weitere Annelierung aufbauen, und X¹ bis X⁴, R² bis R⁴ und R⁶ bis R⁸ die in Anspruch 1 genannte Bedeutung haben, und R⁴ und R⁸ auch gleich oder ungleich Halogen, Nitro, Cyano oder Amino sein können.

10. Vorrichtung nach einem der Ansprüche 1 bis 7, worin die Verbindungen der nachfolgenden Formel **19** enthalten sind 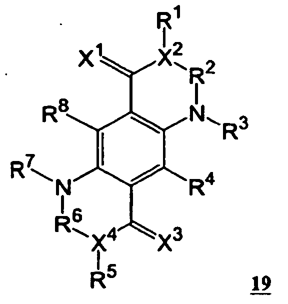 wobei
X² und R² sowie X⁴ und R⁶ Glieder eines 5- bis 6-gliedrigen Ringes sein können,
die einen gesättigten oder ungesättigten Heterocyclus mit oder ohne weitere Annelierung aufbauen, und X¹ bis X⁴, R¹, R³ bis R⁵, R⁷ und R⁸ die in Anspruch 1 genannte Bedeutung haben, und R⁴ und R⁸ auch gleich oder ungleich Halogen, Nitro, Cyano oder Amino sein können.

11. Vorrichtung nach einem der Ansprüche 1 bis 7, worin die Verbindungen der nachfolgenden Formel **20a** enthalten sind 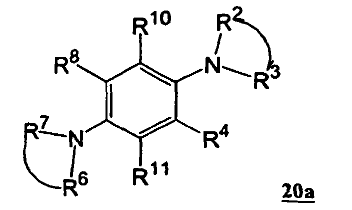 wobei
R¹⁰ einen Nitrilrest -CN oder einen Rest -C(=X¹)-X²R¹ bedeutet,
R¹¹ ist ein Nitrilrest -CN oder ein Rest -C(=X³)-X⁴R⁵ ,
R² und R³ sowie R⁶ und R⁷ Glieder eines 5- bis 6-gliedrigen Ringes sein können,
die einen gesättigten oder ungesättigten Heterocyclus mit oder ohne weitere Annelierung aufbauen, und X¹ bis X⁴, R¹, R⁴, R⁵ und R⁸ die in Anspruch 1 genannte Bedeutung haben, und R⁴ und R⁸ auch gleich oder ungleich Halogen, Nitro, Cyano oder Amino sein können.

12. Vorrichtung nach einem der Ansprüche 1 bis 7, worin die Verbindungen der nachfolgenden Formel **21a** enthalten sind 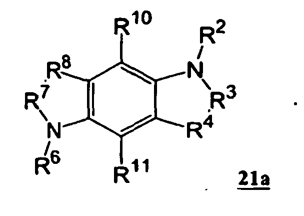 worin R¹⁰ einen Nitrilrest -CN oder einen Rest -C(=X¹)-X²R¹ bedeutet,
R¹¹ ist ein Nitrilrest -CN oder ein Rest -C(=X³)-X⁴R⁵, und
wobei R³ und R⁴ sowie R⁷ und R⁸ Glieder eines 5- bis 6-gliedrigen Ringes sein können, die einen gesättigten oder ungesättigten Heterocyclus mit oder ohne weitere Annelierung aufbauen, und X¹ bis X⁴, R¹, R², R⁵, R⁶ die in Anspruch 1 genannte Bedeutung haben, und R⁴ und R⁸ auch gleich oder ungleich Halogen, Nitro, Cyano oder Amino sein können.

13. Vorrichtung nach einem der Ansprüche 1 bis 7, worin die Verbindungen der nachfolgenden Formel **22** enthalten sind 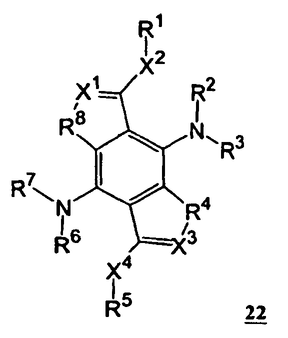 wobei
R⁴ und X³ sowie R⁸ und X¹ Glieder eines 5- bis 6-gliedrigen Ringes sein können,
die einen gesättigten oder ungesättigten Heterocyclus mit oder ohne weitere Annelierung aufbauen, und X¹ und X⁴ gleich Imino sind, X² und X³, R¹ bis R³ und R⁵ bis R⁷ die in Anspruch 1 genannte Bedeutung haben, und R⁴ und R⁸ auch gleich oder ungleich Halogen, Nitro, Cyano oder Amino sein können.

14. Vorrichtung nach einem der Ansprüche 1 - 13, worin die Verbindungen aus den Ansprüchen 1-13 in den einen oder in den mehreren Emitterschichten als Gemisch von 2 - 6 unterschiedlichen Vertretern in dem Device enthalten ist.

15. Vorrichtung nach einem der Ansprüche 1 bis 6, worin die Verbindungen der nachfolgenden Formel **2** enthalten sind, 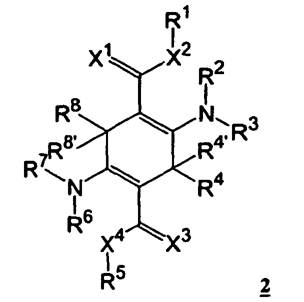 wobei X¹ und X³ sowie X² und X⁴, R¹ bis R⁸, R^{4'} und R^{8'} die in Anspruch 1 angegebene Bedeutung haben, und R⁴ und R⁸ sowie R^{4'} und R^{8'} auch gleich oder ungleich Halogen, Nitro, Cyano, Amino, Trifluormethyl, 2-Fluorphenyl, 3-Fluorphenyl, 4-Fluorphenyl, 2,4-Difluorphenyl, 2,6-Difluorphenyl, 2,3,4,5-Tetrafluorphenyl oder Pentafluorphenyl sein können.

16. Vorrichtung nach einem der Ansprüche 1 bis 6, worin die Verbindungen der nachfolgenden Formel **37** enthalten sind, 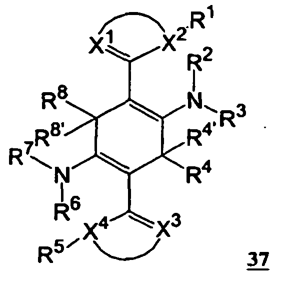 wobei
X¹ und X² sowie X³ und X⁴ Glieder eines 5- bis 6-gliedrigen Ringes sein können,
die einen gesättigten oder ungesättigten Heterocyclus mit oder ohne weitere Annelierung aufbauen, X¹ und X³ sind Imino, X² und X⁴ sowie
R¹ bis R⁸ die in Anspruch 1 genannte Bedeutung haben, und R⁴ und R⁸ sowie R^{4'} und R^{8'} auch gleich oder ungleich Halogen, Nitro, Cyano oder Amino sein können.

17. Vorrichtung nach einem der Ansprüche 1 bis 6, worin die Verbindungen der nachfolgenden Formel **38** enthalten sind, 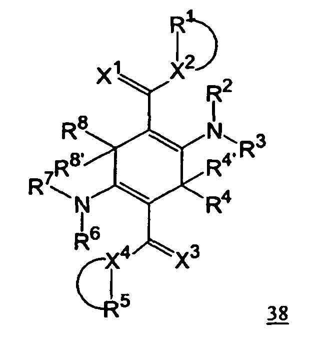 wobei
R¹ und X² sowie R⁵ und X⁴ Glieder eines 5- bis 6-gliedrigen Ringes sein können,
die einen gesättigten oder ungesättigten Heterocyclus mit oder ohne weitere Annelierung aufbauen, und X¹ bis X⁴, R² bis R⁴ und R⁶ bis R⁸ die in Anspruch 1 genannte Bedeutung haben, und R⁴ und R⁸ sowie R^{4'} und R^{8'} auch gleich oder ungleich Halogen, Nitro, Cyano oder Amino sein können.

18. Vorrichtung nach einem der Ansprüche 1 bis 6, worin die Verbindungen der nachfolgenden Formel **39** enthalten sind, 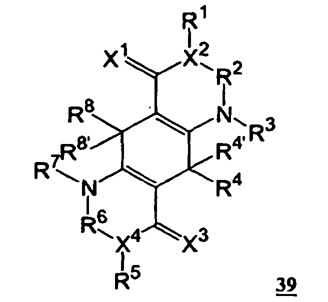 wobei X² und R² sowie X⁴ und R⁶ Glieder eines 5- bis 6-gliedrigen Ringes sein können, die einen gesättigten oder ungesättigten Heterocyclus mit oder ohne weitere Annelierung aufbauen, und X¹ bis X⁴, R¹, R³ bis R⁵, R⁷ und R⁸ die in Anspruch 1 genannte Bedeutung haben, und R⁴ und R⁸ sowie R^{4'} und R^{8'} auch gleich oder ungleich Halogen, Nitro, Cyano oder Amino sein können.

19. Vorrichtung nach einem der Ansprüche 1 bis 6, worin die Verbindungen der nachfolgenden Formel **40a** enthalten sind, 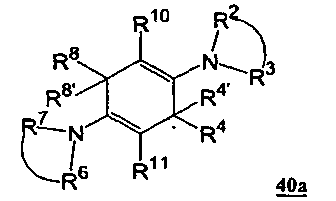 worin R¹⁰ einen Nitrilrest -CN oder einen Rest -C(=X¹)-X²R¹ bedeutet,
R¹¹ ist ein Nitrilrest -CN oder ein Rest -C(=X³)-X⁴R⁵ ,
und wobei R² und R³ sowie R⁶ und R⁷ Glieder eines 5- bis 6-gliedrigen Ringes sein können, die einen gesättigten oder ungesättigten Heterocyclus mit oder ohne weitere Annelierung aufbauen, und X¹ bis X⁴, R¹, R⁴, R⁵ und R⁸ die in Anspruch 1 genannte Bedeutung haben, und R⁴ und R⁸ sowie R^{4'} und R^{8'} auch gleich oder ungleich Halogen, Nitro, Cyano oder Amino sein können.

20. Vorrichtung nach einem der Ansprüche 1 bis 6, worin die Verbindungen der nachfolgenden Formel **41a** enthalten sind, 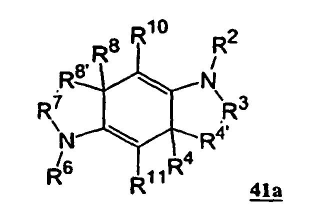 worin R¹⁰ einen Nitrilrest -CN oder einen Rest -C(=X¹)-X²R¹ bedeutet,
R¹¹ ist ein Nitrilrest -CN oder ein Rest -C(=X³)-X⁴R⁵ , wobei
R³ und R⁴ sowie R⁷ und R⁸ Glieder eines 5- bis 6-gliedrigen Ringes sein können,
die einen gesättigten oder ungesättigten Heterocyclus mit oder ohne weitere Annelierung aufbauen, und X¹ bis X⁴, R¹, R², R⁵, R⁶ die in Anspruch 1 genannte Bedeutung haben, und R⁴ und R⁸ auch gleich oder ungleich Halogen, Nitro, Cyano oder Amino sein können.

21. Vorrichtung nach einem der Ansprüche 1 bis 6, worin die Verbindungen der nachfolgenden Formel **43** enthalten sind, 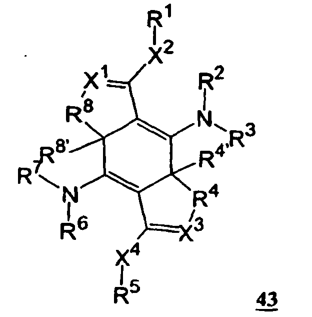 wobei R⁴ und X³ sowie R⁸ und X¹ Glieder eines 5- bis 6-gliedrigen Ringes sein können, die einen gesättigten oder ungesättigten Heterocyclus mit oder ohne weitere Annelierung aufbauen, und X¹ und X³ sind Imino, X² und X⁴ sowie R¹ bis R³ und R⁵ bis R⁷ die in Anspruch 1 genannte Bedeutung haben, und R^{4'} und R^{8'} auch gleich oder ungleich Halogen, Nitro, Cyano oder Amino sein können.

22. Vorrichtung nach einem der Ansprüche 1 bis 6, worin die Verbindungen der nachfolgenden Formel **42a** enthalten sind, 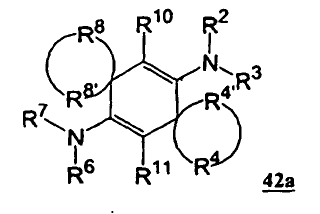 worin R¹⁰ einen Nitrilrest -CN oder einen Rest -C(=X¹)-X²R¹ bedeutet,
R¹¹ ist ein Nitrilrest -CN oder ein Rest -C(=X³)-X⁴R⁵ ,
und wobei R⁴ und R^{4'} sowie R⁸ und R^{8'} Glieder eines 5- bis 6-gliedrigen Ringes sein können, die einen gesättigten oder ungesättigten Heterocyclus mit oder ohne weitere Annelierung aufbauen, und X¹ bis X⁴, R¹ bis R³ und R⁵ bis R⁷ die in Anspruch 1 genannte Bedeutung haben.

23. Vorrichtung nach Anspruch 15 bis 22, worin die Verbindungen aus den Ansprüchen 15 - 22 in einer oder mehreren Emitterschichten als Gemisch von 2 - 6 unterschiedlichen Vertretern in einem Device enthalten sind.

24. Vorrichtung nach einem der Ansprüche 1 bis 6, worin die Verbindungen der nachfolgenden Formel **3** oder **4** enthalten sind, 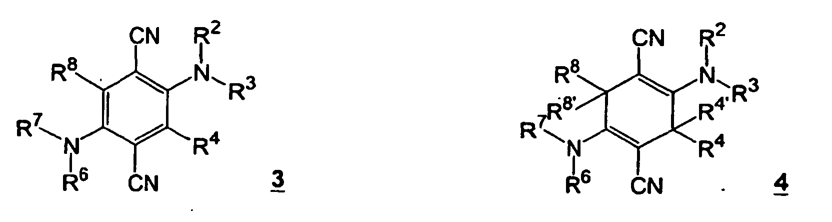 Wobei R² bis R⁸ die in Anspruch 1 und 2 genannte Bedeutung haben, und R⁴ und R⁸ auch gleich oder ungleich Halogen, Nitro, Cyano oder Amino sein können.

25. 2,5-Diaminoterephthalsäurederivate der Formel **19****,** 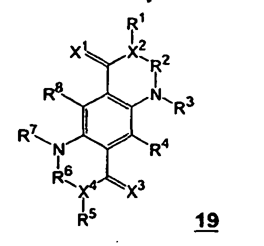 wobei
X¹ gleich O und X² gleich O oder N ist; R² und R⁶ sind Methylen (-CH₂-), das durch Trifluormethyl substituiert sein kann, R³ und R⁷ sind gleich oder verschieden H, C₁-C₈-Alkyl, Aryl oder Heteroaryl, und R⁴ und R⁸ sind gleich oder verschieden H, Alkyl, Aryl oder Trifluormethyl, und R¹ ist null, H oder C₁-C₄-Alkyl.

26. 2,5-Diaminoterephthalsäurederivate der Formel **2**, 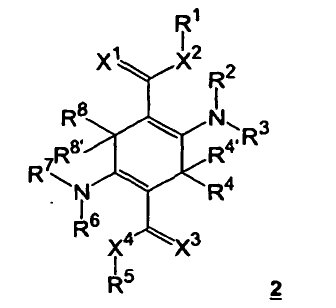 wobei
X¹ gleich O und X² gleich O oder N ist; R² und R⁶ sowie R³ und k⁷ sind gleich oder verschieden H, C₁-C₈-Alkyl, Aryl oder Heteroaryl, und R⁴ und R⁸ sowie R^{4'} und R^{8'} sind ungleich H und gleich oder verschieden Alkyl, Aryl oder Trifluormethyl, und R¹ ist H oder C₁-C₄-Alkyl.

27. 2,5-Diaminoterephthalsäurederivate der Formel **3,** 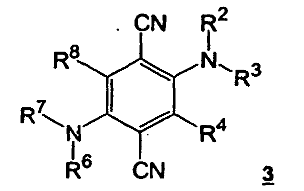 wobei R² und R⁶ sowie R³ und R⁷ sind gleich oder verschieden H, C₁-C₈-Alkyl, Aryl oder Heteroaryl, und R⁴ und R⁸ sind ungleich H und gleich oder verschieden Alkyl, Aryl oder Trifluormethyl.

28. 2,5-Diaminoterephthalsäurederivate der Formel **4****,** 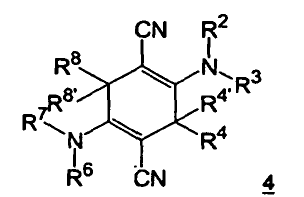 wobei R² und R⁶ sowie R³ und R⁷ sind gleich oder verschieden H, C₁-C₈-Alkyl, Aryl oder Heteroaryl, und R⁴ und R⁸ sowie R^{4'} und R^{8'} sind ungleich H und gleich oder verschieden Alkyl, Aryl oder Trifluormethyl.

29. Derivate nach einem der Ansprüche 25 bis 28, worin Alkyl C₁-C₄-Alkyl ist, Aryl ist Phenyl oder Naphthyl und Heteroaryl ist Pyridyl, Thienyl oder Furyl.

30. Vorrichtung nach Anspruch 1 und 2, worin R¹⁰ und R¹¹ die in den Ansprüchen 1 und 2 genannte Bedeutung haben, R² und R⁶ sind Wasserstoff oder C₁-C₄-Alkyl, R³ und R⁷ sind jeweils Phenyl, einfach oder zweifach substituiert durch H, Halogen, CN, C₁-C₄-Alkyl, C₁-C₄-Alkoxy, Halo-C₁-C₄-alkyl oder Phenyl.

31. Vorrichtung nach Anspruch 10, worin X¹ bis X⁴ Sauerstoff sind, R² und R⁴ jeweils durch gleiche oder verschiedene Reste H und C₁-C₄-Alkyl substituiert sind, R³ und R⁷ sind jeweils Phenyl, einfach oder zweifach substituiert durch H, Halogen, CN, C₁-C₄-Alkyl, C₁-C₄-Alkoxy, Halo- C₁-C₄-alkyl oder Phenyl, und R⁴ und R⁸ Wasserstoff sind.

32. Vorrichtung nach einem der Ansprüche 31 und 32, worin der C₁-C₄-Alkylrest ein C₁-Rest ist.

## Claims

1. An organic electroluminescent device comprising 2,5-diaminoterephthalic acid derivatives of the following formula **1a** in one or several emitter layers in a pure or doped form in a device 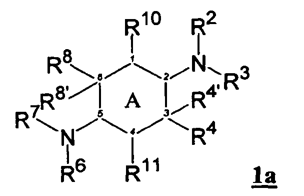 wherein the ring A is a triply unsaturated benzene ring wherein R^{4'} and R^{8'} are omitted, or the ring A is an unsaturated ring having two isolated double bonds in the 1,2-position and the 4,5-position, and
wherein R¹⁰ represents a nitrile radical -CN or a radical -C(=X¹)-X²R¹,
R¹¹ is a nitrile radical -CN or a radical -C(=X³)-X⁴R⁵,
X¹ and X³, which are the same or different, are oxygen, sulphur or imino,
X² and X⁴, which are the same or different, are oxyen, sulphur or substituted or unsubstituted amino,
R¹ to R⁸, R^{4'} and R^{8'}, which can be the same or different, are hydrogen, C1-C20 alkyl, aryl, heteroaryl, wherein aryl and heteroaryl can be substituted singly or multiply with the same or different radicals di-C1-C3-amino, C1-C10 alkoxy, C1-C4 alkyl, cyano, fluorine, chlorine and bromine as well as phenyl,
R⁴ and R⁸ can also be halogen, nitro, cyano or amino,
R² to R⁴, R⁶ to R⁸, R^{4'} and R^{8'} can also be trifluoromethyl, 2-fluorophenyl, 3-fluorophenyl, 4-fluorophenyl, 2,4-difluorophenyl, 2,6-difluorophenyl, 2,3,4,5-tetrafluorophenyl or pentafluorophenyl,
and wherein the following radicals can form a saturated or unsaturated ring
X¹ and X², R¹ and R², R² and X², R² and R³, R³ and R⁴, R⁴ and X³, X³ and X⁴, R⁵ and X⁴, R⁶ and X⁴, R⁶ and R⁷, R⁷ and R⁸, R⁸ and X¹, R³ and R^{4'}, R⁷ and R^{8'}, R⁴ and R⁴', and R⁸ and R^{8'}, to which ring further rings can be fused.

2. A device according to Claim 2, wherein alkyl means C1-C8 alkyl, aryl is phenyl or naphthyl, and heteroaryl is cumaryl, pyridyl, chinolyl, indolyl, carbazolyl, imidazolyl, thienyl, thiazolyl, furyl or oxazolyl.

3. A device according to Claim 1 or 2, wherein R², R³, R⁶ and R⁷ are trifluoromethyl, 2-fluorophenyl, 3-fluorophenyl, 4-fluorophenyl, 2,4-difluorophenyl, 2,6-difluorophenyl, 2,3,4,5-tetrafluorophenyl or pentafluorophenyl, R⁴ and R⁸ are halogen, nitro, cyano or amino, and the other substituents have the meaning indicated in Claim 1.

4. A device according to Claim 1 or 2, wherein R⁴ and R⁸ are trifluoromethyl, 2-fluorophenyl, 3-fluorophenyl, 4-fluorophenyl, 2,4-difluorophenyl, 2,6-difluorophenyl, 2,3,4,5-tetrafluorophenyl or pentafluorophenyl, and the other substituents have the meaning indicated in Claim 1.

5. A device according to any one of Claims 1 to 4, wherein the alkyl radical in alkoxy has the meaning C1-C4.

6. A device according to any one of Claims 1 to 5, wherein X¹ and X³ represent oxygen.

7. A device according to Claim 1, wherein compounds of the following general formula **1** are contained in the one or in the several emitter layers, in a pure or doped form, in the device 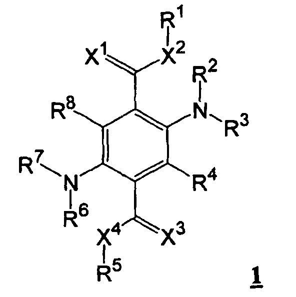 wherein
X¹ and X³ can be the same or different atoms or groups, oxygen, sulphur or imino;
X² and X⁴ can be the same or different atoms or groups, oxygen, sulphur or amino, wherein the amino nitrogen can be substituted;
R¹ to R⁸ can be the same or different substituents, hydrogen, alkyl having 1 to 20 C atoms; aryl, e.g. phenyl or naphthyl; heteroaryl, e.g. pyridyl, thienyl, furyl, and the aforesaid radicals can be substituted with atoms or groups, e.g. dialkylamino, alkoxy, cyano, fluorine or chlorine;
R⁴ and R⁸ can also be or be different from halogen, nitro, cyano or amino.

8. A device according to any one of Claims 1 to 7, wherein the compounds of the following formula **17** are contained 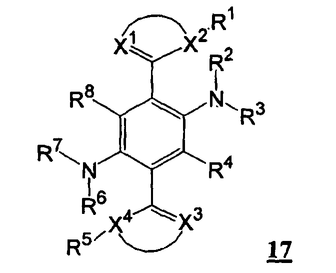 wherein
X¹ and X² as well as X³ and X⁴ can be members of a 5- to 6-membered ring,
forming a saturated or unsaturated heterocycle with or without further anellation, and X¹ is imino, X², X³, X⁴, R¹ to R⁸ have the meaning indicated in Claim 1, and R⁴ and R⁸ can also be halogen, nitro, cyano or amino.

9. A device according to any one of Claims 1 to 7, wherein the compounds of the following formula **18** are contained 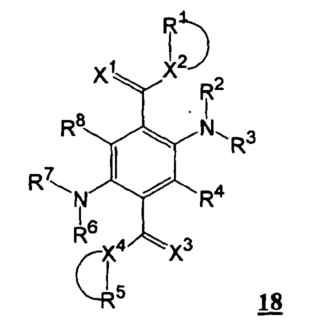 wherein
R¹ and X² as well as R⁵ and X⁴ can be members of a 5- to 6-membered ring,
forming a saturated or unsaturated heterocycle with or without further anellation, and X¹ to X⁴, R² to R⁴ and R⁶ to R⁸ have the meaning indicated in Claim 1, and R⁴ and R⁸ can also be or be different from halogen, nitro, cyano or amino.

10. A device according to any one of Claims 1 to 7, wherein the compounds of the following formula **19** are contained 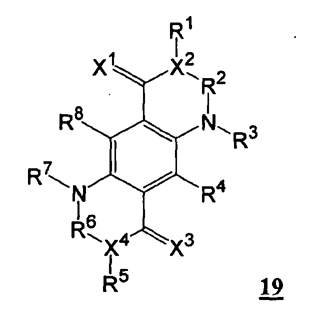 wherein
X² and R² as well as X⁴ and R⁶ can be members of a 5- to 6-membered ring,
forming a saturated or unsaturated heterocycle with or without further anellation, and X¹ to X⁴, R¹, R³ to R⁵, R⁷ and R⁸ have the meaning indicated in Claim 1, and R⁴ and R⁸ can also be or be different from halogen, nitro, cyano or amino.

11. A device according to any one of Claims 1 to 7, wherein the compounds of the following formula **20a** are contained 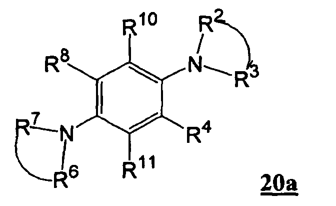 wherein
R¹⁰ represents a nitrile radical -CN or a radical -C(=X¹)-X²R¹,
R¹¹ is a nitrile radical -CN or a radical -C(=X³)-X⁴R⁵,
R² and R³ as well as R⁶ and R⁷ can be members of a 5- to 6-membered ring,
forming a saturated or unsaturated heterocycle with or without further anellation, and X¹ to X⁴, R¹, R⁴, R⁵ and R⁸ have the meaning indicated in Claim 1, and R⁴ and R⁸ can also be or be different from halogen, nitro, cyano or amino.

12. A device according to any one of Claims 1 to 7, wherein the compounds of the following formula **21a** are contained 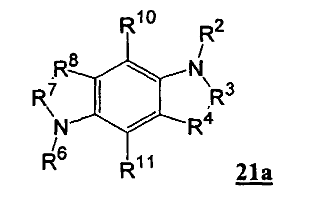 wherein
R¹⁰ represents a nitrile radical -CN or a radical -C(=X¹)-X²R¹,
R¹¹ is a nitrile radical -CN or a radical -C(=X³)-X⁴R⁵, and
wherein
R³ and R⁴ as well as R⁷ and R⁸ can be members of a 5- to 6-membered ring,
forming a saturated or unsaturated heterocycle with or without further anellation, and X¹ to X⁴, R¹, R², R⁵, R⁶ have the meaning indicated in Claim 1, and R⁴ and R⁸ can also be or be different from halogen, nitro, cyano or amino.

13. A device according to any one of Claims 1 to 7, wherein the compounds of the following formula **22** are contained 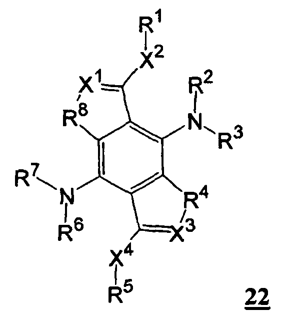 wherein
R⁴ and X³ as well as R⁸ and X¹ can be members of a 5- to 6-membered ring,
forming a saturated or unsaturated heterocycle with or without further anellation, and X¹ and X⁴ are imino, X² and X³, R¹ to R³ and R⁵ to R⁷ have the meaning indicated in Claim 1, and R⁴ and R⁸ can also be or be different from halogen, nitro, cyano or amino.

14. A device according to any one of Claims 1-13, wherein the compounds of Claims 1-13 are contained in the device in the in the one or in the several emitter layers as a mixture of 2-6 different substances.

15. A device according to any one of Claims 1 to 6, wherein the compounds of the following formula **2** are contained, 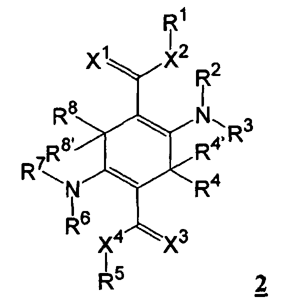 wherein
X¹ and X³ as well as X² and X⁴, R¹ to R⁸, R^{4'} and R^{8'} have the meaning indicated in Claim 1, and R⁴ and R⁸ as well as R^{4'} and R^{8'} can also be or be different from halogen, nitro, cyano, amino, trifluoromethyl, 2-fluorophenyl, 3-fluorophenyl, 4-fluorophenyl, 2,4-difluorophenyl, 2,6-difluorophenyl, 2,3,4,5-tetrafluorophenyl or pentafluorophenyl.

16. A device according to any one of Claims 1 to 6, wherein the compounds of the following formula **37** are contained, 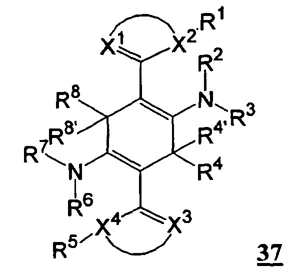 wherein
X¹ and X² as well as X³ and X⁴ can be members of a 5- to 6-membered ring,
forming a saturated or unsaturated heterocycle with or without further anellation, X¹ and X³ are imino, X² and X⁴ as well as
R¹ to R⁸ have the meaning indicated in Claim 1, and R⁴ and R⁸ as well as R^{4'} and R^{8'} can also be or be different from halogen, nitro, cyano or amino.

17. A device according to any one of Claims 1 to 6, wherein the compounds of the following formula **38** are contained, 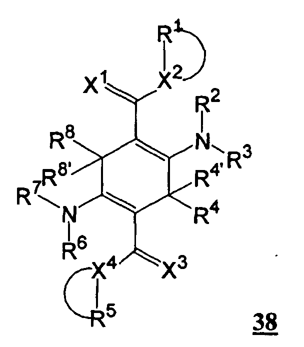 wherein
R¹ and X² as well as R⁵ and X⁴ can be members of a 5- to 6-membered ring,
forming a saturated or unsaturated heterocycle with or without further anellation, and X¹ to X⁴, R² to R⁴ and R⁶ to R⁸ have the meaning indicated in Claim 1, and R⁴ and R⁸ as well as R^{4'} and R^{8'} can also be or be different from halogen, nitro, cyano or amino.

18. A device according to any one of Claims 1 to 6, wherein the compounds of the following formula **39** are contained, 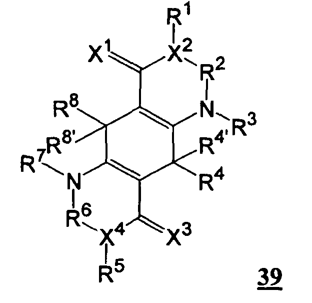 wherein
X² and R² as well as X⁴ and R⁶ can be members of a 5- to 6-membered ring,
forming a saturated or unsaturated heterocycle with or without further anellation, and X¹ to X⁴, R¹, R³ to R⁵, R⁷ and R⁸ have the meaning indicated in Claim 1, and
R⁴ and R⁸ as well as R^{4'} and R^{8'} can also be or be different from halogen, nitro, cyano or amino.

19. A device according to any one of Claims 1 to 6, wherein the compounds of the following formula **40a** are contained, 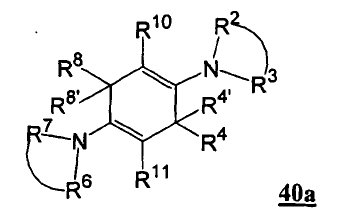 wherein R¹⁰ represents a nitrile radical -CN or a radical -C(=X¹)-X²R¹,
R¹¹ is a nitrile radical -CN or a radical -C(=X³)-X⁴R⁵,
and wherein
R² and R³ as well as R⁶ and R⁷ can be members of a 5- to 6-membered ring,
forming a saturated or unsaturated heterocycle with or without further anellation, and X¹ to X⁴, R¹, R⁴, R⁵ and R⁸ have the meaning indicated in Claim 1, and
R⁴ and R⁸ as well as R^{4'} and R^{8'} can also be or be different from halogen, nitro, cyano or amino.

20. A device according to any one of Claims 1 to 6, wherein the compounds of the following formula **41a** are contained, 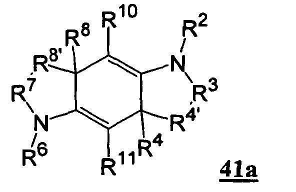 wherein R¹⁰ represents a nitrile radical -CN or a radical -C(=X¹)-X²R¹,
R¹¹ is a nitrile radical -CN or a radical -C(=X³)-X⁴R⁵,
wherein
R³ and R⁴ as well as R⁷ and R⁸ can be members of a 5- to 6-membered ring,
forming a saturated or unsaturated heterocycle with or without further anellation, and X¹ to X⁴, R¹, R², R⁵, R⁶ have the meaning indicated in Claim 1, and R⁴ and R⁸ can also be or be different from halogen, nitro, cyano or amino.

21. A device according to any one of Claims 1 to 6, wherein the compounds of the following formula **43** are contained, 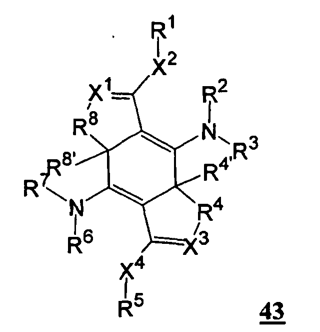 wherein
R⁴ and X³ as well as R⁸ and X¹ can be members of a 5- to 6-membered ring,
forming a saturated or unsaturated heterocycle with or without further anellation, and X¹ and X³ are imino, X² and X⁴ as well as R¹ to R³ and R⁵ to R⁷ have the meaning indicated in Claim 1, and R^{4'} and R^{8'} can also be or be different from halogen, nitro, cyano or amino.

22. A device according to any one of Claims 1 to 6, wherein the compounds of the following formula **42a** are contained, 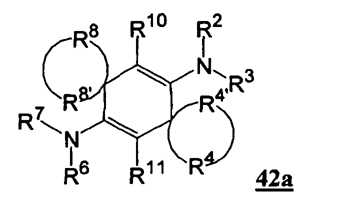 wherein R¹⁰ represents a nitrile radical -CN or a radical -C(=X¹)-X²R¹,
R¹¹ is a nitrile radical -CN or a radical -C(=X³)-X⁴R⁵,
and wherein
R⁴ and R^{4'} as well as R⁸ and R^{8'} can be members of a 5- to 6-membered ring,
forming a saturated or unsaturated heterocycle with or without further anellation, and X¹ to X⁴, R¹ to R³ and R⁵ to R⁷ have the meaning indicated in Claim 1.

23. A device according to Claims 15 to 22, wherein a mixture of 2-6 different substances is contained in a device in one or several emitter layers.

24. A device according to any one of Claims 1 to 6, wherein the compounds of the following formula **3** or **4** are contained, 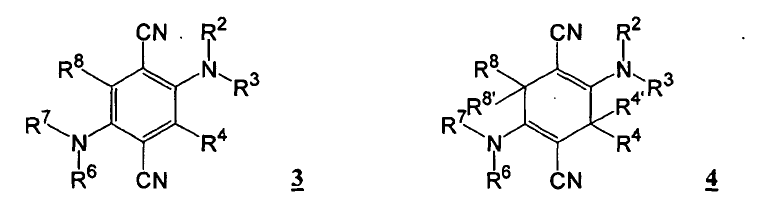 wherein
R² to R⁸ have the meaning indicated in Claim 1, and R⁴ and R⁸ can also be or be different from halogen, nitro, cyano or amino.

25. 2,5-Diaminoterephthalic acid derivatives of the formula **19**, 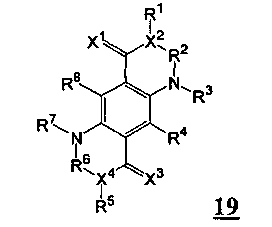 wherein
X¹ is O and X² is O or N; R² and R⁶ are methylene (-CH₂-), which can be substituted with trifluoromethyl, R³ and R⁷ are the same or different, H, C₁-C₈ alkyl, aryl or heteroaryl, and R⁴ and R⁸ are the same or different, H, alkyl, aryl or trifluoromethyl, and R¹ is zero, H or C₁-C₄-Alkyl.

26. 2,5-Diaminoterephthalic acid derivatives of the formula **2**, 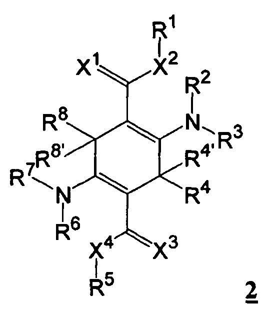 wherein
X¹ is O and X² is O or N; R² and R⁶ as well as R³ and R⁷ are the same or different, H, C₁-C₈ alkyl, aryl or heteroaryl, and R⁴ and R⁸ as well as R^{4'} and R^{8'} are not H and are the same or different, alkyl, aryl or trifluoromethyl and R¹ is H or C₁-C₄-Alkyl.

27. 2,5-Diaminoterephthalic acid derivatives of the formula **3**, 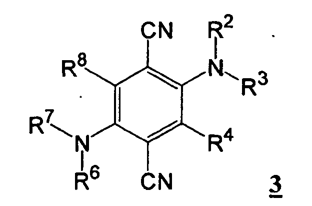 wherein
R² and R⁶ as well as R³ and R⁷ are the same or different, H, C₁-C₈ alkyl, aryl or heteroaryl, and R⁴ and R⁸ are not H and are the same or different, alkyl, aryl or trifluoromethyl.

28. 2,5-Diaminoterephthalic acid derivatives of the formula **4****,** 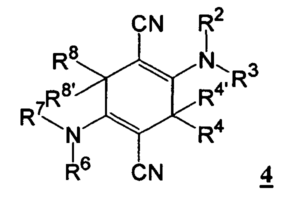 wherein
R² and R⁶ as well as R³ and R⁷ are the same or different, H, C₁-C₈ alkyl, aryl or heteroaryl, and R⁴ and R⁸ as well as R^{4'} and R^{8'} are not H and are the same or different, alkyl, aryl or trifluoromethyl.

29. The derivatives according to one of Claims 25-28, wherein alkyl is C₁-C₄ alkyl, aryl is phenyl or naphthyl, and heteroaryl is pyridyl, thienyl or furyl.

30. A device according to Claims 1 and 2, wherein R¹⁰ and R¹¹ have the meaning indicated therein, R² and R⁶ are hydrogen or C₁-C₄ alkyl, each of R³ and R⁷ are phenyl, which has been substituted singly or doubly with H, halogen, CN, C₁-C₄ alkyl, C₁-C₄ alkoxy, halo-C₁-C₄ alkyl, or phenyl.

31. A device according to Claim 10, wherein X¹ to X⁴ are oxygen, each of R² and R⁴ are substituted with the same or different radicals H and C₁-C₄ alkyl, each of R³ and R⁷ are phenyl, which has been substituted singly or doubly with H, halogen, CN, C₁-C₄ alkyl, C₁-C₄ alkoxy, halo-C₁-C₄ alkyl, or phenyl, and R⁴ and R⁸ are hydrogen.

32. A device according to one of claims 30 and 31, wherein the C₁-C₄ radical is a C₁ radical.

## Revendications

1. Dispositif organique électroluminescent, **caractérisé en ce qu'**il contient dans un dispositif, des dérivés de l'acide 2,5-diaminotéréphthalique de la formule **1a** suivante, dans une ou plusieurs couches émettrices, pures ou dotées, 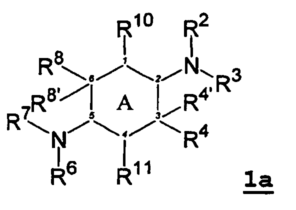 où le cycle A est un cycle benzène trois fois insaturé, dans lequel R^{4'} et R^{8'} disparaissent, ou le cycle A est un cycle insaturé avec deux doubles liaisons isolées en positions 1,2 et 4,5, et
où R¹⁰ représente un reste nitrile -CN ou un reste -C(=X¹)-X²R¹,
R¹¹ est un reste nitrile -CN ou un reste -C(=X³)-X⁴R⁵,
X¹ et X³, qui sont identiques ou différents, sont oxygène, soufre ou imino,
X² et X⁴, qui sont identiques ou différents, sont oxygène, soufre ou amino le cas échéant substitué,
R¹ à R⁸, R^{4'} et R^{8'} , qui peuvent être identiques ou différents, sont hydrogène, alkyle en C₁-C₂₀, aryle, hétéroaryle, où aryle et hétéroaryle peuvent être substitués une ou plusieurs fois, par des restes Di-C₁-C₃-amino, alcoxy en C₁-C₁₀, alkyle en C₁-C₄, cyano, fluor, chlore et brome ainsi que phényle, identiques ou différents,
R⁴ et R⁸ peuvent également être halogène, nitro, cyano ou amino,
R² à R⁴, R⁶ à R⁸, R^{4'} et R^{8'} peuvent également être trifluorométhyle, 2-fluorophényle, 3-fluorophényle, 4-fluorophényle, 2,4-difluorophényle, 2,6-difluorophényle, 2,3,4,5-tétrafluorophényle ou pentafluorophényle,
et où les restes suivants peuvent former un cycle saturé ou insaturé
X¹ et X², R¹ et R², R² et X², R² et R³, R³ et R⁴, R⁴ et X³, X³ et X⁴, R⁵ et X⁴, R⁶ et X⁴, R⁶ et R⁷, R⁷ et R⁸, R⁸ et X¹, R³ et R^{4'} , R⁷ et R^{8'} , R⁴ et R^{4'} et R⁸ et R^{8'}, sur lesquels d'autres cycles peuvent être condensés.

2. Dispositif selon la revendication 1, où alkyle représente alkyle en C₁-C₈, aryle est phényle ou naphtyle , et hétéroaryle est coumaryle, pyridyle, quinoléyle, indolyle, carbazolyle, imidazolyle, thiényle, thiazolyle, furyle ou oxazolyle.

3. Dispositif selon la revendication 1 ou 2, où R², R³, R⁶ et R⁷ sont trifluorométhyle, 2-fluorophényle, 3-fluorophényle, 4-fluorophényle, 2,4-difluorophényle, 2,6-difluorophényle, 2,3,4,5-tétrafluorophényle ou pentafluorophényle, R⁴ et R⁸ sont halogène, nitro, cyano ou amino, et les autres substituants ont la signification donnée à la revendication 1.

4. Dispositif selon la revendication 1 ou 2, où R⁴ et R⁸ sont trifluorométhyle, 2-fluorophényle, 3-fluorophényle, 4-fluorophényle, 2,4-difluorophényle, 2,6-difluorophényle, 2,3,4,5-tétrafluorophényle ou pentafluorophényle, et les autres substituants ont la signification donnée à la revendication 1.

5. Dispositif selon l'une des revendications 1 à 4, où le reste alkyle dans alcoxy a la signification C₁-C₄.

6. Dispositif selon l'une des revendications 1 à 5, où X¹ et X³ représentent oxygène.

7. Dispositif selon la revendication 1, où des composés de la formule générale **1** suivante sont présents dans un dispositif, dans une ou plusieurs couches émettrices, pures ou dotées, 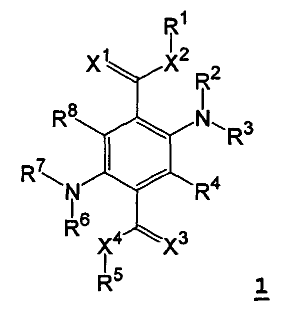 où
X¹ et X³ peuvent être des atomes ou groupes identiques ou différents, oxygène, soufre ou imino;
X² et X⁴ peuvent être des atomes ou groupes identiques ou différents, oxygène, soufre ou amino, où l'atome d'azote peut être substitué;
R¹ à R⁸ peuvent être des substituants identiques ou différents, hydrogène, alkyle avec 1 a 20 atomes C; aryle, comme phényle ou naphtyle, hétéroaryle, comme pyridyle, thiényle, furyle et ces restes peuvent être substitués par des atomes ou groupes comme dialkylamino, alcoxy, cyano, fluor ou chlore;
R⁴ et R⁸ peuvent également être halogène, nitro, cyano ou amino, identiques ou différents.

8. Dispositif selon l'une des revendications 1 à 7, où les composés de la formule **17** suivante sont présents 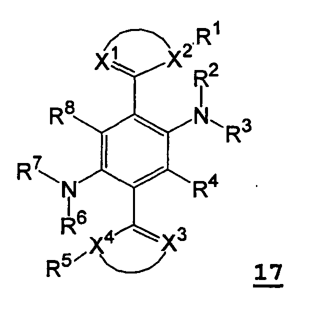 où
X¹ et X² ainsi que X³ et X⁴ peuvent être les membres d'un cycle à 5 ou 6 membres,
qui composent un hétérocycle saturé ou insaturé avec ou sans autre condensation, et X¹ est imino, X², X³, X⁴, R¹ à R⁸ ont la signification donnée à la revendication 1, et R⁴ et R⁸ peuvent être également, halogène, nitro, cyano ou amino.

9. Dispositif selon l'une des revendications 1 à 7, où les composés de la formule **18** suivante sont présents 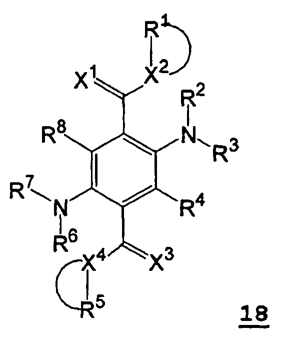 où
R¹ et X² ainsi que R⁵ et X⁴ peuvent être les membres d'un cycle à 5 ou 6 membres,
qui composent un hétérocycle saturé ou insaturé avec ou sans autre condensation, et, et X¹ à X⁴, R² à R⁴ et R⁶ à R⁸ ont la signification donnée à la revendication 1, et R⁴ et R⁸ peuvent être également, halogène, nitro, cyano ou amino.

10. Dispositif selon l'une des revendications 1 à 7, où les composés de la formule **19** suivante sont présents 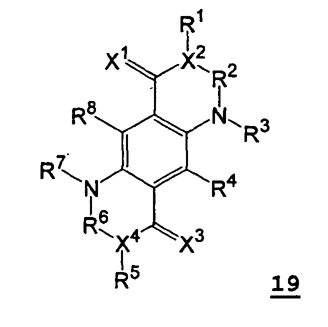 où
X² et R² ainsi que X⁴ et R⁶ peuvent être les membres d'un cycle à 5 ou 6 membres,
qui composent un hétérocycle saturé ou insaturé avec ou sans autre condensation, et, et X¹ à X⁴, R¹ R³ à R⁵, R⁷ et R⁸ ont la signification donnée à la revendication 1, et R⁴ et R⁸ peuvent être également, halogène, nitro, cyano ou amino.

11. Dispositif selon l'une des revendications 1 à 7, où les composés de la formule **20a** suivante sont présents 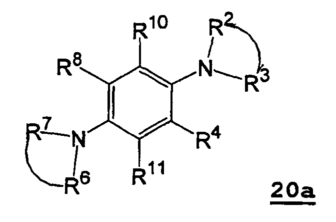 où
R¹⁰ représente un reste nitrile -CN ou un reste-C(=X¹)-X²R¹,
R¹¹ est un reste nitrile -CN ou un reste -C(=X³)-X⁴R⁵,
R² et R³ ainsi que R⁶ et R⁷ peuvent être les membres d'un cycle à 5 ou 6 membres,
qui composent un hétérocycle saturé ou insaturé avec ou sans autre condensation, et, et X¹ à X⁴, R¹, R⁴, R⁵ et R⁸ ont la signification donnée à la revendication 1, et R⁴ et R⁸ peuvent être également, halogène, nitro, cyano ou amino.

12. Dispositif selon l'une des revendications 1 à 7, où les composés de la formule **21a** suivante sont présents 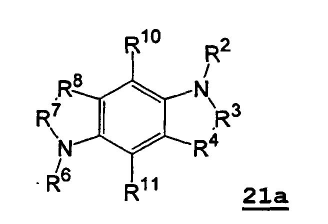 où R¹⁰ représente un reste nitrile -CN ou un reste -C(=X¹)-X²R¹,
R¹¹ est un reste nitrile -CN ou un reste -C(=X³)-X⁴R⁵, et
où R³ et R⁴ ainsi que R⁷ et R⁸ peuvent être les membres d'un cycle à 5 ou 6 membres,
qui composent un hétérocycle saturé ou insaturé avec ou sans autre condensation, et, et X¹ à X⁴, R¹, R², R⁵ et R⁶ ont la signification donnée à la revendication 1, et R⁴ et R⁸ peuvent être également, halogène, nitro, cyano ou amino.

13. Dispositif selon l'une des revendications 1 à 7, où les composés de la formule **22** suivante sont présents 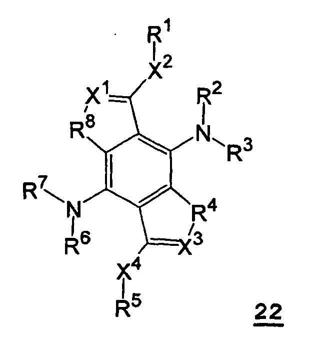 où
R⁴ et X³ ainsi que R⁸ et X¹ peuvent être les membres d'un cycle à 5 ou 6 membres,
qui composent un hétérocycle saturé ou insaturé avec ou sans autre condensation, et, et X¹ et X⁴ sont imino, X² et X³, R¹ à R³ et R⁵ à R⁷ ont la signification donnée à la revendication 1, et R⁴ et R⁸ peuvent être également, halogène, nitro, cyano ou amino.

14. Dispositif selon l'une des revendications 1 à 13, où dans une ou plusieurs couches émettrices, est présent un mélange de 2 - 6 représentants différents, dans un dispositif.

15. Dispositif selon l'une des revendications 1 à 6, où les composés de la formule **2** suivante sont présents, 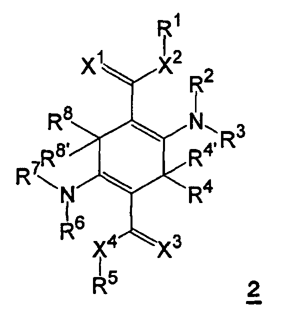 où X¹ et X³ ainsi que X² et X⁴, R¹ à R⁸, R^{4'} et R^{8'} ont la signification donnée à la revendication 1, et R⁴ et R⁸ ainsi que R^{4'} et R^{8'} peuvent être également, de manière identique ou différente, halogène, nitro, cyano, amino, trifluorométhyle, 2-fluorophényle, 3-fluorophényle, 4-fluorophényle, 2,4-difluorophényle, 2,6-difluorophényle, 2,3,4,5-tétrafluorophényle ou pentafluorophényle.

16. Dispositif selon l'une des revendications 1 à 6, où les composés de la formule **37** suivante sont présents, 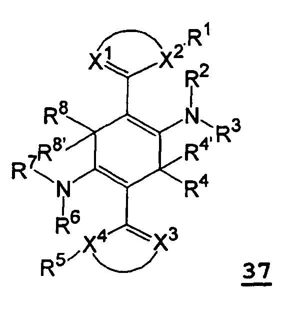 où
X¹ et X² ainsi que X³ et X⁴ peuvent être les membres d'un cycle à 5 ou 6 membres,
qui composent un hétérocycle saturé ou insaturé avec ou sans autre condensation, et, et X¹ et X³ sont imino, X² et X⁴ , ainsi que R¹ à R⁸ ont la signification donnée à la revendication 1, et R⁴ et R⁸ ainsi que R^{4'} et R^{8'} peuvent être également, de manière identique ou différente, halogène, nitro, cyano ou amino.

17. Dispositif selon l'une des revendications 1 à 6, où les composés de la formule **38** suivante sont présents, 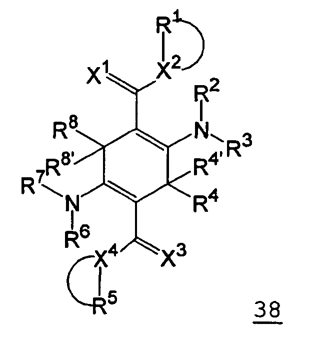 où
R¹ et X² ainsi que R⁵ et X⁴ peuvent être les membres d'un cycle à 5 ou 6 membres,
qui composent un hétérocycle saturé ou insaturé avec ou sans autre condensation, et, et X¹ à X⁴, R² à R⁴ et R⁶ à R⁸ ont la signification donnée à la revendication 1, et R⁴ et R⁸ ainsi que R^{4'} et R^{8'} peuvent être également, de manière identique ou différente, halogène, nitro, cyano ou amino.

18. Dispositif selon l'une des revendications 1 à 6, où les composés de la formule **39** suivante sont présents, 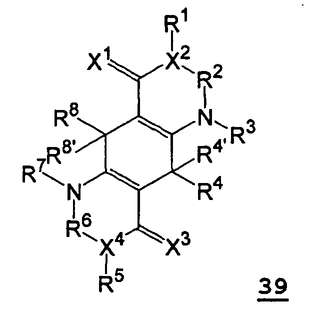 où X² et R² ainsi que X⁴ et R⁶ peuvent être les membres d'un cycle à 5 ou 6 membres,
qui composent un hétérocycle saturé ou insaturé avec ou sans autre condensation, et, et X¹ à X⁴, R¹, R³ à R⁵, R⁷ et R⁸ ont la signification donnée à la revendication 1, et R⁴ et R⁸ ainsi que R^{4'} et R^{8'} peuvent être également, de manière identique ou différente, halogène, nitro, cyano ou amino.

19. Dispositif selon l'une des revendications 1 à 6, où les composés de la formule **40a** suivante sont présents, 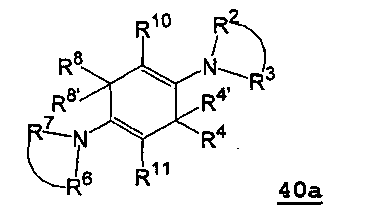 où R¹⁰ représente un reste nitrile -CN ou un reste -C(=X¹)-X²R¹,
R¹¹ est un reste nitrile -CN ou un reste -C(=X³)-X⁴R⁵,
et où R² et R³ ainsi que R⁶ et R⁷ peuvent être les membres d'un cycle à 5 ou 6 membres, qui composent un hétérocycle saturé ou insaturé avec ou sans autre condensation, et, X¹ à X⁴, R¹, R⁴, R⁵ et R⁸ ont la signification donnée à la revendication 1, et R⁴ et R⁸ ainsi que R^{4'} et R^{8'} peuvent être également, de manière identique ou différente, halogène, nitro, cyano ou amino.

20. Dispositif selon l'une des revendications 1 à 6, où les composés de la formule **41a** suivante sont présents, 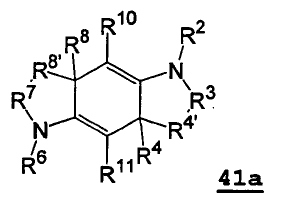 où R¹⁰ représente un reste nitrile -CN ou un reste -C(=X¹)-X²R¹,
R¹¹ est un reste nitrile -CN ou un reste -C(=X³)-X⁴R⁵,
et où R³ et R⁴ ainsi que R⁷ et R⁸ peuvent être les membres d'un cycle à 5 ou 6 membres, qui composent un hétérocycle saturé ou insaturé avec ou sans autre condensation, et, X¹ à X⁴, R¹, R², R⁵, R⁸ ont la signification donnée à la revendication 1, et R⁴ et R⁸ peuvent être également, de manière identique ou différente, halogène, nitro, cyano ou amino.

21. Dispositif selon l'une des revendications 1 à 6, où les composés de la formule **43** suivante sont présents, 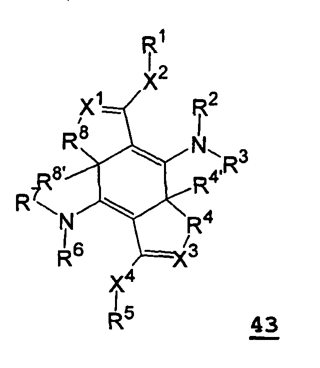 où R⁴ et X³ ainsi que R⁸ et peuvent être les membres d'un cycle à 5 ou 6 membres, qui composent un hétérocycle saturé ou insaturé avec ou sans autre condensation, et, X¹ et X³ sont imino , X² et X⁴ ainsi que R¹ à R³ et R⁵ à R⁷ ont la signification donnée à la revendication 1, et R^{4'} et R^{8'} peuvent être également, de manière identique ou différente, halogène, nitro, cyano ou amino.

22. Dispositif selon l'une des revendications 1 à 6, où les composés de la formule **42a** suivante sont présents, 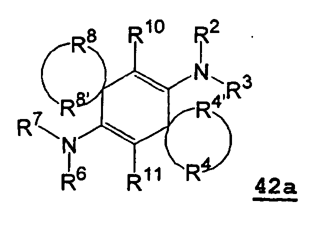 où R¹⁰ représente un reste nitrile -CN ou un reste -C(=X¹)-X²R¹,
R¹¹ est un reste nitrile -CN ou un reste -C(=X³)-X⁴R⁵,
et où R⁴ et R^{4'} ainsi que R⁸ et R^{8'} peuvent être les membres d'un cycle à 5 ou 6 membres, qui composent un hétérocycle saturé ou insaturé avec ou sans autre condensation, et, X¹ à X⁴, R¹ à R³ et R⁵ à R⁷ ont la signification donnée à la revendication 1.

23. Dispositif selon l'une des revendications 15 à 22, où les composés des revendications 15 - 22 sont présents dans une ou plusieurs couches émettrices, sous forme d'un mélange de 2 - 6 représentants différents, dans un dispositif.

24. Dispositif selon l'une des revendications 1 à 6, où les composés de la formule **3** ou **4** suivante sont présents, 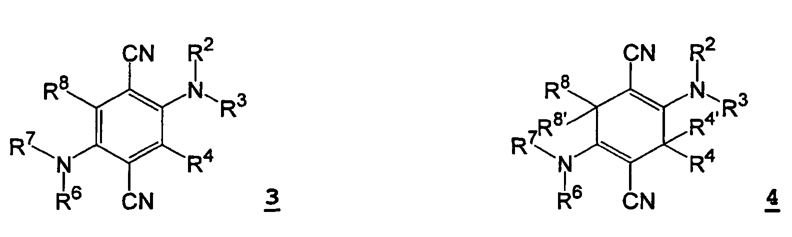 où R² à R⁸ ont la signification donnée aux revendications 1 et 2, et R⁴ et R⁸ peuvent être également, de manière identique ou différente, halogène, nitro, cyano ou amino.

25. Nouveaux dérivés de l'acide 2,5-diaminotéréphthalique de la formule **19**, 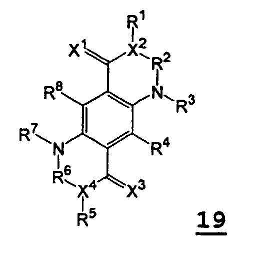 où
X¹ est O et X² est O ou N; R² et R⁶ sont méthylène (-CH₂-), qui peut être substitué par trifluorométhyle, R³ et R⁷ sont de manière identique ou différente, H, alkyle en C₁-C₈, aryle ou hétéroaryle, et R⁴ et R⁸ sont de manière identique ou différente, H, alkyle, aryle ou trifluorométhyle, et R¹ est rien, H ou alkyle en C₁-C₄.

26. Nouveaux dérivés de l'acide 2,5-diaminotéréphthalique de la formule **2**, 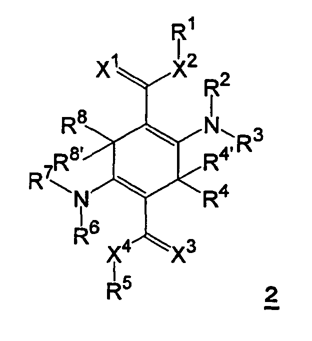 où
X¹ est O et X² est O ou N; R² et R⁶ ainsi que R³ et R⁷ sont de manière identique ou différente, H, alkyle en C₁-C₈, aryle ou hétéroaryle, et R⁴ et R⁸ ainsi que R^{4'} et R^{8'} sont différents de H et de manière identique ou différente, alkyle, aryle ou trifluorométhyle, et R¹ est H ou alkyle en C₁-C₄.

27. Nouveaux dérivés de l'acide 2,5-diaminotéréphthalique de la formule **3**, 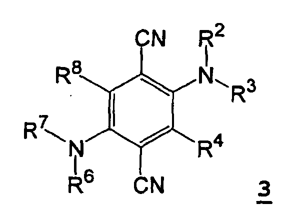 où R² et R⁶ ainsi que R³ et R⁷ sont de manière identique ou différente, H, alkyle en C₁-C₈, aryle ou hétéroaryle, et R⁴ et R⁸ sont différents de H et de manière identique ou différente, alkyle, aryle ou trifluorométhyle.

28. Nouveaux dérivés de l'acide 2,5-diaminotéréphthalique de la formule **4****,** 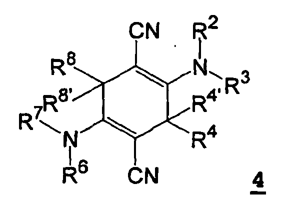 où R² et R⁶ ainsi que R³ et R⁷ sont de manière identique ou différente, H, alkyle en C₁-C₈, aryle ou hétéroaryle, et R⁴ et R⁸ ainsi que R^{4'} et R^{8'} sont différents de H et de manière identique ou différente, alkyle, aryle ou trifluorométhyle.

29. Dérivés selon l'une des revendications 25 à 28, où alkyle est alkyle en C₁-C₄, aryle est phényle ou naphtyle et hétéroaryle est pyridyle, thiényle ou furyle.

30. Dispositif selon les revendications 1 et 2, où R¹⁰ et R¹¹ ont la signification donnée ci-dessus, R² et R⁶ sont hydrogène ou alkyle en C₁-C₄, R³ et R⁷ sont chaque fois, phényle, substitué une ou deux fois par H, halogène,
CN, alkyle en C₁-C₄, alcoxy en C₁-C₄, haloalkyle en C₁-C₄ ou phényle.

31. Dispositif selon la revendication 10, où X¹ à X⁴ sont oxygène, R² et R⁴ sont substitués par des restes identiques ou différents H et alkyle en C₁-C₄, R³ et R⁷ sont chaque fois, phényle, substitué une ou deux fois par H, halogène, CN, alkyle en C₁-C₄, alcoxy en C₁-C₄, haloalkyle en C₁-C₄ ou phényle, et R⁴ et R⁸ sont hydrogène.

32. Dispositif selon l'une des revendications 31 et 32, où le reste alkyle en C₁-C₄ est un reste en C₁.
